# EUROPEAN PATENT APPLICATION

(11) **EP 4 748 861 A1**
(43) Date of publication of application: **27.05.2026**
(21) Application number: 24842440.0
(22) Date of filing: 19.07.2024
(51) Int. Cl.: C07K 16/46, A61K 39/395, A61P 35/00, A61P 35/02

(54) **BISPECIFIC ANTIBODY, DRUG CONJUGATE THEREOF, AND USE THEREOF**

(30) Priority: 19.07.2023 CN 202310895654; 04.03.2024 CN 202410244954; 09.07.2024 CN 202410914157
(71) Applicant: Duality Biologics (Suzhou) Co., Ltd., Suzhou, Jiangsu 215000 (CN)
(72) Inventor: ZHOU, Yunhua, Suzhou, Jiangsu 215000 (CN); HUA, Haiqing, Suzhou, Jiangsu 215000 (CN); YANG, Junjie, Suzhou, Jiangsu 215000 (CN); ZHU, Zhongyuan, Suzhou, Jiangsu 215000 (CN)
(74) Representative: Script Intellectual Property LLP
(86) International application number: PCT/CN2024/106435
(87) International publication number: WO 2025/016453

(57) **Abstract**

Disclosed in the present disclosure are a bispecific antibody, a drug conjugate thereof, and use thereof. The EGFR-binding domain comprises a heavy chain variable region VH1 and a light chain variable region VL1, and the HER3-binding domain comprises a heavy chain variable region VH2 and a light chain variable region VL2, wherein the VH1 comprises H1CDR1, H1CDR2, and H1CDR3 with the amino acid sequences set forth in SEQ ID NO: 1, SEQ ID NO: 2, and SEQ ID NO: 3, respectively; the VL1 comprises L1CDR1, L1CDR2, and L1CDR3 with the amino acid sequences set forth in SEQ ID NO: 4, SEQ ID NO: 5, and SEQ ID NO: 6, respectively; the VH2 comprises H2CDR1, H2CDR2, and H2CDR3 with the amino acid sequences set forth in SEQ ID NO: 7, SEQ ID NO: 8, and SEQ ID NO: 9, respectively; and the VL2 comprises L2CDR1, L2CDR2, and L2CDR3 with the amino acid sequences set forth in SEQ ID NO: 10, SEQ ID NO: 11, and SEQ ID NO: 12, respectively. The bispecific antibody and the drug conjugate thereof disclosed in the present disclosure have good endocytosis effect, proliferation inhibitory activity, tumor growth inhibitory activity, and *in vivo* safety.

## Description

The present application claims priority to Chinese Patent Application No. 2023108956544 filed on July 19, 2023, Chinese Patent Application No. 2024102449540 filed on March 4, 2024, and Chinese Patent Application No. 2024109141579 filed on July 9, 2024, which are incorporated herein by reference in their entirety.

### TECHNICAL FIELD

The present disclosure relates to the field of biotechnology, and specifically to a bispecific antibody comprising an EGFR-binding domain and an HER3-binding domain, a drug conjugate thereof, and use thereof.

### BACKGROUND

Epidermal growth factor receptor (EGFR) is a large transmembrane glycoprotein with a molecular weight of about 170 kDa, and is a member of the ErbB receptor family. The EGFR receptor itself is a tyrosine kinase, which can form a dimer after binding to a ligand such as EGF and TNF-a, and activate downstream signals (pathways such as MAPK, PI3K, and Stat) through transmission of phosphorylation, so that cell growth is maintained, and cell division and proliferation are promoted. As ErbB family receptors are conservative, EGFR can also form heterodimers with other proteins of the family (e.g., HER2, HER3, and HER4), thereby regulating cell growth more broadly.

HER3 is a member of the ErbB family, and plays a key role in cell proliferation, tumor metastasis, and drug resistance. Although drugs targeting EGFR and HER3 have shown great clinical benefits in the alleviation of various cancers, previous efforts to develop anti-HER3 antibodies for cancer treatment have repeatedly failed. An antibody-drug conjugate (ADC) consists of three portions, an antibody or an antigen-binding fragment thereof (targeting), a linker, and a small molecule drug. The antibody or the antigen-binding fragment thereof is conjugated with a bioactive small molecule drug such as a cytotoxin with cytotoxicity, through a cleavable or non-cleavable linker. This effectively utilizes the specificity of the antibody or the antigen-binding fragment thereof for targeting cells of interest (target cells) or binding to a highly expressed antigen, as well as the high efficiency of the small molecule drug, thereby reducing or avoiding toxic side effects on non-targeted cells. This means that antibody-drug conjugates used for tumors can precisely target tumor cells and reduce the effect on non-tumor cells, as compared to conventional tumor chemotherapy drugs.

There is still a need in the art for bispecific antibody-drug conjugates that are superior in affinity, specificity, etc.

### SUMMARY

The technical problem to be solved in the present disclosure is for overcoming the defect that there are relatively few bispecific antibody-drug conjugates targeting EGFR and HER3 in the prior art. Therefore, the present disclosure provides a bispecific antibody, a drug conjugate thereof, and use thereof. The bispecific antibody-drug conjugate of the present disclosure has good endocytosis effect, proliferation inhibitory activity, tumor growth inhibitory activity, and *in vivo* safety.

The present disclosure mainly solves the technical problem described above through the following technical means.

In order to solve the technical problem described above, in a first aspect of the present disclosure, provided is a bispecific antibody comprising an EGFR-binding domain and an HER3-binding domain, the EGFR-binding domain comprising a heavy chain variable region VH1 and a light chain variable region VL1, and the HER3-binding domain comprising a heavy chain variable region VH2 and a light chain variable region VL2, wherein the VH1 comprises H1CDR1, H1CDR2, and H1CDR3 with the amino acid sequences set forth in SEQ ID NO: 1, SEQ ID NO: 2, and SEQ ID NO: 3, respectively; the VL1 comprises L1CDR1, L1CDR2, and L1CDR3 with the amino acid sequences set forth in SEQ ID NO: 4, SEQ ID NO: 5, and SEQ ID NO: 6, respectively; the VH2 comprises H2CDR1, H2CDR2, and H2CDR3 with the amino acid sequences set forth in SEQ ID NO: 7, SEQ ID NO: 8, and SEQ ID NO: 9, respectively; and the VL2 comprises L2CDR1, L2CDR2, and L2CDR3 with the amino acid sequences set forth in SEQ ID NO: 10, SEQ ID NO: 11, and SEQ ID NO: 12, respectively.

In some embodiments of the present disclosure, the amino acid sequence of the H2CDR2 is set forth in SEQ ID NO: 77 or 78.

In some preferred embodiments of the present disclosure, the VH1 comprises framework regions H1FR1, H1FR2, H1FR3, and H1FR4 with amino acid sequences set forth in or having at least 90%, 91%, 92%, 93%, 94%, 95%, 96%, 97%, 98%, or 99% identity to SEQ ID NO: 13, SEQ ID NO: 14, SEQ ID NO: 15, and SEQ ID NO: 16, respectively;
the VL1 comprises framework regions L1FR1, L1FR2, L1FR3, and L1FR4 with amino acid sequences set forth in or having at least 90%, 91%, 92%, 93%, 94%, 95%, 96%, 97%, 98%, or 99% identity to SEQ ID NO: 17, SEQ ID NO: 18, SEQ ID NO: 19, and SEQ ID NO: 20, respectively;
the VH2 comprises a framework region H2FR1 with an amino acid sequence set forth in or having at least 90%, 91%, 92%, 93%, 94%, 95%, 96%, 97%, 98%, or 99% identity to SEQ ID NO: 21, for example, having an E16D mutation on SEQ ID NO: 21; framework regions H2FR2 and H2FR4 with amino acid sequences set forth in or having at least 90%, 91%, 92%, 93%, 94%, 95%, 96%, 97%, 98%, or 99% identity to SEQ ID NO: 22 and SEQ ID NO: 24, respectively; and a framework region H2FR3 with an amino acid sequence set forth in or having at least 90%, 91%, 92%, 93%, 94%, 95%, 96%, 97%, 98%, or 99% identity to SEQ ID NO: 23, for example, having an S18D mutation on SEQ ID NO: 23;
the VL2 comprises a framework region L2FR1 with an amino acid sequence set forth in or having at least 90%, 91%, 92%, 93%, 94%, 95%, 96%, 97%, 98%, or 99% identity to SEQ ID NO: 25, for example, having S9D and V15L mutations, or an S7E mutation, on SEQ ID NO: 25; and framework regions L2FR2, L2FR3, and L2FR4 with amino acid sequences set forth in or having at least 90%, 91%, 92%, 93%, 94%, 95%, 96%, 97%, 98%, or 99% identity to SEQ ID NO: 18, SEQ 'ID NO: 26, and SEQ ID NO: 27, respectively.

In some embodiments of the present disclosure, an amino acid sequence of the VH1 is set forth in SEQ ID NO: 28, an amino acid sequence of the VL1 is set forth in SEQ ID NO: 29, an amino acid sequence of the VH2 is set forth in SEQ ID NO: 30, SEQ ID NO: 79, or SEQ ID NO: 80, and an amino acid sequence of the VL2 is set forth in SEQ ID NO: 31, SEQ ID NO: 81, or SEQ ID NO: 82.

In some preferred embodiments of the present disclosure, the amino acid sequences of the VH1, the VL1, the VH2, and the VL2 of the bispecific antibody are set forth in SEQ ID NO: 28, SEQ ID NO: 29, SEQ ID NO: 30, and SEQ ID NO: 31, respectively, or set forth in SEQ ID NO: 28, SEQ ID NO: 29, SEQ ID NO: 79, and SEQ ID NO: 81, respectively, or set forth in SEQ ID NO: 28, SEQ ID NO: 29, SEQ ID NO: 79, and SEQ ID NO: 31, respectively, or set forth in SEQ ID NO: 28, SEQ ID NO: 29, SEQ ID NO: 80, and SEQ ID NO: 82, respectively.

In some embodiments of the present disclosure, the EGFR-binding domain and the HER3-binding domain further each comprise a light chain constant region and a heavy chain constant region, the EGFR-binding domain comprising a light chain constant region CL1 and a heavy chain constant region HC1, and the HER3-binding domain comprising a light chain constant region CL2 and a heavy chain constant region HC2, wherein amino acid sequences of the CL1 and the CL2 are set forth in SEQ ID NO: 32 or SEQ ID NO: 33, respectively, or have at least 90%, 91%, 92%, 93%, 94%, 95%, 96%, 97%, 98%, or 99% identity thereto, and the amino acid sequences of the CL1 and the CL2 are not identical sequences; and/or the HC1 comprises C1H1 and Fc1, and the HC2 comprises C2H1 and Fc2, wherein amino acid sequences of the C1H1 and the C2H1 are set forth in SEQ ID NO: 34 or SEQ ID NO: 35, or have at least 90%, 91%, 92%, 93%, 94%, 95%, 96%, 97%, 98%, or 99% identity thereto, and the amino acid sequences of the C1H1 and the C2H1 are not identical sequences; amino acid sequences of the Fc1 and the Fc2 are variant sequences of the amino acid sequence set forth in SEQ ID NO: 36, or have at least 90%, 91%, 92%, 93%, 94%, 95%, 96%, 97%, 98%, or 99% identity thereto, for example, having a T146W mutation, or S134C and T146W mutations, or T146S, L148A and Y187V mutations, or Y349C, T366S, L368A and Y407V mutations, on SEQ ID NO: 36, and the amino acid sequences of the Fc1 and the Fc2 are not identical sequences.

In some preferred embodiments of the present disclosure, the amino acid sequences of the CL1 and the CL2 are set forth in SEQ ID NO: 32 or SEQ ID NO: 33, respectively, the amino acid sequences of the C1H1 and the C2H1 are set forth in SEQ ID NO: 34 or SEQ ID NO: 35, and the amino acid sequences of the Fc1 and the Fc2 are variant sequences of the amino acid sequence set forth in SEQ ID NO: 36, for example, having a T146W mutation, or S134C and T146W mutations, or T146S, L148A and Y187V mutations, or Y349C, T366S, L368A and Y407V mutations, on SEQ ID NO: 36.

In some more preferred embodiments of the present disclosure, the Fc1 and the Fc2 are connected by a disulfide bond in a hinge region and a knob-into-hole structure, wherein the Fc1 is knob-Fc and the Fc2 is hole-Fc, or the Fc2 is knob-Fc and the Fc1 is hole-Fc.

In some further more preferred embodiments of the present disclosure, the C1H1 and the Fc1, as well as the C2H1 and the Fc2, are connected by a hinge region, wherein an amino acid sequence of the hinge region is set forth in SEQ ID NO: 89.

In some preferred embodiments of the present disclosure, the EGFR-binding domain comprises a light chain constant region CL1 and a heavy chain constant region HC1, and the HER3-binding domain comprises a heavy chain constant region HC2, wherein an amino acid sequence of the CL1 is set forth in SEQ ID NO: 32, or has at least 90%, 91%, 92%, 93%, 94%, 95%, 96%, 97%, 98%, or 99% identity thereto; and/or the HC1 comprises C1H1 and Fe1, and the HC2 comprises Fc2, wherein an amino acid sequence of the C1H1 is set forth in SEQ ID NO: 34, or has at least 90%, 91%, 92%, 93%, 94%, 95%, 96%, 97%, 98%, or 99% identity thereto; amino acid sequences of the Fe1 and the Fc2 are variant sequences of the amino acid sequence set forth in SEQ ID NO: 36, or have at least 90%, 91%, 92%, 93%, 94%, 95%, 96%, 97%, 98%, or 99% identity thereto.

In some preferred embodiments of the present disclosure, the amino acid sequence of the CL1 is set forth in SEQ ID NO: 32, the amino acid sequence of the C1H1 is set forth in SEQ ID NO: 34, and the amino acid sequences of the Fc1 and the Fc2 are set forth in SEQ ID NOs: 93 and 94, respectively;
in some preferred embodiments of the present disclosure, the Fc1 and the Fc2 are connected by a disulfide bond in a hinge region and a knob-into-hole structure, wherein the Fc1 is knob-Fc and the Fc2 is hole-Fc, or the Fc2 is knob-Fc and the Fc1 is hole-Fc;
in some preferred embodiments of the present disclosure, the C1H1 and the Fc1 are connected by a hinge region with the amino acid sequence set forth in SEQ ID NO: 89; the VL2 and the VH2 are connected by a hinge region with the amino acid sequence set forth in SEQ ID NO: 95; the VH2 and the Fc2 are connected by a hinge region with the amino acid sequence set forth in SEQ ID NO: 96.

In some preferred embodiments of the present disclosure, the bispecific antibody comprises a heavy chain H1, a light chain L1, and a heavy chain H2, wherein an amino acid sequence of the H1 is set forth in SEQ ID NO: 37, or has at least 90%, 91%, 92%, 93%, 94%, 95%, 96%, 97%, 98%, or 99% identity thereto, and/or an amino acid sequence of the L1 is set forth in SEQ ID NO: 38, or has at least 90%, 91%, 92%, 93%, 94%, 95%, 96%, 97%, 98%, or 99% identity thereto, and/or an amino acid sequence of the H2 is set forth in SEQ ID NO: 90, or has at least 90%, 91%, 92%, 93%, 94%, 95%, 96%, 97%, 98%, or 99% identity thereto;
in some preferred embodiments of the present disclosure, the bispecific antibody is DBXT005-01 comprising a heavy chain H1, a light chain L1, and a heavy chain H2 with the amino acid sequences set forth in SEQ ID NO: 37, SEQ ID NO: 38, and SEQ ID NO: 90, respectively. In some preferred embodiments of the present disclosure, the heavy chain H2 has an Fc + scFv structure.

In some embodiments of the present disclosure, the bispecific antibody comprises a heavy chain H1, a light chain L1, a heavy chain H2, and a light chain L2.

In some preferred embodiments of the present disclosure, the bispecific antibody is DBXT001-01 comprising a heavy chain H1, a light chain L1, a heavy chain H2, and a light chain L2 with the amino acid sequences set forth in SEQ ID NO: 37, SEQ ID NO: 38, SEQ ID NO: 39, and SEQ ID NO: 40, respectively; or
DBXT001-02 comprising a heavy chain H1, a light chain L1, a heavy chain H2, and a light chain L2 with the amino acid sequences set forth in SEQ ID NO: 41, SEQ ID NO: 38, SEQ ID NO: 42, and SEQ ID NO: 40, respectively; or
DBXT001-03 comprising a heavy chain H1, a light chain L1, a heavy chain H2, and a light chain L2 with the amino acid sequences set forth in SEQ ID NO: 43, SEQ ID NO: 38, SEQ ID NO: 44, and SEQ ID NO: 40, respectively; or
DBXT001-04 comprising a heavy chain H1, a light chain L1, a heavy chain H2, and a light chain L2 with the amino acid sequences set forth in SEQ ID NO: 45, SEQ ID NO: 38, SEQ ID NO: 46, and SEQ ID NO: 40, respectively; or
DBXT001-05 comprising a heavy chain H1, a light chain L1, a heavy chain H2, and a light chain L2 with the amino acid sequences set forth in SEQ ID NO: 47, SEQ ID NO: 48, SEQ ID NO: 49, and SEQ ID NO: 50, respectively; or
DBXT001-06 comprising a heavy chain H1, a light chain L1, a heavy chain H2, and a light chain L2 with the amino acid sequences set forth in SEQ ID NO: 51, SEQ ID NO: 48, SEQ ID NO: 52, and SEQ ID NO: 50, respectively; or
DBXT001-07 comprising a heavy chain H1, a light chain L1, a heavy chain H2, and a light chain L2 with the amino acid sequences set forth in SEQ ID NO: 53, SEQ ID NO: 48, SEQ ID NO: 54, and SEQ ID NO: 50, respectively; or
DBXT001-08 comprising a heavy chain H1, a light chain L1, a heavy chain H2, and a light chain L2 with the amino acid sequences set forth in SEQ ID NO: 55, SEQ ID NO: 48, SEQ ID NO: 56, and SEQ ID NO: 50, respectively; or
DBXT002-01 comprising a heavy chain H1, a light chain L1, a heavy chain H2, and a light chain L2 with the amino acid sequences set forth in SEQ ID NO: 37, SEQ ID NO: 38, SEQ ID NO: 57, and SEQ ID NO: 58, respectively; or
DBXT002-02 comprising a heavy chain H1, a light chain L1, a heavy chain H2, and a light chain L2 with the amino acid sequences set forth in SEQ ID NO: 41, SEQ ID NO: 38, SEQ ID NO: 59, and SEQ ID NO: 58, respectively; or
DBXT002-03 comprising a heavy chain H1, a light chain L1, a heavy chain H2, and a light chain L2 with the amino acid sequences set forth in SEQ ID NO: 43, SEQ ID NO: 38, SEQ ID NO: 60, and SEQ ID NO: 58, respectively; or
DBXT002-04 comprising a heavy chain H1, a light chain L1, a heavy chain H2, and a light chain L2 with the amino acid sequences set forth in SEQ ID NO: 45, SEQ ID NO: 38, SEQ ID NO: 61, and SEQ ID NO: 58, respectively; or
DBXT002-05 comprising a heavy chain H1, a light chain L1, a heavy chain H2, and a light chain L2 with the amino acid sequences set forth in SEQ ID NO: 47, SEQ ID NO: 48, SEQ ID NO: 62, and SEQ ID NO: 63, respectively; or
DBXT002-06 comprising a heavy chain H1, a light chain L1, a heavy chain H2, and a light chain L2 with the amino acid sequences set forth in SEQ ID NO: 51, SEQ ID NO: 48, SEQ ID NO: 64, and SEQ ID NO: 63, respectively; or
DBXT002-07 comprising a heavy chain H1, a light chain L1, a heavy chain H2, and a light chain L2 with the amino acid sequences set forth in SEQ ID NO: 53, SEQ ID NO: 48, SEQ ID NO: 65, and SEQ ID NO: 63, respectively; or
DBXT002-08 comprising a heavy chain H1, a light chain L1, a heavy chain H2, and a light chain L2 with the amino acid sequences set forth in SEQ ID NO: 55, SEQ ID NO: 48, SEQ ID NO: 66, and SEQ ID NO: 63, respectively; or
DBXT003-01 comprising a heavy chain H1, a light chain L1, a heavy chain H2, and a light chain L2 with the amino acid sequences set forth in SEQ ID NO: 37, SEQ ID NO: 38, SEQ ID NO: 57, and SEQ ID NO: 40, respectively; or
DBXT003-02 comprising a heavy chain H1, a light chain L1, a heavy chain H2, and a light chain L2 with the amino acid sequences set forth in SEQ ID NO: 41, SEQ ID NO: 38, SEQ ID NO: 59, and SEQ ID NO: 40, respectively; or
DBXT003-03 comprising a heavy chain H1, a light chain L1, a heavy chain H2, and a light chain L2 with the amino acid sequences set forth in SEQ ID NO: 43, SEQ ID NO: 38, SEQ ID NO: 60, and SEQ ID NO: 40, respectively; or
DBXT003-04 comprising a heavy chain H1, a light chain L1, a heavy chain H2, and a light chain L2 with the amino acid sequences set forth in SEQ ID NO: 45, SEQ ID NO: 38, SEQ ID NO: 61, and SEQ ID NO: 40, respectively; or
DBXT003-05 comprising a heavy chain H1, a light chain L1, a heavy chain H2, and a light chain L2 with the amino acid sequences set forth in SEQ ID NO: 47, SEQ ID NO: 48, SEQ ID NO: 62, and SEQ ID NO: 50, respectively; or
DBXT003-06 comprising a heavy chain H1, a light chain L1, a heavy chain H2, and a light chain L2 with the amino acid sequences set forth in SEQ ID NO: 51, SEQ ID NO: 48, SEQ ID NO: 64, and SEQ ID NO: 50, respectively; or
DBXT003-07 comprising a heavy chain H1, a light chain L1, a heavy chain H2, and a light chain L2 with the amino acid sequences set forth in SEQ ID NO: 53, SEQ ID NO: 48, SEQ ID NO: 65, and SEQ ID NO: 50, respectively; or
DBXT003-08 comprising a heavy chain H1, a light chain L1, a heavy chain H2, and a light chain L2 with the amino acid sequences set forth in SEQ ID NO: 55, SEQ ID NO: 48, SEQ ID NO: 66, and SEQ ID NO: 50, respectively; or
DBXT004-01 comprising a heavy chain H1, a light chain L1, a heavy chain H2, and a light chain L2 with the amino acid sequences set forth in SEQ ID NO: 37, SEQ ID NO: 38, SEQ ID NO: 67, and SEQ ID NO: 68, respectively; or
DBXT004-02 comprising a heavy chain H1, a light chain L1, a heavy chain H2, and a light chain L2 with the amino acid sequences set forth in SEQ ID NO: 41, SEQ ID NO: 38, SEQ ID NO: 69, and SEQ ID NO: 68, respectively; or
DBXT004-03 comprising a heavy chain H1, a light chain L1, a heavy chain H2, and a light chain L2 with the amino acid sequences set forth in SEQ ID NO: 43, SEQ ID NO: 38, SEQ ID NO: 70, and SEQ ID NO: 68, respectively; or
DBXT004-04 comprising a heavy chain H1, a light chain L1, a heavy chain H2, and a light chain L2 with the amino acid sequences set forth in SEQ ID NO: 45, SEQ ID NO: 38, SEQ ID NO: 71, and SEQ ID NO: 68, respectively; or
DBXT004-05 comprising a heavy chain H1, a light chain L1, a heavy chain H2, and a light chain L2 with the amino acid sequences set forth in SEQ ID NO: 47, SEQ ID NO: 48, SEQ ID NO: 72, and SEQ ID NO: 73, respectively; or
DBXT004-06 comprising a heavy chain H1, a light chain L1, a heavy chain H2, and a light chain L2 with the amino acid sequences set forth in SEQ ID NO: 51, SEQ ID NO: 48, SEQ ID NO: 74, and SEQ ID NO: 73, respectively; or
DBXT004-07 comprising a heavy chain H1, a light chain L1, a heavy chain H2, and a light chain L2 with the amino acid sequences set forth in SEQ ID NO: 53, SEQ ID NO: 48, SEQ ID NO: 75, and SEQ ID NO: 73, respectively; or
DBXT004-08 comprising a heavy chain H1, a light chain L1, a heavy chain H2, and a light chain L2 with the amino acid sequences set forth in SEQ ID NO: 55, SEQ ID NO: 48, SEQ ID NO: 76, and SEQ ID NO: 73, respectively.

The DBXT001 series comprises DBXT001-01 to DBXT001-08, the DBXT002 series comprises DBXT002-01 to DBXT002-08, the DBXT003 series comprises DBXT003-01 to DBXT003-0108, and the DBXT004 series comprises DBXT004-01 to DBXT004-08.

In order to solve the technical problem described above, in a second aspect of the present disclosure, provided is an isolated nucleic acid, encoding the bispecific antibody according to the first aspect of the present disclosure.

In order to solve the technical problem described above, in a third aspect of the present disclosure, provided is a recombinant expression vector, comprising the nucleic acid according to the second aspect of the present disclosure.

In order to solve the technical problem described above, in a fourth aspect of the present disclosure, provided is a transformant, comprising the nucleic acid according to the second aspect of the present disclosure or the recombinant expression vector according to the third aspect of the present disclosure in a host cell.

In some preferred embodiments of the present disclosure, the host cell is a eukaryotic cell, preferably a mammalian cell, such as a CHO cell.

In order to solve the technical problem described above, in a fifth aspect of the present disclosure, provided is a method for preparing the bispecific antibody according to the first aspect of the present disclosure, comprising culturing the transformant according to the fourth aspect of the present disclosure to obtain the bispecific antibody.

The HC1 is the heavy chain constant region of a first heavy chain, and the HC2 is the heavy chain constant region of a second heavy chain. The VH1, the C1H1, and the Fc1 are VH, CH1 and Fc regions of the first heavy chain H1, respectively; the CL1 and the VL1 are CL and VL regions of the first light chain L1, respectively; the VH2, the C2H1, and the Fc2 are VH, CH1 and Fc regions of the second heavy chain H2, respectively; the CL2 and the VL2 are CL and VL regions of the second light chain L2, respectively. The L1FR1, the L1FR2, the L1FR3, and the L1FR4 are the framework regions of the light chain variable region of the first light chain; the H1FR1, the H1FR2, the H1FR3, and the H1FR4 are the framework regions of the heavy chain variable region of the first heavy chain; the L2FR1, the L2FR2, the L2FR3, and the L2FR4 are the framework regions of the light chain variable region of the second light chain; the H2FR1, the H2FR2, the H2FR3, and the H2FR4 are the framework regions of the heavy chain variable region of the second heavy chain.

The antibody sequences are numbered using the Kabat numbering system.

The present disclosure further provides a bispecific antibody-drug conjugate, or a tautomer, an enantiomer, a diastereoisomer or a mixture of isomers thereof, or a pharmaceutically acceptable salt thereof, comprising the following fragments: the bispecific antibody comprising the EGFR-binding domain and the HER3-binding domain or an antigen-binding fragment thereof, a linker unit L, and a cytotoxic drug, wherein the bispecific antibody or the antigen-binding fragment thereof is as described in any one of the embodiments of the present disclosure.

In some embodiments, the cytotoxic drug is camptothecin and a derivative thereof.

In some embodiments, the cytotoxic drug is of a structure of formula (A-1), or a tautomer, an enantiomer or a diastereoisomer thereof: 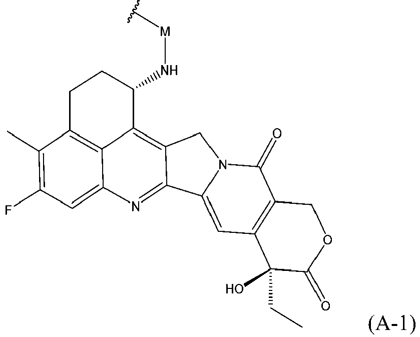 wherein
M is -L²-L¹-C(O)-;
L² is selected from -NH-, O, and S, and L² is linked to the linker unit L;
L1 is -(C(R^{1a})(R^{1b}))ₘ-CH₂-, saturated C₃-C₆ cycloalkylene, or saturated 3- to 6-membered heterocyclylene, wherein the saturated C₃-C₆ cycloalkylene and the saturated 3- to 6-membered heterocyclylene are each independently optionally substituted with one or more R^{2a};
m is selected from 1, 2, 3, and 4; each heteroatom in the saturated 3- to 6-membered heterocyclylene is independently N, O, or S, and the number of the heteroatom is 1, 2, or 3;
R^{1a} and R^{1b} are each independently selected from hydrogen, halogen, hydroxyl, amino, and C₁-C₆ alkyl, the C₁-C₆ alkyl being optionally substituted with one or more halogens;
R^{2a} is selected from halogen, hydroxyl, amino, and C₁-C₆ alkyl, the C₁-C₆ alkyl being optionally substituted with one or more halogens.

In certain preferred embodiments of the present disclosure, in the compound of formula (A-1), (A-2), (A-2a) or (A-2b), certain groups are defined as follows, and groups not mentioned are as described in any one of the embodiments of the present disclosure ("in some embodiments" for short).

In some embodiments, L² is preferably -O- or -S-, and more preferably -O-.

In some embodiments, L¹ is -(C(R^{1a})(R^{1b}))ₘ-CH₂-; R^{1a} is selected from: hydrogen, halogen, and C₁-C₆ alkyl; R^{1b} is selected from: hydrogen, halogen, and C₁-C₆ alkyl.

In some embodiments, L¹ is -(C(R^{1a})(R^{1b}))ₘ-CH₂-; R^{1a} is C₁-C₆ alkyl, preferably C₁-C₃ alkyl; R^{1b} is selected from: hydrogen and C₁-C₆ alkyl, preferably selected from: hydrogen and C₁-C₃ alkyl.

In some embodiments, L¹ is -(C(R^{1a})(R^{1b}))ₘ-CH₂-; R^{1a} is -CH₃; R^{1b} is selected from: hydrogen and -CH₃.

In some embodiments, L¹ is -(C(R^{1a})(R^{1b}))ₘ-CH₂-; m is 1 or 2, preferably 1.

In some embodiments, L¹ is selected from:

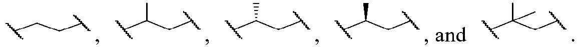

In some embodiments, L¹ is saturated C₃-C₆ cycloalkylene or saturated 3- to 6-membered heterocyclylene, preferably saturated C₃-C₆ cycloalkylene, wherein the saturated C₃-C₆ cycloalkylene and the saturated 3- to 6-membered heterocyclylene are each independently optionally substituted with one or more R^{2a}, each R^{2a} being independently selected from: halogen and C₁-C₆ alkyl.

In some embodiments, L¹ is cyclopropyl, cyclobutyl, cyclopentyl, or cyclohexyl, optionally substituted with one or more R^{2a}, each R^{2a} being independently selected from: halogen and C₁-C₆ alkyl.

In some embodiments, L¹ is selected from:

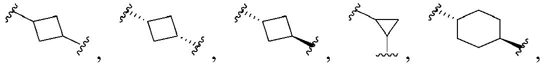

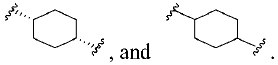

In some embodiments, in the definitions of L¹ and X₁, the C₃-C₆ cycloalkylene is cyclobutyl or cyclohexyl, preferably cyclobutyl.

In some embodiments, in the definition of L¹, each heteroatom of the 3- to 6-membered heterocyclylene is independently N or O, and the number of the heteroatom is preferably 1 or 2.

In some embodiments, in the definitions of R^{1a}, R^{1b}, and R^{2a}, the C₁-C₆ alkyl is C₁-C₃ alkyl, preferably methyl. In some embodiments, in the definitions of R, R^{1a}, R^{1b}, and R^{2a}, the halogen is F, Cl, Br, or I, preferably F, Cl, or Br.

In some embodiments, in the structure of formula (A-1),
M is -L²-L¹-C(O)-;
L² is -O-;
L¹ is -(C(R^{1a})(R^{1b}))ₘ-CH₂- or saturated C₃-C₆ cycloalkylene, wherein the saturated C₃-C₆ cycloalkylene is optionally substituted with one or more R^{2a};
m is selected from 1 or 2;
R^{1a} and R^{1b} are each independently selected from hydrogen, halogen, and C₁-C₆ alkyl, the C₁-C₆ alkyl being optionally substituted with one or more halogens;
R^{2a} is selected from halogen and C₁-C₆ alkyl, the C₁-C₆ alkyl being optionally substituted with one or more halogens.

In some embodiments, M is:

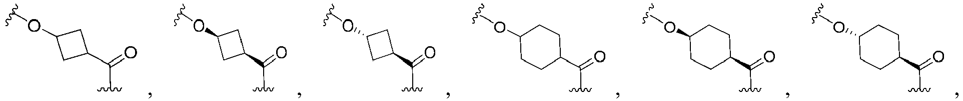

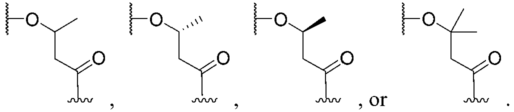

In some embodiments, M is

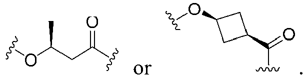

In some embodiments, the cytotoxic drug is selected from any one of the following structures:

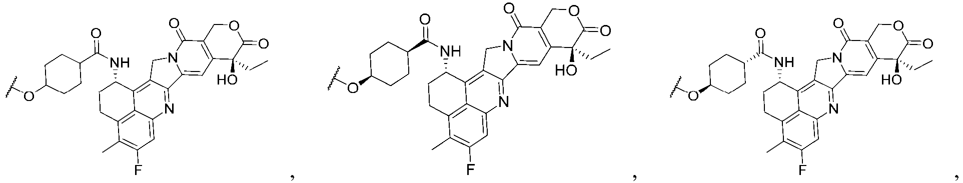

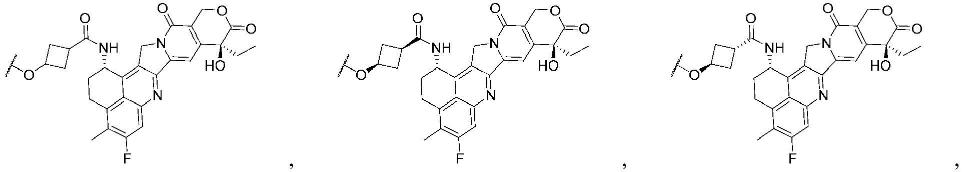

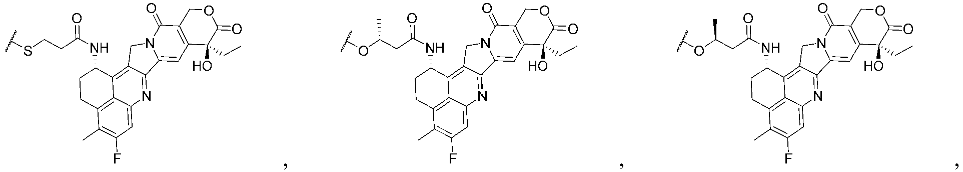

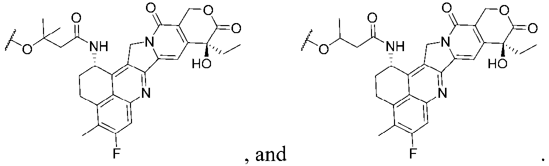

In some embodiments, the linker unit L is -Lₐ-L_{b}-L_{c}-, wherein L_{c} is linked to the cytotoxic drug; -Lₐ- is 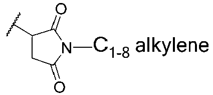 or -C₁₋₈ alkylene-C(O)-, preferably 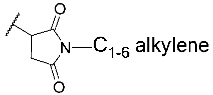 or -C₁₋₆ alkylene-C(O)-, and more preferably 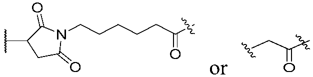 ; in the definition of -Lₐ-, each group fragment is preferably linked to the L_{b} at the right end; -Lₐ- is further preferably 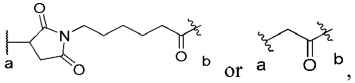 wherein the a end is connected to Ab, and the b end is connected to L_{b};
- L_{b}- is -(polypeptide of 1 to 6 natural amino acids)-NH-, preferably -(polypeptide of 2 to 4 natural amino acids)-NH-, more preferably selected from any one of the following structures: 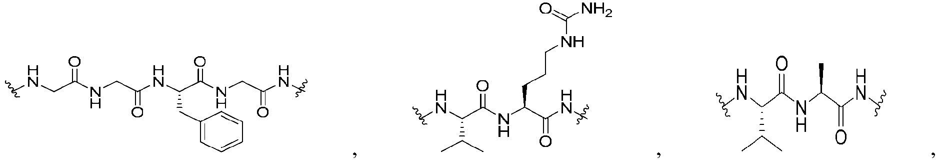 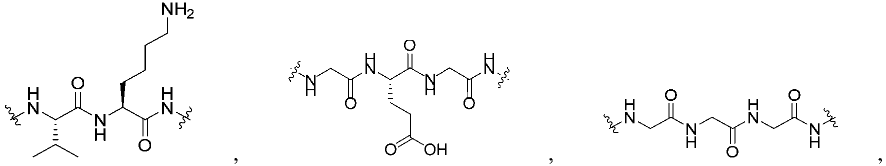 and 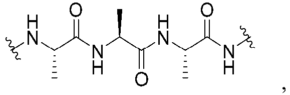 and further preferably 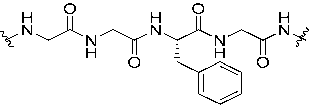 or 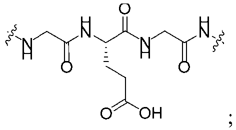 in the definition of -L_{b}-, each group fragment is preferably linked to the L_{c} at the right end;
- L_{b}- is more preferably 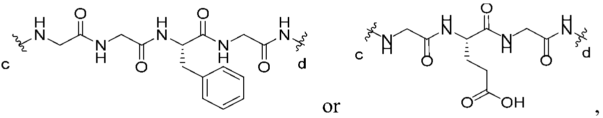 wherein the c end is connected to Lₐ, and the d end is connected to L_{c};
- L_{c}- is C₁₋₆ alkylene, preferably C₁₋₃ alkylene, and more preferably 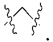 In some embodiments, provided is the bispecific antibody-drug conjugate of the present disclosure, wherein the linker unit L is 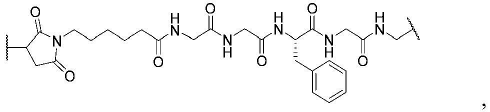 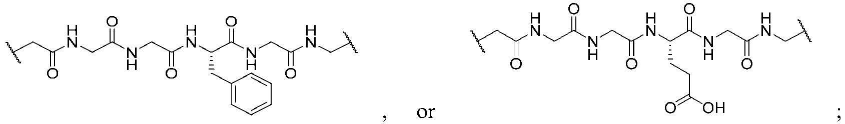 preferably

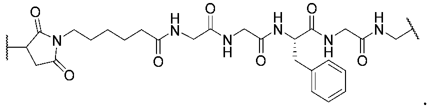

In some embodiments, provided is the bispecific antibody-drug conjugate of the present disclosure, having a structure of formula (A-2): 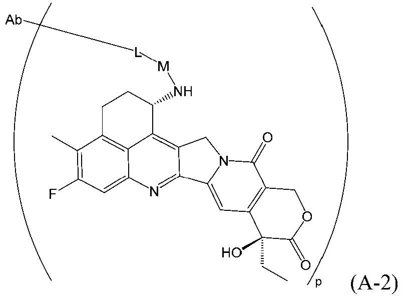 wherein p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8; Ab and M are each as defined in any one of the embodiments of the present disclosure; L is the linker unit L as described in any one of the embodiments of the present disclosure.

In some embodiments, provided is the bispecific antibody-drug conjugate of the present disclosure, having a structure of formula (A-2): 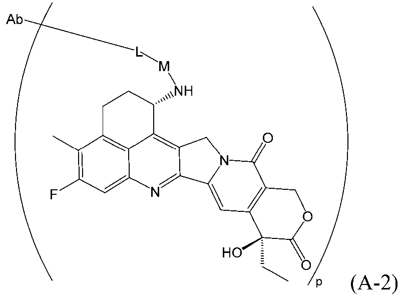 wherein
p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8;
Ab is the bispecific antibody as described in any one of the embodiments of the present disclosure or an antigen-binding fragment thereof;
L is the linker unit L as described in any one of the embodiments of the present disclosure;
M is -L²-L¹-C(O)-;
L² is -O- or -S-, and L² is linked to L;
L¹ is -(C(R^{1a})(R^{1b}))ₘ-CH₂-, saturated C₃-C₆ cycloalkylene, or saturated 3- to 6-membered heterocyclylene, wherein the saturated C₃-C₆ cycloalkylene and the saturated 3- to 6-membered heterocyclylene are each independently optionally substituted with one or more R^{2a};
m is 1, 2, 3, or 4; each heteroatom in the saturated 3- to 6-membered heterocyclyl is independently N, O, or S, and the number of the heteroatom is 1, 2, or 3;
R^{1a} and R^{1b}R^{2a} are each independently hydrogen, halogen, hydroxyl, amino, or C₁-C₆ alkyl, the C₁-C₆ alkyl being optionally substituted with one or more halogens;
R^{2a} is selected from halogen, hydroxyl, amino, and C₁-C₆ alkyl, the C₁-C₆ alkyl being optionally substituted with one or more halogens.

In some embodiments, provided is the bispecific antibody-drug conjugate of the present disclosure, having a structure of formula (A-2a) or (A-2b): 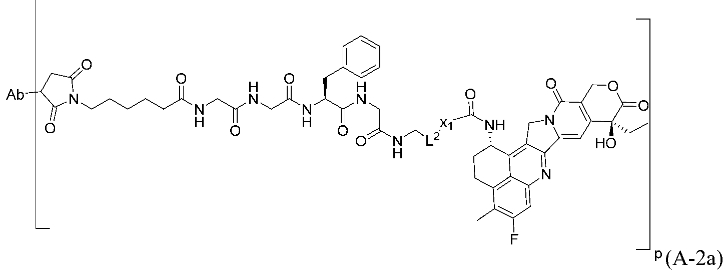 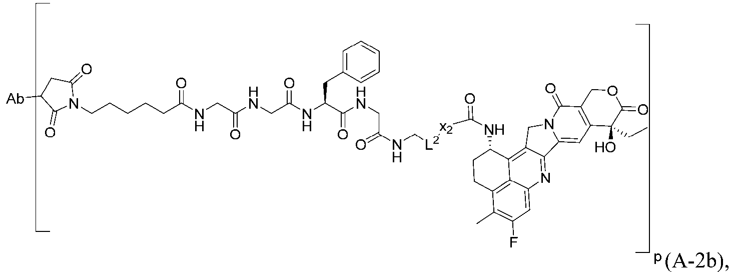 wherein
p represents an average connection number, and p is selected from any one of integers or decimals from 1 to 10, preferably any one of integers or decimals from 3 to 9, such as 4, 4.06, 4.10, 6, 6.11, 6.05, 7.99, 7.98, or 8;
Ab is the bispecific antibody as described in any one of the embodiments of the present disclosure or an antigen-binding fragment thereof;
L² is -NH-, O, or S, preferably -O- or -S-, and more preferably -O-;
X₁ is selected from saturated C₃-C₆ cycloalkylene optionally substituted with 1, 2, or 3 R^{2a};
X₂ is selected from -(C(R^{1a})(R^{1b}))ₘ₋CH₂₋;
m is selected from 1 or 2;
R^{1a} and R^{1b} are each independently hydrogen, halogen, or C₁-C₆ alkyl optionally substituted with 1, 2, or 3 halogens;
R^{2a} is selected from halogen, hydroxyl, amino, and C₁-C₆ alkyl, the C₁-C₆ alkyl being optionally substituted with one or more halogens.

In some embodiments, provided is the bispecific antibody-drug conjugate of the present disclosure, being selected from the following: 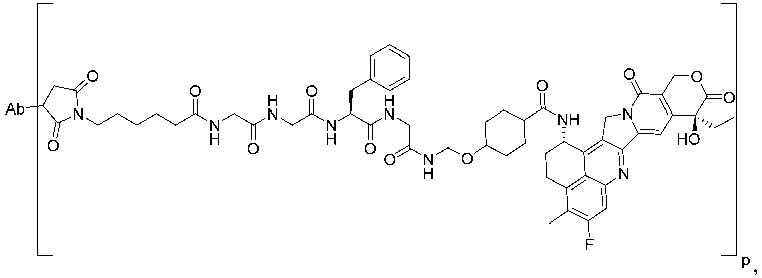 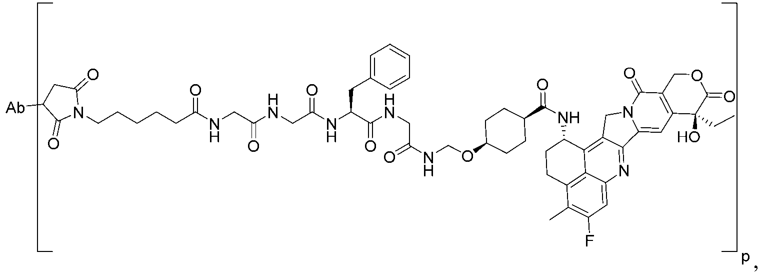 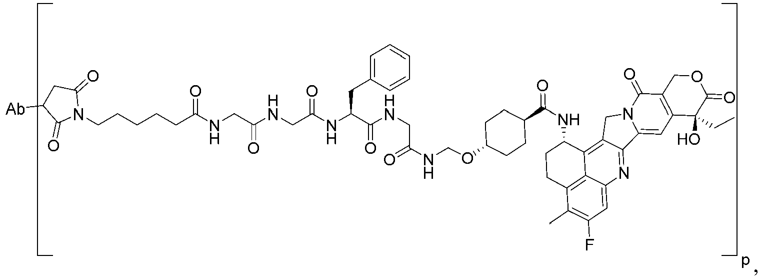 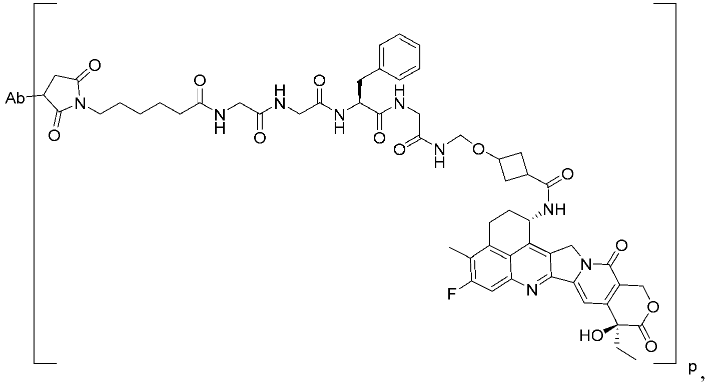 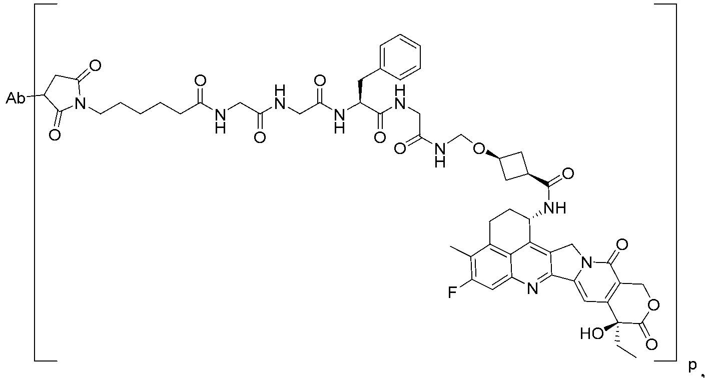 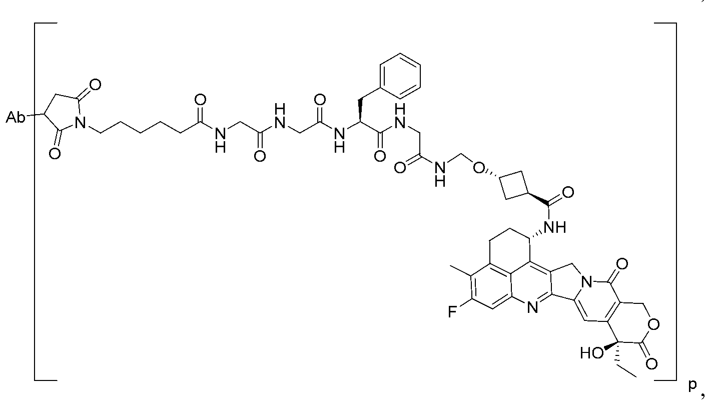 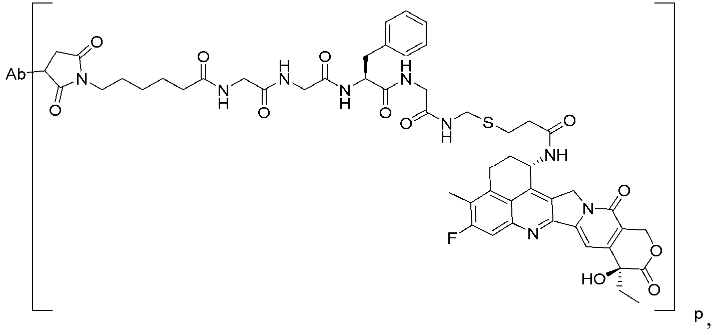 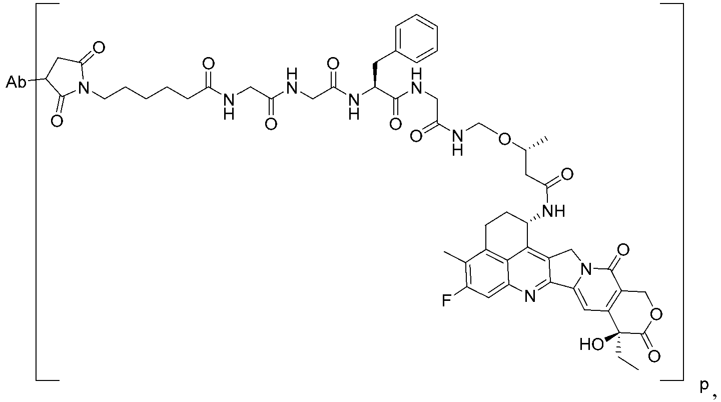 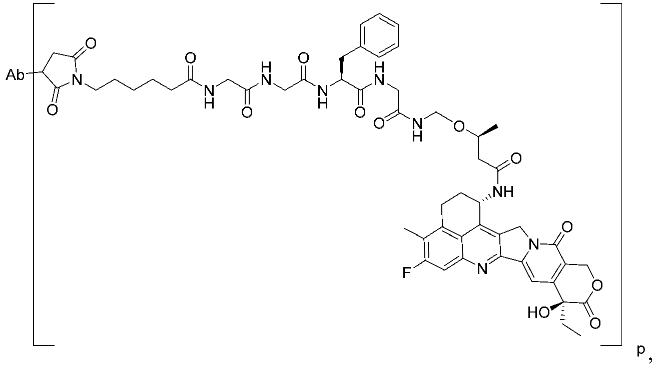 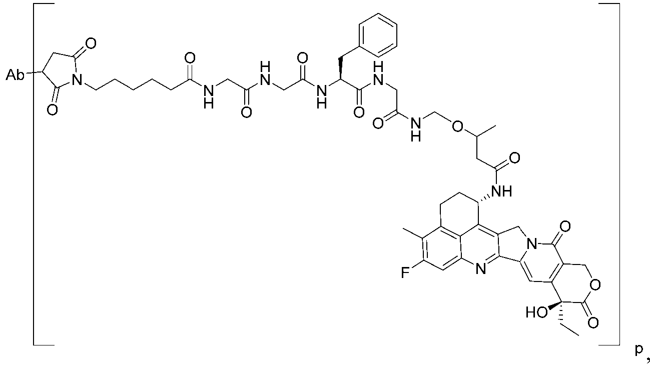 and 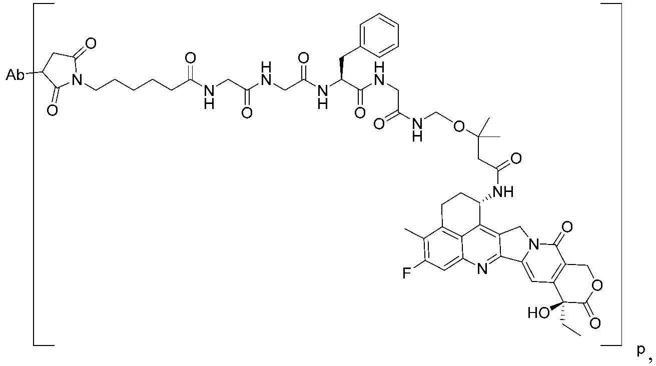 wherein
p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8;
Ab is the bispecific antibody as described in any one of the embodiments of the present disclosure or an antigen-binding fragment thereof.

In some embodiments, Ab is selected from the bispecific antibodies of the DBXT001 series (DBXT001-01 to 08), the DBXT002 series (DBXT DBXT002-01 to 08), the DBXT003 series (DBXT003-01 to 08), and the DBXT004 series (DBXT004-01 to 08) comprising the EGFR-binding domain and the HER3-binding domain of the present disclosure; preferably, Ab is selected from the bispecific antibodies of the DBXT001 series, the DBXT002 series, and DBXT005-01 comprising the EGFR-binding domain and the HER3-binding domain of the present disclosure; more preferably, Ab is selected from the bispecific antibodies of the DBXT001 series and DBXT005-01 comprising the EGFR-binding domain and the HER3-binding domain of the present disclosure; further preferably, Ab is selected from the bispecific antibodies DBXT001-01 and DBXT005-01 comprising the EGFR-binding domain and the HER3-binding domain of the present disclosure.

In some embodiments, Ab is the bispecific antibody DBXT005-01 comprising the EGFR-binding domain and the HER3-binding domain of the present disclosure.

In some embodiments, the bispecific antibody-drug conjugate is selected from any one of the following structures: 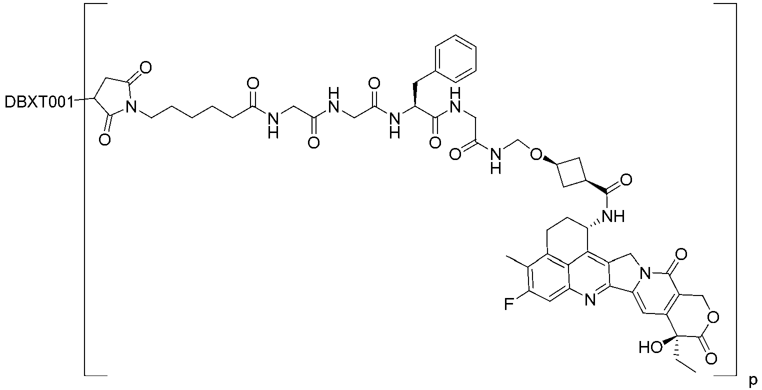 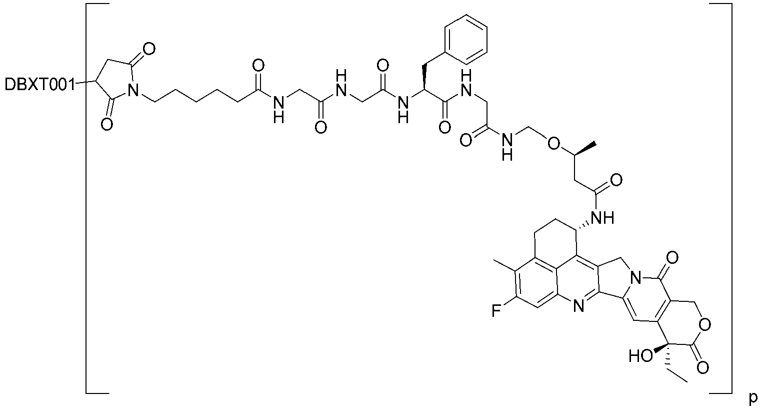 wherein p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8. In some embodiments, the bispecific antibody-drug conjugate is selected from any one of the following structures:

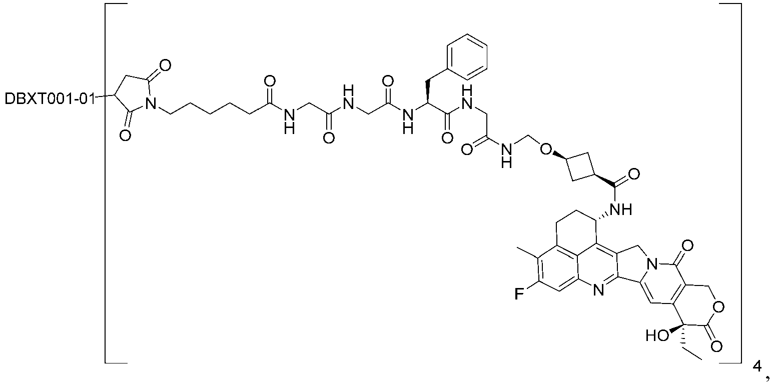

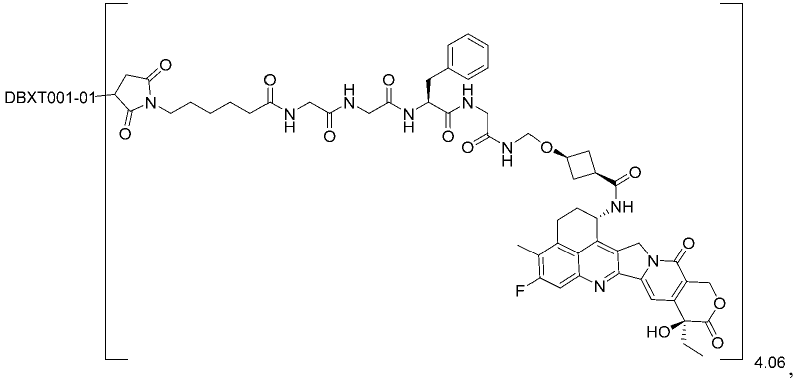

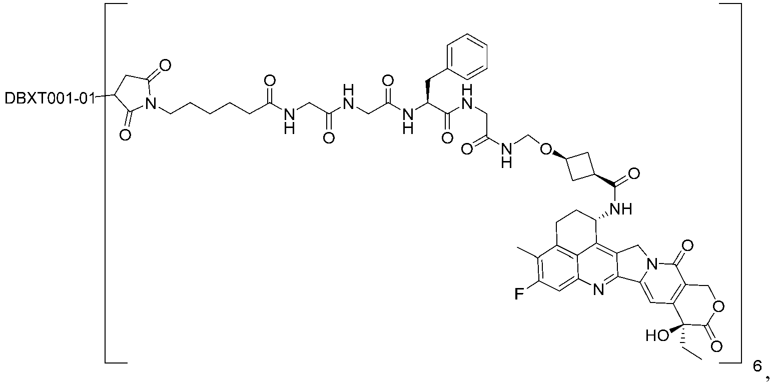

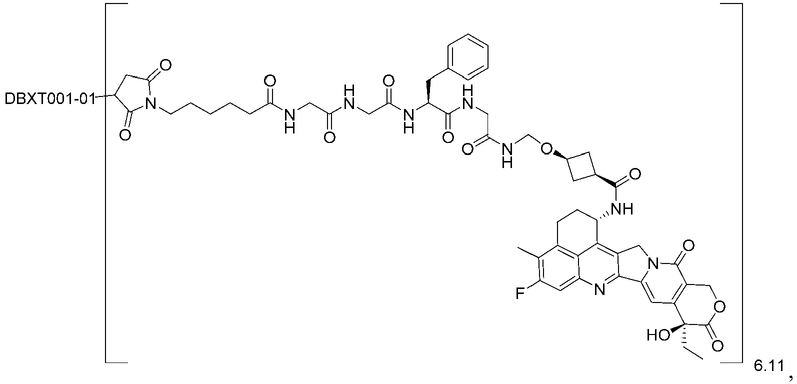

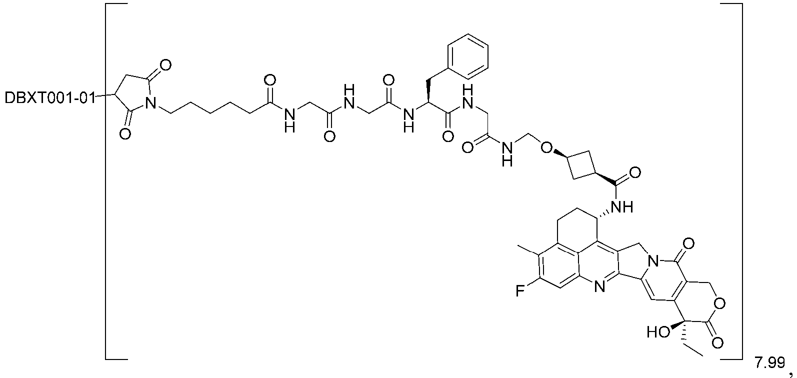

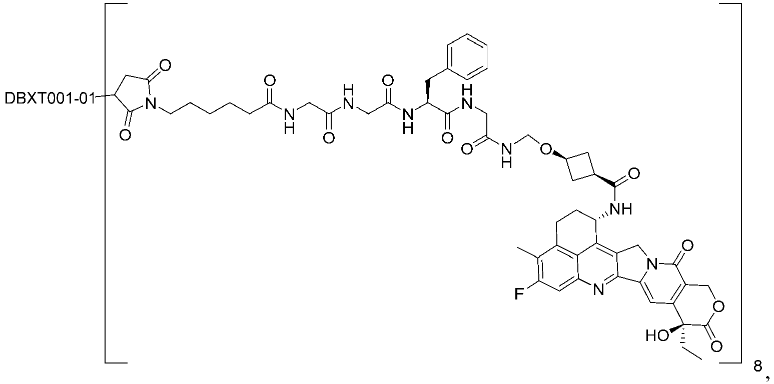

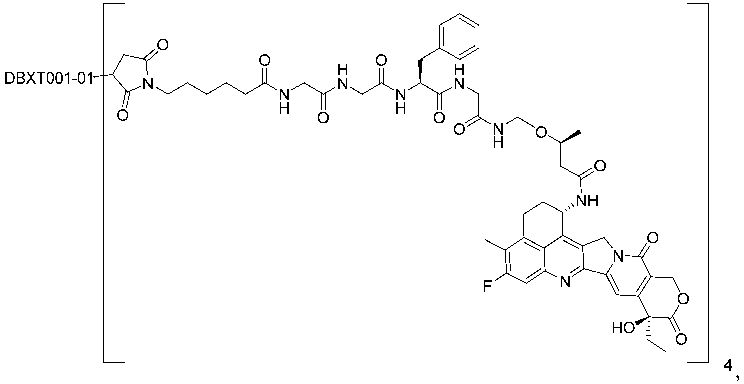

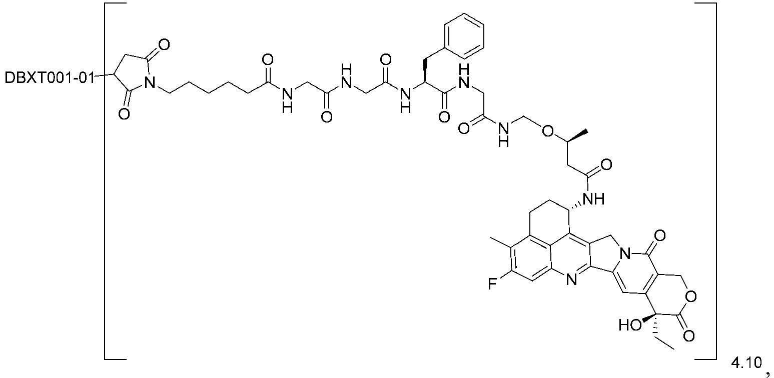

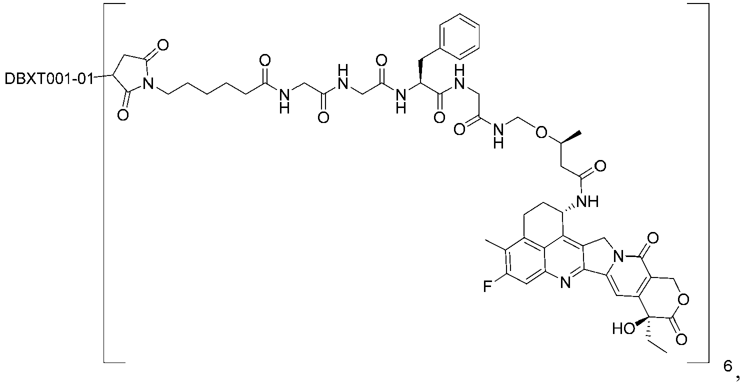

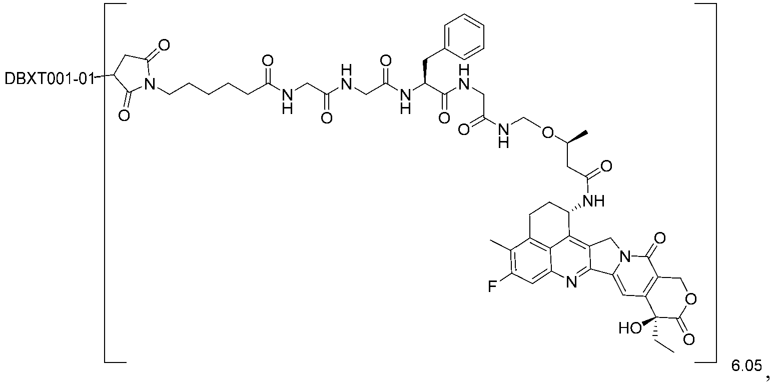

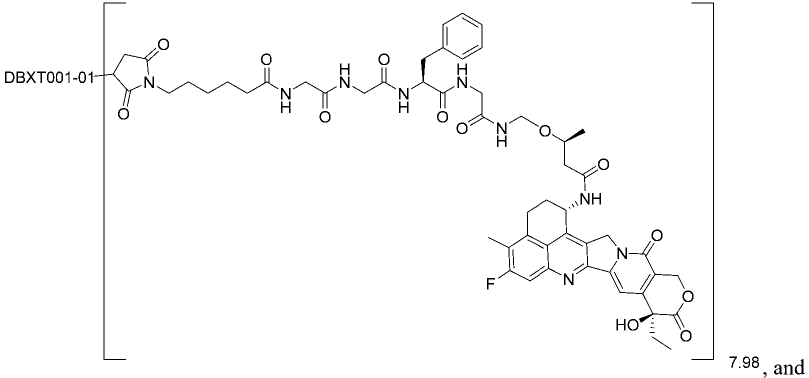

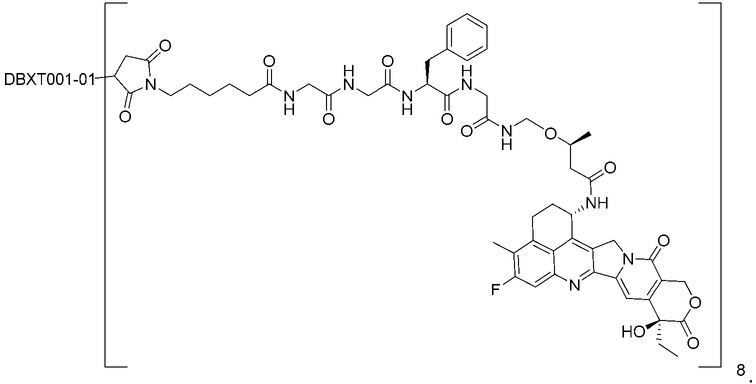

The amino acid sequences of the bispecific antibodies of DBXT001 (DBXT001-01 to 08), DBXT002 (DBXT002-01 to 08), DBXT003 (DBXT003-01 to 08), and DBXT004 (DBXT004-01 to 08) comprising the EGFR-binding domain and the HER3-binding domain of the present disclosure are set forth in the sequence listing of the present disclosure.

The antibody CDRs of the present disclosure are numbered using the Kabat numbering method.

In some embodiments, the bispecific antibody-drug conjugate is: 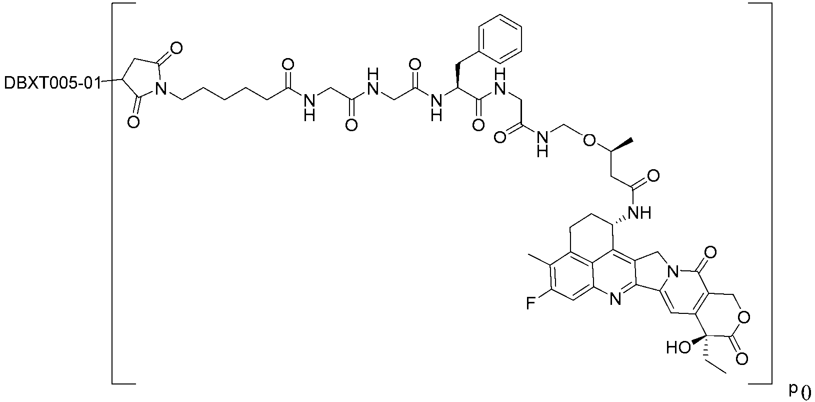 wherein p represents an average connection number, and p is any one of integers or decimals from 1 to 10, preferably any one of integers or decimals from 3 to 9, and more preferably any one of integers or decimals from 4 to 6, such as 5.99;
DBXT005-01 is an anti-EGFR/HER3 bispecific antibody, comprising a heavy chain H1 with the amino acid sequence set forth in SEQ ID NO: 37, a light chain L1 with the amino acid sequence set forth in SEQ ID NO: 38, and a heavy chain H2 with the amino acid sequence set forth in SEQ ID NO: 90.

In some embodiments, the bispecific antibody-drug conjugate is selected from the following conjugate: 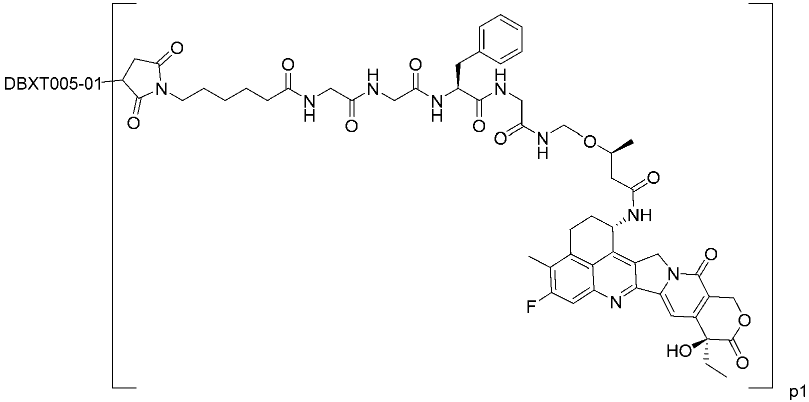 wherein p1 represents a connection number, and p1 is any one of integers from 1 to 10, preferably any one of integers from 3 to 9, and more preferably any one of integers from 4 to 6, such as 4, 5, or 6.

DBXT005-01 is an anti-EGFR/HER3 bispecific antibody, comprising a heavy chain H1 with the amino acid sequence set forth in SEQ ID NO: 37, a light chain L1 with the amino acid sequence set forth in SEQ ID NO: 38, and a heavy chain H2 with the amino acid sequence set forth in SEQ ID NO: 90.

In some embodiments, the average connection number p of the present disclosure may be any one of integers or decimals from 1 to 10. For example, the average connection number p may be any one of integers or decimals from 3 to 9. For example, the average connection number p may be any one of integers or decimals from 1 to 2, 2 to 3, 3 to 4, 4 to 5, 5 to 6, 6 to 7, 7 to 8, 8 to 9, or 9 to 10. Preferably, the average connection number p is 4, 4.06, 4.10, 6, 6.11, 6.05, 7.99, 7.98, or 8.

In some embodiments, the connection number p1 of the present disclosure is any one of integers from 1 to 10. For example, the connection number p1 is any one of integers from 3 to 9. For example, the connection number p1 is 1, 2, 3, 4, 5, 6, 7, 8, 9, or 10. Preferably, the connection number p1 is 4, 5, or 6.

In yet another aspect, the present disclosure provides a method for preparing the bispecific antibody-drug conjugate as described in any one of the embodiments of the present disclosure, comprising the following step: under the action of a reducing agent, mixing the bispecific antibody dissolved in a buffer with the linker unit L-cytotoxic drug dissolved in a solvent to obtain the bispecific antibody-drug conjugate.

In some embodiments, the preparation method comprises reacting the anti-EGFR/HER3 bispecific antibody with a compound of formula X2, for example, reacting DBXT005-01 with a compound of formula X2:

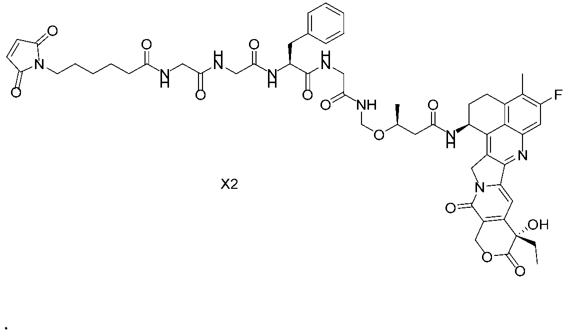

In some embodiments, the reducing agent is a conventional reducing agent for such reactions in the art, such as tris(2-carboxyethyl)phosphine hydrochloride.

In some embodiments, the buffer is a conventional buffer for such reactions in the art, such as ethylenediaminetetraacetic acid.

In some embodiments, the solvent is a conventional solvent for such reactions in the art, such as dimethylacetamide.

In still another aspect, the present disclosure provides a pharmaceutical composition, comprising the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, and/or the bispecific antibody-drug conjugate as described in any one of the embodiments of the present disclosure, and a pharmaceutically acceptable carrier or excipient. In still another aspect, the present disclosure provides a method for preparing the pharmaceutical composition of the present disclosure, comprising combining the bispecific antibody-drug conjugate as described in any one of the embodiments of the present disclosure, or a pharmaceutically acceptable form thereof, or a mixture thereof, with one or more pharmaceutically acceptable carriers or excipients.

In the present disclosure, suitable examples of the pharmaceutically acceptable carrier used in the pharmaceutical composition are as described in *Remington 's Pharmaceutical Sciences (2005).*

In the present disclosure, the pharmaceutical composition may be administered in any form as long as it achieves prevention, alleviation, inhibition, or cure of a symptom in a human or animal patient. For example, it may be formulated into various suitable dosage forms according to the administration route.

In other embodiments, the administration of the bispecific antibody-drug conjugate or the pharmaceutical composition as described in any one of the embodiments of the present disclosure may be combined with an additional treatment method. The additional treatment method may be selected from, but is not limited to: radiation therapy, chemotherapy, immunotherapy, or a combination thereof.

In still another aspect, the present disclosure provides a pharmaceutical formulation, comprising the bispecific antibody-drug conjugate as described in any one of the embodiments of the present disclosure, or a pharmaceutically acceptable form thereof, or a mixture thereof as an active ingredient, or the pharmaceutical composition as described in any one of the embodiments of the present disclosure. In some embodiments, the formulation is in the form of a solid formulation, a semi-solid formulation, a liquid formulation, or a gaseous formulation.

In still another aspect, the present disclosure provides use of the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure in the preparation of a medicament for treating and/or preventing a cancer, wherein preferably, the cancer is a cancer with positive expression of EGFR and/or HER3.

In still another aspect, the present disclosure provides a method for treating and/or preventing a cancer, comprising administering to a subject in need the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure, wherein preferably, the cancer is a cancer with positive expression of EGFR and/or HER3.

In still another aspect, the present disclosure provides the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure for use in the treatment and/or prevention of a cancer, wherein preferably, the cancer is a cancer with positive expression of EGFR and/or HER3.

In some embodiments, the cancer of the present disclosure is selected from breast cancer, skin cancer, gastric cancer, colorectal cancer, lung cancer, esophageal cancer, biliary tract cancer, head and neck cancer, thyroid cancer, ovarian cancer, endometrial cancer, pancreatic cancer, prostate cancer, bladder cancer, gastrointestinal cancer, digestive tract cancer, cervical cancer, squamous cell carcinoma, peritoneal cancer, liver cancer, kidney cancer, thyroid cancer, leukemia, malignant lymphoma, plasmacytoma, myeloma, glioma, osteosarcoma, sarcoma, oral squamous cell carcinoma, and melanoma.

In some embodiments, the cancer of the present disclosure is preferably selected from breast cancer, colorectal cancer, skin cancer, lung cancer, esophageal cancer, and oral squamous cell carcinoma.

In some embodiments, the lung cancer is preferably non-small cell lung cancer, the skin cancer is preferably cutaneous squamous cell carcinoma, and the colorectal cancer is preferably rectal cancer.

In still another aspect, the present disclosure provides use of the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure in the preparation of EGFR and/or HER3 inhibitors.

In some embodiments, the administration routes of the present disclosure include but are not limited to oral administration, intravenous administration, subcutaneous administration, intramuscular administration, intraarterial administration, intra-articular administration (e.g., in arthritic joints), administration by inhalation or aerosol delivery, intratumoral administration, and the like.

In some embodiments, the present disclosure provides co-administering to a subject a therapeutically effective amount of one or more therapies (e.g., therapeutic modalities and/or additional therapeutic agents). In some embodiments, the therapies include surgical treatment and/or radiation therapy.

In some embodiments, the method or use provided in the present disclosure further comprises administering to the individual one or more therapies (e.g., therapeutic modalities and/or additional therapeutic agents). The antibody-drug conjugate or the pharmaceutically acceptable salt thereof of the present disclosure may be used alone or in combination with an additional therapeutic agent in a therapy. For example, it may be co-administered with at least one additional therapeutic agent.

In still another aspect, the present disclosure provides a pharmaceutical combination, comprising the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure, as well as one or more additional therapeutic agents.

In still another aspect, the present disclosure provides a kit, comprising the bispecific antibody as described in any one of the embodiments of the present disclosure, the isolated nucleic acid as described in any one of the embodiments of the present disclosure, the recombinant expression vector as described in any one of the embodiments of the present disclosure, the transformant as described in any one of the embodiments of the present disclosure, the bispecific antibody-drug conjugate as described in any one of the embodiments of the present disclosure, and/or the pharmaceutical composition as described in any one of the embodiments of the present disclosure.

### Definitions of Terms

Unless otherwise stated, embodiments of the present disclosure will employ conventional technologies of molecular biology (including recombinant technology), microbiology, cytobiology, biochemistry, and immunology, which are all within the skill of the art.

Unless otherwise specifically defined herein, all technical and scientific terms used herein have the meanings as commonly understood by those of ordinary skill in the art to which the present disclosure relates. For definitions and terminology in the art, professionals can refer specifically to Current Protocols in Molecular Biology (Ausubel). Abbreviations for amino acid residues are standard 3-letter and/or 1-letter codes used in the art to denote one of the 20 commonly used L-amino acids.

In order to facilitate the understanding of the present disclosure, some technical and scientific terms are specifically defined as follows.

In the present disclosure, the term "EGFR (epidermal growth factor receptor)" refers to a receptor for cell proliferation and signaling of an epidermal growth factor (EGF). EGFR is a member of the ErbB receptor family, which includes EGFR (ErbB-1), HER2/c-neu (ErbB-2), HER3 (ErbB-3), and HER4 (ErbB-4). EGFR is also known as HER1 or ErbB-1. Mutations or overexpression of EGFR generally cause tumors. EGFR is a glycoprotein, which is a tyrosine kinase-type receptor. It spans the cell membrane and has a molecular weight of 170 kDa. EGFR is located on the surface of the cell membrane and is activated by binding to ligands, including EGF and TGFα (transforming growth factor α). Upon activation, EGFR transforms from a monomer to a dimer, although there is also evidence indicating that dimers exist prior to activation. EGFR may also dimerize with other members of the ErbB receptor family, such as ErbB-2/HER2/neu, to become activated. In the present disclosure, the term "human epidermal growth factor receptor 3 (HER3)", also known as receptor tyrosine-protein kinase ErbB-3 (ErbB3), is a member of the EGFR/ErbB family. Unlike other ErbB family members HER2 and EGFR, HER3 itself does not have kinase activity. Thus, HER3 must bind to its kinase active member EGFR or HER2 to trigger its downstream activity as a heterodimer. Upon binding to its natural ligand NRG1, HER3 undergoes conformational changes, heterodimerization, and phosphorylation, and then activates MAPK, PI3K/Akt, and PLCγ through signal transduction.

In the present disclosure, the term "about", when used in combination with a numerical value, is intended to encompass the numerical values in a range from a lower limit less than the specified numerical value by 5% to an upper limit greater than the specified numerical value by 5%, including but not limited to +5%, ±2%, +1%, and ±0.1%, as these variations are suitable for implementing the disclosed methods.

In the present disclosure, the term "and/or" should be understood to mean any one of the options or a combination of any two or more of the options.

In the present disclosure, the term "or" should be understood as having the same meaning as "and/or" defined above. For example, when items in a list are separated, "or" or "and/or" should be interpreted as being inclusive, that is, including at least one number or one of a list of elements, but also including more than one, and, optionally, additional unlisted items. Only in cases where contrary terms such as "only one", "exactly one", or "consisting of ..." as used in the claims are explicitly indicated will it refer to the one number solely listed or one element of the list.

In the present disclosure, the words "a" and "an" should be understood to mean "at least one", unless the contrary is explicitly indicated in the context.

In the present disclosure, the term "antibody-drug conjugate" generally means that an antibody is linked to a biologically active cytotoxic drug by a stable linking unit. In the present disclosure, the "antibody-drug conjugate" may be a bispecific antibody-drug conjugate, which may mean that a bispecific antibody or an antigen-binding fragment thereof is linked to a biologically active cytotoxic drug fragment by a stable linking unit.

In the present disclosure, the term "cytotoxic drug" generally refers to a toxic drug, and the cytotoxic drug may have a chemical molecule within the tumor cell that is strong enough to disrupt its normal growth. Cytotoxic drugs can kill tumor cells at a sufficiently high concentration. The "cytotoxic drug" may include toxins such as small molecule toxins or enzymatically active toxins of bacterial, fungal, plant or animal origin, radioisotopes (e.g., At²¹¹, I¹³¹, I¹²⁵, Y⁹⁰, Re¹⁸⁶, Re¹⁸⁸, Sm¹⁵³, Bi²¹², P³², or radioisotopes of Lu) , toxic drugs, chemotherapy drugs, antibiotics, nucleolytic enzymes, or derivatives thereof. For example, the cytotoxic drug may be a toxic drug, including but not limited to camptothecin derivatives; for example, it may be the camptothecin derivative exatecan (chemical name: (1*S*,9*S*)-1-amino-9-ethyl-5-fluoro-2,3-dihydro-9-hydroxy-4-methyl-1H,12H-benzo[de]pyrano[3',4':6,7]imidazo[1,2-b]quinoline-10,13(9*H*,15*H*)-dione).

In the present disclosure, the term "antibody (Ab)" generally refers to an immunoglobulin that is reactive to a specified protein or peptide or a fragment thereof. The antibody can be an antibody of any class, including but not limited to IgG, IgA, IgM, IgD, and IgE, and of any subclass (e.g., IgG1, IgG2, IgG3, and IgG4). The antibody may have a heavy chain constant region selected from, for example, IgG1, IgG2, IgG3, or IgG4. The antibody may also have a light chain selected from, for example, kappa (x) or lambda (λ). The antibody of the present disclosure can be derived from any species. The term "antibody" may include intact polyclonal antibodies, intact monoclonal antibodies, multispecific antibodies (e.g., bispecific antibodies), chimeric antibodies, humanized antibodies, human antibodies, fusion proteins comprising an antibody, and any other modified immunoglobulin molecules, so long as the antibodies exhibit the desired biological activity.

In the present disclosure, the term "antigen-binding fragment" or "antigen-binding domain" generally refers to a portion of an antibody molecule that comprises amino acids responsible for specific binding of the antibody to an antigen. The portion of the antigen specifically recognized and bound by the antibody is referred to as the "epitope" described herein. As described herein, the antigen-binding domain may typically comprise an antibody light chain variable region (VL) and an antibody heavy chain variable region (VH); however, it does not necessarily have to comprise both. An Fd fragment, for example, has two VH regions and typically retains some of the antigen-binding functions of the intact antigen-binding domain. Examples of antigen-binding fragments of antibodies include: (1) a Fab fragment, a monovalent fragment having a VL, a VH, a constant light chain (CL) and a CH1 domain; (2) a F(ab')₂ fragment, a bivalent fragment having two Fab fragments linked by a disulfide bridge in the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli", Nature 341: 544-546 (1989), which is incorporated herein by reference in its entirety), having a VH domain; (6) an isolated complementarity-determining region (CDR); and (7) a single-chain Fv (scFv), e.g., derived from an scFV-library; although the two domains of the Fv fragment, VL and VH, are encoded by separate genes, they may be joined by a recombinant method using a synthetic linker that allows them to be prepared as a single protein chain in which the VL and VH regions pair to form a monovalent molecule (known as single-chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85: 5879-5883 (1988)); and (8) "VHH", which relates to a variable antigen-binding domain of a heavy chain antibody from Camelidae (camel, dromedary, llama, alpaca, etc.) (see Nguyen V.K. et al., 2000, The EMBO Journal, 19, 921-930; Muyldermans S., 2001, J Biotechnol., 74, 277-302; and a review of Vanlandschoot P. et al., 2011, Antiviral Research 92, 389-407). VHH may also be referred to as nanobody (Nb).

In the present disclosure, the term "variable region" or "variable domain" or "variable antigen-binding domain" generally refers to a domain of a heavy or light chain of an antibody that is involved in the binding of the antibody to an antigen. In the present disclosure, the term "variable" generally means that certain portions of the sequences of the variable domains of antibodies vary considerably, resulting in the binding and specificity of various particular antibodies to their particular antigens. Variability is not evenly distributed throughout the variable regions of the antibody. It is concentrated in three segments in each of the light and heavy chain variable regions known as complementarity-determining regions (CDRs) or hypervariable regions (HVRs), namely LCDR1, LCDR2, LCDR3, HCDR1, HCDR2, and HCDR3. The more highly conserved portions of the variable regions are known as framework regions (FRs). The variable domains of native heavy and light chains each comprise four FRs (H-FR1, H-FR2, H-FR3, H-FR4, L-FR1, L-FR2, L-FR3, and L-FR4) largely in a β-sheet configuration. The FRs are connected by three CDR structural loop regions. The CDRs in each chain are held together in close proximity by the FRs and, together with the CDRs from the other chain, form the antigen-binding sites of the antibody.

In the present disclosure, the variable regions of an antibody can be encoded or the CDRs of an antibody can be divided by various methods, such as the Kabat numbering scheme and definition rule based on sequence variability (see Kabat et al., Sequences of Proteins of Immunological Interest, fifth edition, National Institutes of Health, Bethesda, Md. (1991)), the Chothia numbering scheme and definition rule based on the positions of structural loop regions (see A1-Lazikani et al., J Mol Biol 273: 927-48, 1997), the IMGT numbering scheme and definition rule based on alignment of amino acid sequences of germline V genes by Lefranc et al., as well as the Honneger's numbering scheme (AHo's), the Martin numbering scheme, the Gelfand numbering scheme, etc., see Mathieu Dondelinger et al., Understanding the Significance and Implications of Antibody Numbering and Antigen-Binding Surface/Residue Definition, Front. Immunol., October 16, 2018.

In the present disclosure, the term "monoclonal antibody" or "mAb" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

In the present disclosure, the term "multispecific antibody" refers to an antibody comprising two or more antigen-binding domains, capable of binding to two or more different epitopes (e.g., two, three, four, or more different epitopes) which may be on the same antigen or different antigens. Examples of multispecific antibodies include "bispecific antibodies" (abbreviated as BsAbs) or "bispecific molecules" that bind to two different antigens or two different epitopes. The bispecific antibody targeting EGFR and HER3 herein may be referred to, for example, as an "anti-EGFR/HER3" or "EGFR×HER3" bispecific molecule, or a bispecific antibody comprising an EGFR-binding domain and an HER3-binding domain, or other similar terms.

In the present disclosure, the term "Fc region" is used herein to define the C-terminal region of an immunoglobulin heavy chain that comprises at least a portion of the constant region. The term includes Fc regions of native sequences and variant Fc regions. In some embodiments, the Fc region of a human IgG heavy chain extends from Cys226 or Pro230 to the carboxyl-terminus of the heavy chain. However, the C-terminal lysine (Lys447) of the Fc region may or may not exist (numbering in this paragraph is according to the EU numbering system, also known as the EU index, Kabat et al., Sequences of Proteins of Immunological Interest, 5th Ed. Public Health Service, National Institutes of Health, Bethesda, MD, 1991).

In the present disclosure, the term "knob-into-hole structure" refers to the mutation of the hydrophobic amino acids of CH3 of the antibody Fc. A side-chain amino acid of CH3 of one chain is mutated to form a larger hydrophobic amino acid molecule (knob) to enhance the hydrophobic force. A side-chain amino acid of CH3 of another chain is mutated to form a smaller amino acid (hole) to reduce steric hindrance. After the mutations, the CH3 with the knob and the CH3 with the hole form a knob-into-hole structure (KiH) in a hydrophobic manner, which facilitates the formation of a heavy chain heterodimer. The KiH mutations mainly occur at internal hydrophobic amino acids of the spatial structure of the CH3 domain, and the amino acids exposed to the outside are barely changed after the mutations, so that the effector function of Fc and the induced immunogenicity are not affected. The term "knob-Fc" refers to the inclusion of a point mutation of T366W in the Fc region of an antibody to form a knob-like spatial structure. Correspondingly, "hole-Fc" refers to the inclusion of point mutations of T366S, L368A and Y407V in the Fc region of an antibody to form a hole-like spatial structure. Point mutations of S354C and Y349C can further be introduced into knob-Fc and hole-Fc, respectively, to further promote the formation of heterodimers through disulfide bonds. At the same time, point mutations of H435R and Y436F can further be introduced into hole-Fc to reduce the binding to protein A.

In the present disclosure, the term "humanized antibody" refers to an antibody form containing sequences from both human and non-human (e.g., mouse and rat) antibodies. In general, a humanized antibody comprises substantially all of at least one, and typically two, variable domains, where all or substantially all of the variable domains correspond to those of a non-human immunoglobulin, while all or substantially all of the framework regions (FRs) are those of a human immunoglobulin sequence. The humanized antibody may optionally comprise at least a portion of a human immunoglobulin constant region (Fc).

In the present disclosure, "isotypes" of antibodies refer to types of antibodies (e.g., IgM, IgE, and IgG (such as IgG1, IgG2, or IgG4)) provided by heavy chain constant region genes. Isotypes also include modified forms of one of these types in which modifications have been made to alter Fc function, for example, to enhance or attenuate effector function or binding to Fc receptors.

In the present disclosure, the term "cross-reaction" refers to binding to antigenic fragments of the same target molecule of human, monkey and/or murine (mouse or rat) origin. Thus, "cross-reaction" should be understood as an interspecies reaction of an antigen-binding molecule (e.g., an antibody) with a similar molecule expressed in a different species (e.g., BDCA2). The cross-reaction specificity of monoclonal antibodies recognizing human BDCA2, and monkey and/or murine (mouse or rat) BDCA2 can be determined by FACS analysis.

In the present disclosure, "affinity" or "binding affinity" refers to the inherent binding affinity that reflects the interaction between the members involved in the binding. The affinity of molecule X for its partner Y can be generally represented by the equilibrium dissociation constant (KD), which is a ratio of the dissociation rate constant to the association rate constant (K_{off} (Kd) and Kₒₙ (Ka), respectively). Affinity can be measured by common methods known in the art. In some embodiments of the present disclosure, the affinity, e.g., between the antibody of the present disclosure and an antigen, is measured using the surface plasmon resonance (SPR) technology. In some preferred embodiments of the present disclosure, one specific method for measuring affinity is the BIAcore method.

In the present disclosure, the term "not bind to" a protein or cell means not binding to the protein or cell, or not binding to it with high affinity, that is, binding to the protein or cell with a KD of 1.0 × 10⁻⁶ M or higher, preferably 1.0 × 10⁻⁵ M or higher, more preferably 1.0 × 10⁻⁴ M or higher or 1.0 × 10⁻³ M or higher, and further preferably 1.0 × 10⁻² M or higher.

In the present disclosure, the term "high affinity" of IgG antibodies refers to a KD for an antigen of 1.0 × 10⁻⁶ M or lower, preferably 5.0 × 10⁻⁸ M or lower, more preferably 1.0 × 10⁻⁸ M or lower or 5.0 × 10⁻⁹ M or lower, and further preferably 1.0 × 10⁻⁹ M or lower. For other antibody subtypes, "high-affinity" binding may vary. For example, "high-affinity" binding of the IgM subtype refers to a K_{D} of 10⁻⁶ M or lower, preferably 10⁻⁷ M or lower, and more preferably 10⁻⁸ M or lower.

In the present disclosure, the term "percent (%) amino acid sequence identity" or simply "identity" is defined as the percentage of amino acid residues in a candidate amino acid sequence that are identical to those in a reference amino acid sequence after aligning the amino acid sequences (with gaps introduced if necessary) to achieve the maximum percent sequence identity without considering any conservative substitution as part of the sequence identity. Sequence alignment for the purpose of determining percent amino acid sequence identity can be performed using various methods in the art, for example, using computer software available to the public, such as the BLAST, BLAST-2, ALIGN or MEGALIGN (DNASTAR) software. Those skilled in the art can determine suitable parameters for measuring alignment, including any algorithm required to achieve the maximum alignment over the full length of the aligned sequences.

In the present disclosure, the term "halogen" generally refers to fluorine, chlorine, bromine, or iodine, and it may be, for example, fluorine or chlorine.

In the present disclosure, the term "alkyl" generally refers to a residue derived from an alkane by the removal of a hydrogen atom. The term "alkyl" generally refers to a saturated linear or branched aliphatic hydrocarbon group having a residue derived from a parent alkane by the removal of hydrogen atoms from the same carbon atom or two different carbon atoms, and it may be a linear or branched group containing 1 to 20 carbon atoms (e.g., 1 to 12 carbon atoms), such as an alkyl chain containing 1 to 6 carbon atoms (e.g., an alkyl chain containing 1 to 3 carbon atoms). Non-limiting examples of alkyl include but are not limited to methyl, ethyl, propyl, propyl, butyl, and the like. Alkyl may be substituted or unsubstituted, replaced or unreplaced; for example, when it is substituted, substitution with a substituent may occur at any available point of attachment. In the present disclosure, the term "alkylene" generally refers to a saturated linear or branched aliphatic hydrocarbon group having 2 residues derived from a parent alkane by the removal of two hydrogen atoms from the same carbon atom or two different carbon atoms, and it may be a linear or branched group containing 1 to 20 carbon atoms; for example, the term "methylene" may refer to a residue derived from a one-carbon atom group by the removal of two hydrogen atoms. Methylene may be substituted or unsubstituted, or replaced or unreplaced. For example, alkylene contains 1 to 12 carbon atoms, e.g., alkylene containing 1 to 6 carbon atoms. Non-limiting examples of alkylene include but are not limited to methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,5-butylene (-CH₂CH₂CH₂CH₂CH₂-), and the like.

In the present disclosure, the term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms, preferably 3 to 10 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "cycloalkylene" is a divalent group that is connected to the rest of the molecule by two single bonds, with the rest of the definition being the same as that for the term "cycloalkyl".

In the present disclosure, the term "partially unsaturated" generally means that the cyclic structure contains at least one double or triple bond between the ring molecules. The term "partially unsaturated" encompasses cyclic structures having multiple sites of unsaturation, but is not intended to include aromatic or heteroaromatic rings defined herein. The term "unsaturated" means that the moiety has one or more degrees of unsaturation. In the present disclosure, the term "heterocyclyl" refers to a saturated or partially unsaturated monocyclic or polycyclic light substituent containing 3 to 20 ring atoms, where one or more of the ring atoms are heteroatoms selected from nitrogen, oxygen, and sulfur, and the remaining ring atoms are carbon atoms. Preferably, heterocyclyl contains 3 to 12 ring atoms, 1 to 4 of which are heteroatoms; more preferably, heterocyclyl contains 3 to 8 ring atoms, 1 to 3 of which are heteroatoms; further preferably, heterocyclyl contains 3 to 6 ring atoms, 1 to 3 of which are heteroatoms; most preferably, heterocyclyl contains 5 or 6 ring atoms, 1 to 3 of which are heteroatoms. Non-limiting examples of monocyclic heterocyclyl include pyrrolidinyl, tetrahydropyranyl, piperidinyl, morpholinyl, thiomorpholinyl, homopiperazinyl, and the like. Non-limiting examples of polycyclic heterocyclyl include spiro heterocyclyl, fused heterocyclyl, and bridged heterocyclyl. The heterocyclyl ring may be fused to an aryl, heteroaryl or cycloalkyl ring, and the ring connected to the parent structure is heterocyclyl.

The term "heteroalkylene" is a divalent group that is connected to the rest of the molecule by two single bonds, with the rest of the definition being the same as that for the term "cycloalkylene".

In the present disclosure, the term "each independently" generally means that a variable applies in any case irrespective of the presence or absence of variables having the same definition or different definitions in the same compound. For example, the variable may refer to the type or number of substituents in the compound, the type of atoms in the compound, and so on. For example, where R occurs twice in a compound and R is defined as "independently carbon or nitrogen", both R may be carbon, both R may be nitrogen, or one R may be carbon while the other R is nitrogen.

In the present disclosure, the term "optional" or "optionally" generally means that the event or circumstance subsequently described may, but not necessarily, occur, and that the description includes instances where the event or circumstance occurs or does not occur. For example, "heterocyclyl group optionally substituted with alkyl" means that alkyl substitution may be, but not necessarily, present, and that the description can include instances where the heterocyclyl group is or is not substituted with alkyl.

In the present disclosure, the term "substituted" generally means that one or more hydrogen atoms in the group, for example, up to 5 (e.g., 1 to 3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxyl having free hydrogen is bound to a carbon atom having an unsaturated (e.g., olefinic) bond.

In the present disclosure, unless otherwise specified, the "linking" of a group to a group may generally be in any orientation; the "linking" of group X to group Y may generally be in any orientation, which generally means that when group X is used for linker Y and group Z, two or more linking sites of the group X may be linked arbitrarily to either group Y or group Z.

In the present disclosure, as known to those skilled in the art, the terms such as "alkyl" and "cycloalkyl" may be preceded by a notation to indicate the number of atoms present in the group under particular circumstances as in C₁-C₄ alkyl, C₃-C₇ cycloalkoxy, C₁-C₄ alkylcarbonylamino, and the like, and the subscript numeral following "C" indicates the number of carbon atoms present in the group. For example, C₃ alkyl refers to an alkyl group containing three carbon atoms (e.g., n-propyl or isopropyl); in C₁₋₁₀, members of the group may have any number of carbon atoms within the range of 1-10.

In the present disclosure, the compound or antibody-drug conjugate of the present disclosure includes tautomers, mesomers, racemates, enantiomers, and/or diastereoisomers thereof. In the present disclosure, the term "diastereoisomer" generally refers to a stereoisomer that has two or more chiral centers and whose molecules are not mirror images of each other. Diastereoisomers may have different physical properties, e.g., melting points, boiling points, spectral properties, and reactivities. In the present disclosure, the terms "tautomer" and "tautomeric form" are used interchangeably and generally refer to structural isomers of different energies that can be converted into each other by crossing a low energy barrier. For example, proton tautomers (also known as prototropic tautomers) include interconversions by proton migration, such as keto-enol isomerization and imine-enamine isomerization. Valence tautomers include interconversions by recombination of some bonding electrons. In the present disclosure, the term "mesomer" generally means that the molecule contains asymmetric atoms but has a total optical rotation of zero due to the presence of symmetric factors. The term "racemate" or "racemic mixture" refers to a composition of two enantiomeric substances in equimolar amounts.

In the present disclosure, the term "linker unit" or "linker structure" generally refers to a chemical structural fragment or bond that is linked to a ligand at one end and to a cytotoxic drug at the other end, or that is linked to other linkers before being linked to the cytotoxic drug. The direct or indirect linking to a ligand may mean that the group is directly linked to the ligand by a covalent bond, and may also be linked to the ligand by a linker structure. For example, a chemical structural fragment or bond containing an acid-labile linker structure (e.g., hydrazone), a protease-sensitive (e.g., peptidase-sensitive) linker structure, a photolabile linker structure, a dimethyl linker structure or a disulfide-containing linker structure may be used as a linker structure.

In the present disclosure, the term a structure being "optionally linked to other molecular moieties" generally means that the structure is not linked to any other chemical structure, or that the structure is linked (e.g., by a chemical bond or a linker structure) to one or more other chemical structures (e.g., ligands described herein) different from the structure.

In the present disclosure, the term "drug loading" generally refers to the average number of cytotoxic drug payloads loaded onto each ligand and may also be expressed as the amount ratio of cytotoxic drug to antibody (drug/antibody ratio, DAR), and the cytotoxic drug loading may range from 0 to 12 (e.g., 1-10) cytotoxic drugs per ligand (Ab). In the embodiments of the present disclosure, the drug loading is expressed as p or p1, and may illustratively be a mean of 1, 2, 3, 4, 5, 6, 7, 8, 9, and 10. The drug loading per ADC molecule after the conjugation reaction can be characterized by conventional methods such as UV/visible spectroscopy, mass spectrometry, ELISA, and HPLC.

In the present disclosure, certain atoms of the compound or antibody-drug conjugate of the present disclosure may be present in one or more isotopic forms. For example, hydrogen may occur as protium (¹H), deuterium (²H), and tritium (³H), and carbon may naturally occur as three different isotopes (¹²C, ¹³C, and ¹⁴C). Examples of isotopes that can be incorporated into the compounds of the present disclosure also include but are not limited to ¹⁵N, ¹³O, ¹⁷O, ¹⁸F, ³²P, ³³P, ¹²⁹I, ¹³¹I, ¹²³I, ¹²⁴I, ¹²⁵I, or similar isotopes. Thus, one or more of such isotopes may be enriched in the compound or antibody-drug conjugate of the present disclosure relative to the natural abundance of such isotopes. Such isotopically enriched compounds can be used for a variety of purposes, as known to those skilled in the art. For example, replacement with heavy isotopes such as deuterium (²H) may offer certain therapeutic advantages, possibly due to higher metabolic stability. For example, the natural abundance of deuterium (²H) is about 0.015%. Accordingly, one out of about 6500 hydrogen atoms is a deuterium atom. Thus, the deuterium abundance of one or more sites (as the case may be) in a deuterium-containing compound or antibody-drug conjugate of the present disclosure is greater than 0.015%. Unless otherwise indicated, the structures described herein may also include compounds or antibody-drug conjugates that differ only in the presence or absence of one or more isotopically enriched atoms. For example, compounds or antibody-drug conjugates having a structure identical to the structure disclosed herein except for the substitution of the hydrogen atom with deuterium or tritium or the substitution of the carbon atom by carbon 13 or carbon 14 shall fall within the scope of the present disclosure.

In the present disclosure, the term "pharmaceutical composition" generally refers to a mixture containing one or more of the compounds described herein or physiologically/pharmaceutically acceptable salts or pro-drugs thereof, and other chemical components such as physiologically/pharmaceutically acceptable carriers and excipients. The pharmaceutical composition can promote the administration to an organism and facilitate the absorption of the active ingredient, thereby exerting biological activity. The preparation of conventional pharmaceutical compositions can be found in various national pharmacopeias. The pharmaceutical composition may be in the form of a sterile injectable aqueous or oily suspension for intramuscular and subcutaneous administration. The suspension can be formulated according to a known technique using suitable dispersing agents or wetting agents and suspending agents. The sterile injectable formulation may also be a sterile injectable solution or suspension prepared in a parenterally acceptable non-toxic diluent or solvent, such as a solution prepared in 1,3-butanediol. In addition, a sterile fixed oil may be conveniently used as a solvent or a suspending medium. For example, any blend fixed oil including synthetic mono- or di-glycerides can be used. In addition, fatty acids such as oleic acid may also be used in the preparation of injections.

In the present disclosure, the term "pharmaceutically acceptable salt" generally refers to a salt of the compound or antibody-drug conjugate of the present disclosure, which may be safe and/or effective when used in a mammal and possess the desired biological activity; the compound or antibody-drug conjugate of the present disclosure can form a salt with an acid.

In the present disclosure, the term "pharmaceutically acceptable carrier" generally refers to a carrier or vehicle for providing therapeutic agents, such as antibodies or polypeptides, genes, and other therapeutic agents. The term refers to any pharmaceutical carrier that does not itself induce the production of antibodies harmful to the individual receiving the composition and can be administered without producing undue toxicity. Suitable carriers may be large, slow-metabolizing macromolecules, such as proteins, polysaccharides, polylactic acids, polyglycolic acids, polyamino acids, amino acid copolymers, lipid aggregates, and inactivated virus particles. Such carriers are well known to those skilled in the art. Pharmaceutically acceptable carriers in therapeutic compositions may include liquids such as water, saline, glycerol, and ethanol. Auxiliary substances, such as wetting agents or emulsifying agents, or pH buffering substances, may also be present in these carriers.

In the present disclosure, the terms "treatment" and "treating" generally refer to a method for achieving beneficial or desired results, including but not limited to therapeutic benefits. The therapeutic benefits include but are not limited to eradication, inhibition, reduction, or amelioration of the underlying disorder being treated. In addition, the therapeutic benefits are achieved by eradicating, inhibiting, reducing, or ameliorating one or more physiological symptoms associated with the underlying disorder, and thus improvements are observed in the patient, but the patient may still be afflicted with the underlying disorder.

In the present disclosure, the terms "prevention" and "preventing" generally refer to a method for achieving beneficial or desired outcomes, including but not limited to prophylactic benefits. For the purpose of prophylactic benefits, a pharmaceutical composition can be administered to a patient at risk of developing a particular disease, or to a patient who reports he/she has one or more physiological symptoms of the disease, even if the disease has not yet been diagnosed.

In the present disclosure, the term "subject" or "patient" generally refers to humans (i.e., males or females in any age group, e.g., pediatric subjects (e.g., infants, children, or adolescents) or adult subjects (e.g., young adults, middle-aged adults, or elderly adults)) and/or other primates (e.g., cynomolgus monkey or rhesus monkey); mammals, including commercially relevant mammals, such as cows, pigs, horses, sheep, goats, cats and/or dogs; and/or birds, including commercially relevant birds, such as chickens, ducks, geese, quail and/or turkeys.

In the present disclosure, the terms "therapeutically effective amount", "therapeutically effective dose" and "effective amount" refer to an amount of the compound or antibody-drug conjugate of the present disclosure that is effective in preventing or ameliorating one or more symptoms of a disease or condition or the development of the disease or condition, when administered alone or in combination with other therapeutic drugs to a cell, tissue or subject. The therapeutically effective dose also refers to a dose sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose only refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will result in an increase in a diagnostic index or parameter by at least 10%, generally at least 20%, preferably at least about 30%, more preferably at least 40%, and most preferably at least 50%.

In the present disclosure, the term "cancer" refers to a group of cells that exhibit abnormally high levels of proliferation and growth. Cancer may be benign (also known as benign tumor), premalignant, or malignant. Cancer cells may be solid cancer cells or leukemia cancer cells. In the present disclosure, the term "tumor" refers to one or more cells comprising cancer. In the present disclosure, the term "tumor growth" is used to refer to the proliferation or growth of one or more cells comprising cancer, which results in a corresponding increase in the size or extent of the cancer. The terms "cancer with positive expression of EGFR and/or HER3" and "cancer expressing EGFR and/or HER3" have the same definition and refer to cancer where the cancer cells express EGFR and/or HER3, preferably on the surface of the cancer cells.

The amino acid numbering of the antibodies of the present disclosure is based on the natural order from the N-terminus to the C-terminus according to the antibody sequence.

On the basis of the general knowledge in the art, the preferred conditions described above can be combined arbitrarily to obtain preferred embodiments of the present disclosure.

The reagents and starting materials used in the present disclosure are all commercially available.

The positive and progressive effects of the present disclosure are as follows:
The bispecific antibody of the present disclosure has the following advantages:
1. It can bind to both EGFR and HER3 antigens and EGFR/HER3 cell lines, demonstrating an anti-tumor activity superior to that of monoclonal antibody administration; compared to the parent mAbs and the control antibody SI-1X6.4, the bispecific antibody of the present disclosure exhibits a better endocytosis effect on the human tumor cell lines T47D and NCI-H1975, as well as the mouse cell line CT26, which express both EGFR and HER3.
2. Compared with combination therapy with the related monoclonal antibodies, the bispecific antibody of the present disclosure has the advantages of good compliance and controllable quality.
3. The stability characterization of the bispecific antibody in the present disclosure is mainly reflected in studies on monomer purity and thermal stability. After affinity purification and molecular sieve separation, the monomer content of the bispecific antibody can reach 95%. The structure and activity analysis results of the antibody after heat treatment demonstrate that the antibody can still maintain good molecular conformation and complete biological activity in harsh environments, which is beneficial for its industrial production, packaging, and storage. Overall, the anti-EGFR/HER3 bispecific antibody of the present disclosure exhibits good molecular stability and has significantly better *in vitro* activity (binding at the molecular and cellular levels) as compared to cetuximab and EGFR. *In vivo* data demonstrate that in EGFR + HER3 tumor cells, the anti-tumor activity of the bispecific antibody is superior to that of the combined administration group of EGFR and HER3 monoclonal antibodies. Thus, the bispecific antibody of the present disclosure has broad application prospects due to its excellent developability and activity.

The bispecific antibody-drug conjugate as described in any one of the embodiments of the present disclosure has an *in vivo* anti-tumor effect, which is specifically manifested as follows:
(1) it has a better anti-tumor effect on the human esophageal cancer cell line OE-19 with low EGFR expression and medium HER3 expression;
(2) it has a better anti-tumor effect on the human non-small cell lung cancer cell line NCI-H441 with medium EGFR expression and medium HER3 expression;
(3) it has a better anti-tumor effect on the human oral squamous cell carcinoma cell line CAL-27 with high EGFR expression and low HER3 expression;
(4) it has a better anti-tumor effect in a human esophageal cancer OE-19 subcutaneous xenograft model resistant to EGFR ADC;
(5) it has a better anti-tumor effect in a human skin cancer A431 subcutaneous xenograft model with high EGFR expression and low HER3 expression;
(6) it has a better anti-tumor effect in a human colon cancer SW620 subcutaneous xenograft model that does not express EGFR but only expresses HER3;
(7) it has a better anti-tumor effect in a human colon cancer SW48 subcutaneous xenograft model with medium EGFR expression and low HER3 expression; and
(8) it has a better anti-tumor effect in a non-small cell lung cancer NCI-H1975 (EGFR L858R/T790M/C797S triple mutation) subcutaneous xenograft model resistant to third-generation TKI.

### BRIEF DESCRIPTION OF THE DRAWINGS

FIG. 1 shows the binding of a bispecific antibody and its corresponding parent mAbs to cell lines with different levels of EGFR/HER3 expression.
FIG. 2 shows the binding of bispecific antibodies and their corresponding parent mAbs to cell lines with different levels of EGFR/HER3 expression.
FIG. 3 shows the endocytosis activity of a bispecific antibody and its corresponding parent mAbs (pHrodo method).
FIG. 4 shows the endocytosis activity of bispecific antibody and its corresponding parent mAbs and control antibody (incucyte method).
FIG. 5 shows the endocytosis activity of bispecific antibodies and their corresponding parent mAbs and control antibody (incucyte method).
FIG. 6 shows an evaluation of the *in vivo* efficacy of antibody-drug conjugates in mice bearing the human esophageal cancer cell line OE-19 with low EGFR expression and medium HER3 expression.
FIG. 7 shows an evaluation of the *in vivo* efficacy of antibody-drug conjugates in mice bearing the human non-small cell lung cancer cell line NCI-H441 with medium EGFR expression and medium HER3 expression.
FIG. 8 shows an evaluation of the *in vivo* efficacy of antibody-drug conjugates in mice bearing the human oral squamous cell carcinoma cell line CAL-27 with high EGFR expression and low HER3 expression.
FIG. 9 shows an evaluation of the *in vivo* efficacy of bispecific antibody-drug conjugates with different DAR values in NCI-H1975 tumor-bearing mice.
FIG. 10 shows an evaluation of the *in vivo* efficacy of bispecific antibody-drug conjugates with different DAR values in OE-19 tumor-bearing mice.
FIG. 11 shows a comparison of the *in vivo* efficacy of the bispecific antibody-drug conjugate of the present disclosure and control antibody-drug conjugates in NCI-H1975 tumor-bearing mice.
FIG. 12 shows a comparison of the *in vivo* efficacy of the bispecific antibody-drug conjugates of the present disclosure and control antibody-drug conjugates in OE-19 tumor-bearing mice.
FIG. 13 shows the *in vivo* efficacy of the conjugate of the bispecific antibody drug with linker-cytotoxin X1 of the present disclosure in NCI-H1975 tumor-bearing mice.
FIG. 14 shows the *in vivo* efficacy of the bispecific antibody-drug conjugate of the present disclosure in human esophageal cancer OE-19 tumor-bearing mice.
FIG. 15 shows the *in vivo* efficacy of the bispecific antibody-drug conjugate of the present disclosure in mice bearing human skin cancer A431 xenograft tumors with high EGFR expression and low HER3 expression.
FIG. 16 shows the *in vivo* efficacy of the bispecific antibody-drug conjugate of the present disclosure in mice bearing human colon cancer cell line SW620 xenograft tumors that do not express EGFR but only express HER3.
FIG. 17 shows the *in vivo* efficacy of the bispecific antibody-drug conjugate of the present disclosure in mice bearing human colon cancer cell line SW48 xenograft tumors with medium EGFR expression and low HER3 expression.
FIG. 18 shows the *in vivo* efficacy of the bispecific antibody-drug conjugate of the present disclosure in mice bearing non-small cell lung cancer NCI-H1975 (EGFR L858R/T790M/C797S triple mutation) xenograft tumors.

### DETAILED DESCRIPTION

The present disclosure is further illustrated by the following examples; however, these examples should not be construed as limiting the present disclosure. Experimental procedures without specified conditions in the following examples were performed in accordance with conventional procedures and conditions, or in accordance with product instructions.

### Sample assay

The present disclosure includes all combinations of the specific embodiments described. The full scope of further embodiments and applicability of the present disclosure will become apparent from the detailed description provided below. However, it should be understood that the detailed description and the specific examples, while indicating preferred embodiments of the present disclosure, are provided by way of illustration only, as various changes and modifications within the spirit and scope of the present disclosure will become apparent to those skilled in the art from the detailed description. All publications, patents and patent applications cited herein, including the citations, are hereby incorporated by reference in their entirety for all purposes.

### Examples

The following examples are provided to demonstrate and further illustrate some preferred embodiments and aspects of the present disclosure and should not be construed as limiting the scope of the present disclosure.

### Example 1: Preparation of Bispecific Antibodies

In the anti-EGFR×HER3 bispecific antibodies of the present disclosure, the anti-HER3 antibodies or antigen-binding fragments thereof were prepared with reference to clone 2 of hu3F8 (PCT/CN2022/098929), and the anti-EGFR antibodies or antigen-binding fragments thereof were prepared with reference to zalutumumab (WO2002100348). Meanwhile, mutations were made on some amino acids in the CDRs/FRs/Fe regions of these antibodies. The CDRs of the bispecific antibodies were determined according to the Kabat numbering scheme.

According to the amino acid sequences of the antibody variable regions, primers were designed for PCR to construct VH/VK gene fragments and thus obtain the variable regions. The antibody variable regions were then combined with the constant region gene fragments to construct an intact bispecific antibody sequence. After transfection in CHO cells, an anti-EGFR mAb and an anti-HER3 mAb, as well as bispecific antibodies DBXT001-01 to 08, DBXT002-01 to 08, DBXT003-01 to 08, DBXT004-01 to 08 and DBXT005-01, were obtained according to conventional expression and purification methods.

The mAb cetuximab was prepared using a conventional method; the control antibody SI-1X6.4 was prepared with reference to WO2023083381A1.

The names and sequences of the antibodies are shown in Table 1.

**Table 1. Amino acid sequences of antibodies**

| **Name of antibody** | **Heavy chain 1 (SEQ ID NO:)** | **Light chain 1 (SEQ ID NO:)** | **Heavy chain 2 (SEQ ID NO:)** | **Light chain 2 (SEQ ID NO:)** |
|---|---|---|---|---|
| anti-EGFR | 83 | 84 | / | / |
| anti-HER3 | 85 | 86 | / | / |
| Cetuximab | 87 | 88 | / | / |
| SI-1X6.4 | 91 | 92 | / | / |
| DBXT001-01 | 37 | 38 | 39 | 40 |
| DBXT001-02 | 41 | 38 | 42 | 40 |
| DBXT001-03 | 43 | 38 | 44 | 40 |
| DBXT001-04 | 45 | 38 | 46 | 40 |
| DBXT001-05 | 47 | 48 | 49 | 50 |
| DBXT001-06 | 51 | 48 | 52 | 50 |
| DBXT001-07 | 53 | 48 | 54 | 50 |
| DBXT001-08 | 55 | 48 | 56 | 50 |
| DBXT002-01 | 37 | 38 | 57 | 58 |
| DBXT002-02 | 41 | 38 | 59 | 58 |
| DBXT002-03 | 43 | 38 | 60 | 58 |
| DBXT002-04 | 45 | 38 | 61 | 58 |
| DBXT002-05 | 47 | 48 | 62 | 63 |
| DBXT002-06 | 51 | 48 | 64 | 63 |
| DBXT002-07 | 53 | 48 | 65 | 63 |
| DBXT002-08 | 55 | 48 | 66 | 63 |
| DBXT003-01 | 37 | 38 | 57 | 40 |
| DBXT003-02 | 41 | 38 | 59 | 40 |
| DBXT003-03 | 43 | 38 | 60 | 40 |
| DBXT003-04 | 45 | 38 | 61 | 40 |
| DBXT003-05 | 47 | 48 | 62 | 50 |
| DBXT003-06 | 51 | 48 | 64 | 50 |
| DBXT003-07 | 53 | 48 | 65 | 50 |
| DBXT003-08 | 55 | 48 | 66 | 50 |
| DBXT004-01 | 37 | 38 | 67 | 68 |
| DBXT004-02 | 41 | 38 | 69 | 68 |
| DBXT004-03 | 43 | 38 | 70 | 68 |
| DBXT004-04 | 45 | 38 | 71 | 68 |
| DBXT004-05 | 47 | 48 | 72 | 73 |
| DBXT004-06 | 51 | 48 | 74 | 73 |
| DBXT004-07 | 53 | 48 | 75 | 73 |
| DBXT004-08 | 55 | 48 | 76 | 73 |
| DBXT005-01 | 37 | 38 | 90 | - |

### Expression and purification of antibodies

1. CHO-K1 cells from ECACC were thawed, inoculated into a culture medium (Shanghai OPM Biosciences Co., Ltd., Cat. No. C673017), and passaged at 37 °C with 8% CO₂. During transfection, the cell density was adjusted to 6.0 × 10⁶ cells/mL. 1.45 × 10⁸ cells were taken and centrifuged, and the supernatant was removed.
2. To the cell pellet was added 0.5 mL of electroporation buffer, and the mixture was mixed well.
3. Four plasmids in a ratio of H1:L1:H2:L2 = 2:3:2:3 were added, and the cell-plasmid suspension was mixed thoroughly and added into an electroporation cuvette, which was then placed in an electroporation apparatus for electroporation.
4. After the electroporation was completed, the cells in the electroporation cuvette were transferred into a shake flask containing 20 mL of culture medium and incubated at room temperature for 40 min without shaking.
5. After the incubation was completed, the shake flask was placed in a shaker for culture at 37 °C and 110 rpm with 8% CO₂. After 24 h, feed/sodium butyrate/antibiotics (penicillin + streptomycin) were added, and the culture was performed for another 4 days.
6. On day 4 after transfection, centrifugation was performed, and the supernatant was collected, purified by a Protein A affinity chromatography column (Bestchrom), eluted with a sodium acetate buffer (pH 3.4), collected in separate tubes, and sent for SEC analysis.
7. The fractionated eluate that met the requirements was further purified by gel filtration chromatography (Bestchrom 200, specification: 16 mm × 700 mm), eluted with 1× PBS, collected in separate tubes, and sent for SEC analysis. The eluates in the separate tubes that met the requirements were buffer-exchanged into a buffer (25 mM His-HCl, 6% Sucrose, pH 6.0) and then stored at -20°C.

### Example 2: Antibody Affinity Assay (SPR)

### 2.1 Objective: The affinity of the anti-EGFR/HER3 BsAb and parent mAbs was assayed using Biacore T200 (Cytiva).

### Experimental procedures:

1. An EGFR-HER3 BsAb (1 µg/mL) was captured by a ProA chip, and the analytes were as follows: human EGFR (His-Avi Tag, Kactus Biosystem, EGF-HM401) serially diluted into concentrations in the range of 3.125-400 nM for a total of 8 concentrations; human HER3 (His-Avi Tag, Kactus Biosystem, HER-HM403) serially diluted into concentrations in the range of 6.25-200 nM for a total of 8 concentrations; monkey EGFR (rhesus EGFR, C-His Tag, Kactus Biosystem, EGF-CM101) serially diluted into concentrations in the range of 6.25-400 nM for a total of 8 concentrations; and monkey HER3 (rhesus HER3, C-His Tag, Kactus Biosystem, HER-CM403) serially diluted into concentrations in the range of 3.125-200 nM for a total of 8 concentrations.
2. An EGFR parent mAb (1 µg/mL) was captured by a ProA chip, and the analyte was human EGFR (His-Avi Tag, Kactus Biosystem, EGF-HM401) serially diluted into concentrations in the range of 6.25-400 nM for a total of 8 concentrations. An HER3 parent mAb (3 µg/mL) was captured by a ProA chip, and the analyte was human HER3 (His-Avi Tag, Kactus Biosystem, HER-HM403) serially diluted into concentrations in the range of 3.125-100 nM for a total of 8 concentrations.
3. Affinity data were analyzed and fitted using a 1:1 Langmuir binding model. The affinity (KD) is the ratio of the dissociation constant (Kd) to the association constant (Ka). The affinity results are shown in Table 2.

**Table 2: Affinity of BsAb and mAbs for human and monkey antigens**

| | **hEGFR** | | | **hHER3** | | | **rhesusEGFR** | | | **rhesusHER3** | | |
|---|---|---|---|---|---|---|---|---|---|---|---|---|
| | KD (M) | kon (1/Ms) | koff (1/s) | KD (M) | kon (1/Ms) | koff (1/s) | KD (M) | kon (1/Ms) | koff (1/s) | KD (M) | kon (1/Ms) | koff (1/s) |
| **Anti-EGFR** | 3.36E-09 | 2.18E+05 | 7.33E-04 | - | - | - | | | | | | |
| **Anti-HER3** | - | - | - | 4.93E-10 | 7.68E+05 | 3.79E-04 | | | | | | |
| **DBXT001-01** | 9.29E-09 | 5.92E+04 | 5.50E-04 | 5.60E-10 | 3.22E+05 | 1.80E-04 | 1.13E-08 | 4.72E+04 | 5.34E-04 | 5.63E-10 | 2.78E+05 | 1.57E-04 |

Experimental conclusion: The affinity of the BsAb DBXT001-01 for human EGFR was about 16 times weaker than its affinity for HER3; the affinity of the BsAb DBXT001-01 for human EGFR was about 3 times weaker than that of the parent EGFR antibody, while the affinity of the BsAb DBXT001-01 for HER3 was comparable to that of the parent HER3 antibody. In addition, the affinity of the BsAb DBXT001-01 for human EGFR or HER3 was comparable to its affinity for monkey EGFR or HER3. Such a design for the BsAb affinity can reduce the on-target toxicity of the BsAb to normal tissues widely expressing EGFR.

### 2.2 Antibody affinity assay (SPR)

Objective: The affinity of the anti-EGFR/HER3 BsAb was assayed using Biacore T200 (Cytiva). Experimental procedures:
1. SI-1X6.4 (7.5 µg/mL) was captured by a ProA chip, and the analytes were as follows: human EGFR (His-Avi Tag, Kactus Biosystem, EGF-HM401) serially diluted into concentrations in the range of 1.17-18.75 nM for a total of 5 concentrations; and human HER3 (His-Avi Tag, Kactus Biosystem, HER-HM403) serially diluted into concentrations in the range of 4.69-75 nM for a total of 5 concentrations. Similarly, DBXT005-01 (6 µg/mL) was captured by a ProA chip, and the analyte were as follows: human EGFR serially diluted into concentrations in the range of 9.38-150 nM for a total of 5 concentrations; and human HER3 serially diluted into concentrations in the range o 4.69-75 nM for a total of 5 concentrations.
2. Affinity data were analyzed and fitted using a 1:1 Langmuir binding model. The affinity (KD) is the ratio of the dissociation constant (Kd) to the association constant (Ka). The affinity results are shown in Table 3.

**Table 3. Affinity of BsAbs for human antigens**

| | **hEGFR** | | | **hHER3** | | |
|---|---|---|---|---|---|---|
| | KD (M) | kon (1/Ms) | koff (1/s) | KD (M) | kon (1/Ms) | koff (1/s) |
| SI-1X6.4 | 1.41E-09 | 8.75E+05 | 1.24E-03 | 8.84E-08 | 2.44E+06 | 2.16E-01 |
| DBXT005-01 | 2.16E-08 | 6.13E+04 | 1.32E-03 | 3.54E-09 | 1.74E+05 | 6.16E-04 |

Experimental conclusion: The affinity of the BsAb DBXT005-01 of the present disclosure for human EGFR was about 15 times weaker than that of the control antibody SI-1X6.4, while its affinity for HER3 was 25 times stronger than that of the control antibody SI-1X6.4. Such a design for the BsAb affinity can reduce the on-target toxicity of the BsAb to normal tissues widely expressing EGFR and enhance the affinity for tumors with high EGFR expression and low HER3 expression.

### Example 3: Binding of BsAb and Its Corresponding Parent mAbs to Cell Lines with Different Levels of EGFR/HER3 Expression

### 3.1 Assay objective:

To compare the affinity of DBXT001-01 and its parent mAbs for cell lines with different levels of EGFR/HER3 expression by flow cytometry.

### Experimental procedures:

1. The mouse CT26 monoclonal cell line (Cobioer Biosciences) stably expressing human EGFR and HER3 was established; the tumor cell lines NCI-H441, NCI-H1975 and T47D were from ATCC.
2. All the cell lines were cultured in a complete medium at 37 °C with 5% CO₂.
3. Cells in the logarithmic growth phase were harvested, and the cell viability, determined by the trypan blue exclusion method, was kept at 90% or more. The cells were centrifuged at 1000 r/min for 5 min, and then the supernatant was discarded. The cells were washed once with PBS and resuspended in FACS Buffer to give a single-cell suspension. The cell density was adjusted to 5 × 10⁶ cells/mL.
4. To each well of a 96-well plate was added 50 µL of the cell suspension. The working solution of the test sample was 5-fold diluted with an initial concentration of 100 nM to give a total of 6 concentrations and then added to the plate. The mixture was mixed well, placed at 4 °C, and incubated for 40 min.
5. The cells were washed 3 times with FACS Buffer, using 400 µL each time, then centrifuged at 1000 r/min for 5 min, and finally resuspended in 100 µL of FACS Buffer.
6. 2 µL of PE-labeled secondary antibody (PE anti-human IgG Fc Antibody) was added, and the mixture was mixed well, placed at 4 °C, and incubated for 40 min in the dark.
7. The cells were washed 3 times with FACS Buffer, using 400 µL each time, then centrifuged at 1000 r/min for 5 min, and finally resuspended in 250 µL of FACS Buffer.
8. The fluorescence values were detected by a flow cytometer.

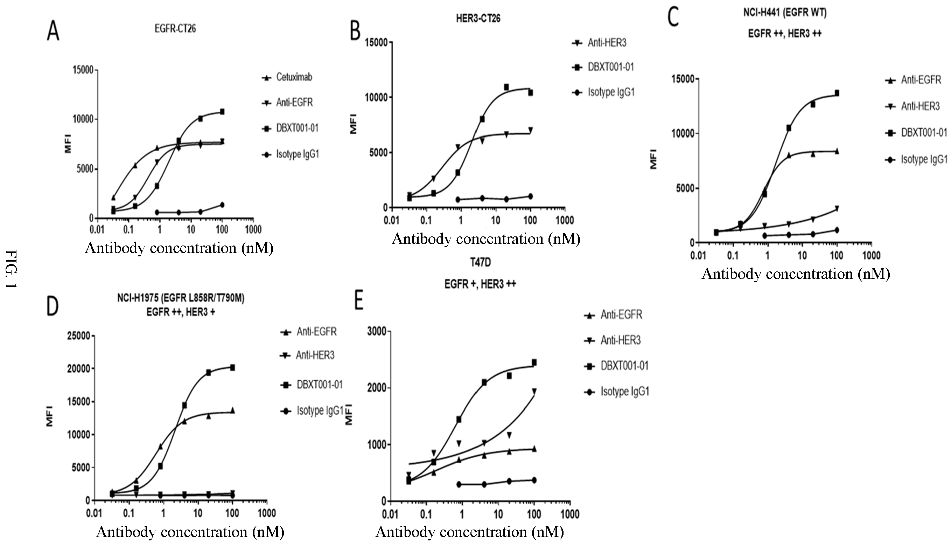

The experimental results are shown in FIG. 1 and Table 4.

**Table 4. Affinity of parent mAbs and BsAb for cell lines**

| | **EGFR-CT26** | | **HER3-CT26** | | **NCI-H441** | | **NCI-H1975** | | **T47D** | |
|---|---|---|---|---|---|---|---|---|---|---|
| | EC50 (nM) | Span | EC50 (nM) | Span | EC50 (nM) | Span | EC50 (nM) | Span | EC50 (nM) | Span |
| Cetuximab | 0.0336 | 10986 | | | | | | | | |
| Anti-EGFR | 0.4141 | 6787 | N/A | N/A | 0.7311 | 7394 | 0.5956 | 12644 | N/A | N/A |
| Anti-HER3 | N/A | N/A | 0.2802 | 6132 | N/A | N/A | N/A | N/A | N/A | N/A |
| DBXT001-01 | 1.904 | 10131 | 1.963 | 9912 | 1.663 | 12630 | 2.135 | 19298 | 0.6289 | 2155 |

### Experimental conclusion:

The affinity of DBXT001-01 for the cell line overexpressing EGFR or HER3 alone was lower than that of the bivalent parent mAbs, and its affinity for the EGFR-overexpressing cell line was also lower than that of cetuximab; however, the affinity of DBXT001-01 for the tumor cell lines expressing both EGFR and HER3 was not weaker than that of the bivalent parent mAbs. This may help the BsAb reduce binding to normal tissues while enhancing binding to tumor tissues expressing both EGFR and HER3.

### 3.2 Affinity of different BsAbs for cell lines with different levels of EGFR/HER3 expression

Experimental objective: The affinity of different BsAbs and parent mAbs for cell lines with different levels of EGFR/HER3 expression was compared by flow cytometry

### Experimental procedures:

1. The tumor cell lines BT-474 and MDA-MB-468 were from ATCC; the mouse CT26 monoclonal cell line stably expressing human EGFR or human HER3 was from Cobioer Biosciences.
2. All the cell lines were cultured in a complete medium at 37 °C with 5% CO₂.
3. Cells in the logarithmic growth phase were harvested, and the cell viability, determined by the trypan blue exclusion method, was kept at 90% or more. The cells were centrifuged at 1000 r/min for 5 min, and then the supernatant was discarded. The cells were washed once with PBS and resuspended in FACS Buffer to give a single-cell suspension. The cell density was adjusted to 5 × 10⁶ cells/mL.
4. To each well of a 96-well plate was added 50 µL of the cell suspension. The working solution of the test sample was 4-fold diluted with an initial concentration of 150 nM to give a total of 8 concentrations and then added to the plate. The mixture was mixed well, placed at 4 °C, and incubated for 40 min.
5. The cells were washed 3 times with FACS Buffer, using 400 µL each time, then centrifuged at 1000 r/min for 5 min, and finally resuspended in 100 µL of FACS Buffer.
6. 2 µL of PE-labeled secondary antibody (PE anti-human IgG Fc Antibody) was added, and the mixture was mixed well, placed at 4 °C, and incubated for 40 min in the dark.
7. The cells were washed 3 times with FACS Buffer, using 400 µL each time, then centrifuged at 1000 r/min for 5 min, and finally resuspended in 250 µL of FACS Buffer.
8. The fluorescence values were detected by a flow cytometer.

The codes of the test samples are shown in Table 5

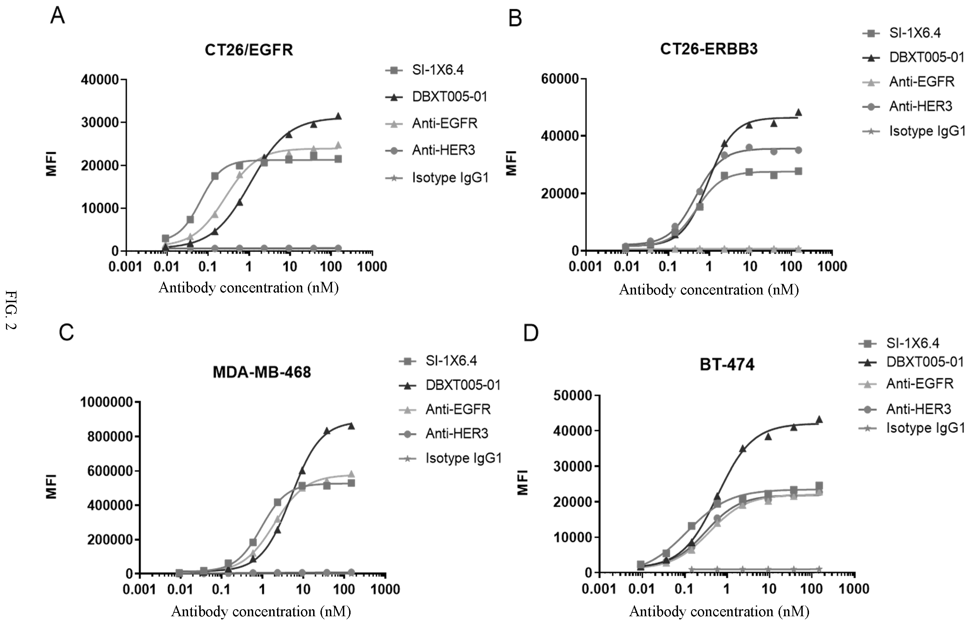

The experimental results are shown in FIG. 2 and Table 6.

**Table 5. Different compositions of BsAbs or mAbs**

| Name | EGFR arm | HER3 arm |
|---|---|---|
| SI-1X6.4 | SI-1X6.4 anti-EGFR (bivalent) | SI-1X6.4 anti-HER3 (bivalent) |
| DBXT005-01 | anti-EGFR Fab (monovalent) | anti-HER3 scFv (monovalent) |
| Anti-EGFR | anti-EGFR mAb (bivalent) | N/A |
| Anti-HER3 | N/A | anti-HER3 mAb (bivalent) |

**Table 6. Affinity of different BsAbs for cell lines with different levels of EGFR/HER3 expression**

| | **EGFR/CT26 (EGFR+++, HER3-)** | **HER3/CT26 (EGFR-, HER3+++)** | **MDA-MB-468 (EGFR+++, HER3+)** | **BT-474 (EGFR++, HER3+++)** |
|---|---|---|---|---|
| Name | SPAN (MFI) | SPAN (MFI) | SPAN (MFI) | SPAN (MFI) |
| S1-1X6.4 | 19146 | 26128 | 514963 | 23955 |
| DBXT005-01 | 30622 | 44784 | 876058 | 40801 |
| Anti-EGFR | 22885 | N/A | 571785 | 21095 |
| Anti-HER3 | N/A | 33562 | 11078 | 20649 |

Experimental conclusion: DBXT005-01 exhibited a stronger ability to saturate target antigens in cell lines with different levels of EGFR and HER3 expression as compared to the control antibody SI-1X6.4; it also demonstrated a stronger ability to saturate antigens compared to the corresponding parent mAbs.

### Example 4: Endocytosis Activity of Antibodies (pHrodo Method)

### Assay objective

The antibody drug targeting EGFR and HER3 of the present disclosure on T47D cells expressing both EGFR and HER3 was assayed for endocytosis effect, and compared with the parent mAbs for endocytosis activity. Cells were co-incubated with the antibody drug at a fixed concentration and an endocytosis indicating reagent pHrodo, and the endocytosis capability of the antibody drug was evaluated by observing the intracellular fluorescence signals produced by pHrodo entering the cells along with the antibody drug at different time points.

### Experimental procedures

1. Cell culture: T47D cells were cultured in an RPMI-1640 medium containing 0.2 Units/mL bovine insulin and 10% FBS.
2. Cell preparation: T47D cells in the logarithmic growth phase were taken, washed once with PBS, and then digested for 2-3 min. After the cells were completely digested, 10-15 mL of cell culture medium was added to elute the digested cells. The eluate was centrifuged at 1000 rpm for 5 min, and the supernatant was discarded. The resulting cells were resuspended in cell culture medium to give a single-cell suspension, with the viable cell density adjusted to 3 × 10⁵ cells/mL.
3. Cell plating: The cell suspension was added to a 96-well cell culture plate at 50 µL/well. The culture plate was incubated in an incubator for 48 h (37 °C, 5% CO₂).
4. Sample addition: The test antibody drug was co-incubated with Fab-pHrodo to form a complex, which was then adjusted to a concentration of 6 nM or 60 nM and added to the cells at 50 µL/well.
5. Cell incubation: The culture plate was incubated in an incubator for 48 h (37 °C, 5% CO₂).
6. Plate reading: At the corresponding time point, the 96-well cell culture plate was taken out, the cells were digested, and then the cell count and fluorescence values were read by FACS.

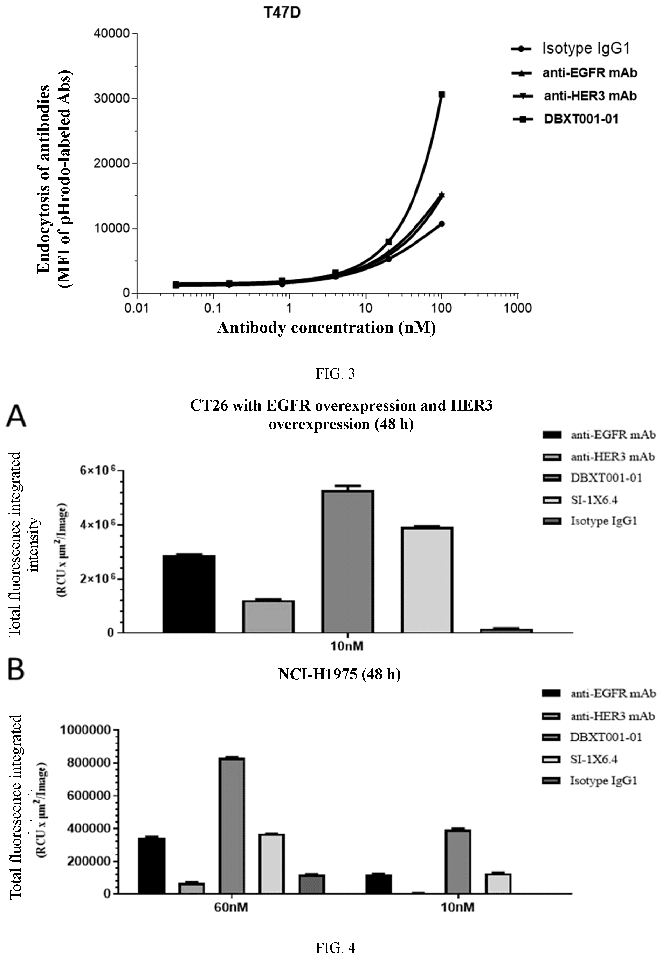

### The experimental results are shown in FIG. 3.

### Experimental conclusion:

The antibody drug targeting EGFR and HER3 of the present disclosure had a better endocytosis effect on T47D cells expressing both EGFR and HER3 as compared to the parent mAbs.

### Example 5: Endocytosis Activity of Antibodies (Incucyte Method)

### 5.1 Assay objective:

The endocytosis efficiency of the antibody drug targeting EGFR and HER3 of the present disclosure was assayed in the NCI-H1975 cell line expressing both EGFR and HER3, and in the CT26 cell line overexpressing both EGFR and HER3, as compared to another antibody drug targeting EGFR and HER3. Cells were co-incubated with the antibody-drug conjugate at a fixed concentration and an endocytosis indicating reagent Incucyte^{®} Fabfluor-pH, and the endocytosis capability of the antibody-drug conjugate was evaluated by continuously observing the changes in fluorescence signals of live cells for 48 h.

### Experimental procedures:

1. Cell culture: NCI-H1975 cells were cultured in an RPMI1640 medium containing 10% FBS; the culture medium for the CT26 cell line stably transfected with both EGFR and HER3 was 1640 + 10% FBS + 10 µg/mL puromycin + 20 µg/mL blasticidin.
2. Cell preparation: Cells in the logarithmic growth phase were taken, washed once with PBS, and then digested for 2-3 min. After the cells were completely digested, 10-15 mL of cell culture medium was added to elute the digested cells. The eluate was centrifuged at 1000 rpm for 5 min, and the supernatant was discarded. The resulting cells were resuspended in cell culture medium to give a single-cell suspension, with the viable cell density adjusted to 1 × 10 ⁵ cells/mL.
3. Cell plating: The cell suspension was added to a 96-well cell culture plate at 50 µL/well. The plate was incubated in an incubator overnight (37 °C, 5% CO₂).
4. Labeling of test antibody-drug conjugates: For high-concentration antibody labeling, a stock solution of the test sample was diluted to a 4× working solution at 240 nM (with a final concentration of 60 nM), a stock solution of Incucyte^{®} Fabfluor-pH was diluted to a 4× working solution at 720 nM (with a final concentration of 180 nM), and then the two working solutions were mixed thoroughly and incubated at 37 °C in the dark for 15 min. For low-concentration antibody labeling, a stock solution of the test sample was diluted to a 4× working solution at 40 nM (with a final concentration of 10 nM), a stock solution of Incucyte^{®} Fabfluor-pH was diluted to a 4× working solution at 120 nM (with a final concentration of 30 nM), and then the two working solutions were mixed thoroughly and incubated at 37 °C in the dark for 15 min.
5. Capture of analysis images: The labeled working solution of the test sample was transferred to corresponding wells of the experiment plate. The experiment plate was then transferred to the Incucyte live-cell analysis device. A scanning and photographing program was set up, and images were captured using the Incucyte live-cell analysis system. Quantification was performed at 2-hour intervals over a period of 48 h. The analysis results are expressed as: total fluorescence integrated intensity (RCU X µm²/image).

The experimental results are shown in FIGs. 4A and 4B.

### Experimental conclusion:

The antibody drug targeting EGFR and HER3 of the present disclosure had a better endocytosis effect on cells expressing both EGFR and HER3 at both high and low concentrations as compared to the parent mAbs. Meanwhile, the antibody drug targeting EGFR and HER3 of the present disclosure had a better endocytosis effect at both high and low concentrations as compared to the control antibody SI-1X6.4.

### 5.2 Endocytosis of different BsAbs on cell lines with different levels of EGFR/HER3 expression (incucyte method)

### Assay objective:

The endocytosis efficiency of the antibody drugs targeting EGFR and HER3 of the present disclosure was assayed in the A431 and NCI-H1975 cell lines with different levels of EGFR and HER3 expression, and in the CT26 cell line overexpressing both EGFR and HER3, as compared to another antibody drug targeting EGFR and HER3. Cells were co-incubated with the antibody-drug conjugate at a fixed concentration and an endocytosis indicating reagent Incucyte^{®} Fabfluor-pH, and the endocytosis capability of the antibody-drug conjugate was evaluated by continuously observing the changes in fluorescence signals of live cells for 24 h.

### Experimental procedures:

1. Cell culture: A431 cells were cultured in a DMEM medium containing 10% FBS; NCI-H1975 cells were cultured in an RPMI1640 medium containing 10% FBS; the culture medium for the CT26 cell line stably transfected with both EGFR and HER3 was 1640 + 10% FBS + 10 µg/mL puromycin + 20 µg/mL blasticidin.
2. Cell preparation: Cells in the logarithmic growth phase were taken, washed once with PBS, and then digested for 2-3 min. After the cells were completely digested, 10-15 mL of cell culture medium was added to elute the digested cells. The eluate was centrifuged at 1000 rpm for 5 min, and the supernatant was discarded. The resulting cells were resuspended in cell culture medium to give a single-cell suspension, with the viable cell density adjusted to 1 × 10 ⁵ cells/mL.
3. Cell plating: The cell suspension was added to a 96-well cell culture plate at 50 µL/well. The plate was incubated in an incubator overnight (37 °C, 5% CO₂).
4. Labeling of test antibody-drug conjugate: A working solution of the test sample/control sample and a working solution of the labeling reagent were mixed at a molar ratio of 1:3 and a volume ratio of 1:1, and then incubated in a cell incubator (37 °C., 5% CO₂) for 15 min in the dark to allow them to be conjugated sufficiently.
5. Capture of analysis images: The labeled working solution of the test sample was transferred to corresponding wells of the experiment plate. The experiment plate was then transferred to the Incucyte live-cell analysis device. A scanning and photographing program was set up, and images were captured using the Incucyte live-cell analysis system. Quantification was performed at 2-hour intervals over a period of 24 h. The analysis results are expressed as: total fluorescence integrated intensity (RCU X µm²/image).

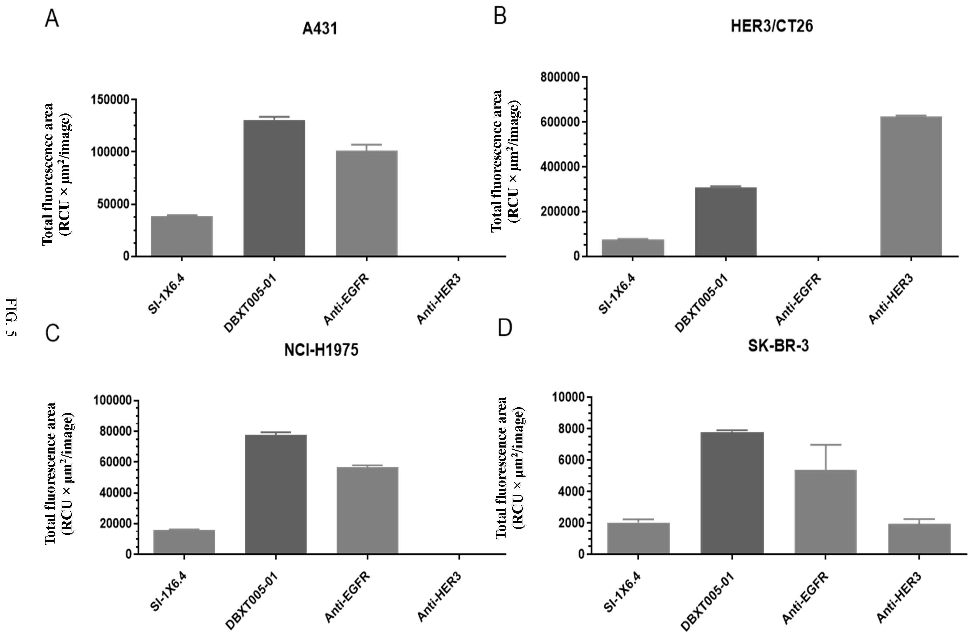

The experimental results are shown in FIGs. 5A, 5B, 5C and 5D and Table 7.

**Table 7. Endocytosis of BsAbs at 1 nM on cell lines with different levels of EGFR/HER3 expression over 24 h**

| Name | A431 (RCU X µm²/image) | HER3/CT26 (RCU X µm²/image) | NCI-H1975 (RCU X µm²/image) | SK-BR-3 (RCU X µm²/image) |
|---|---|---|---|---|
| SI-1X6.4 | 38,045 | 76,766 | 15,828 | 2,041 |
| DBXT005-01 | 130,882 | 309,260 | 77,406 | 7,805 |
| Anti-EGFR | 101,106 | 603 | 56,406 | 5,394 |
| Anti-HER3 | 186 | 627,334 | 43 | 1,957 |

### Experimental conclusion:

The antibody DBXT005-01 targeting EGFR and HER3 of the present disclosure had a better endocytosis effect on tumor cells with different levels of EGFR or HER3 expression as compared to the control antibody.

### Example 6: Preparation of Bispecific Antibody-Drug Conjugates (ADCs)

### 6.1 Preparation of linker-cytotoxin (linker-payload)

### Preparation of linker-payload X1

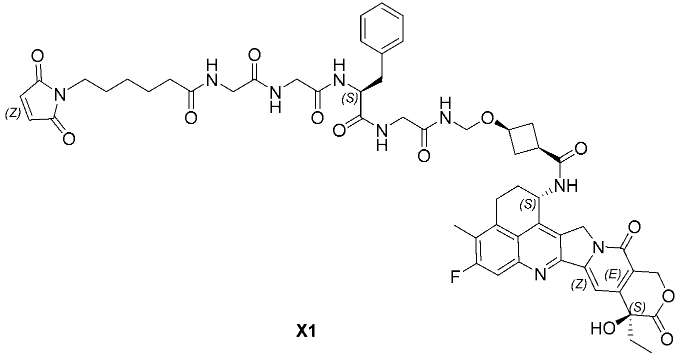

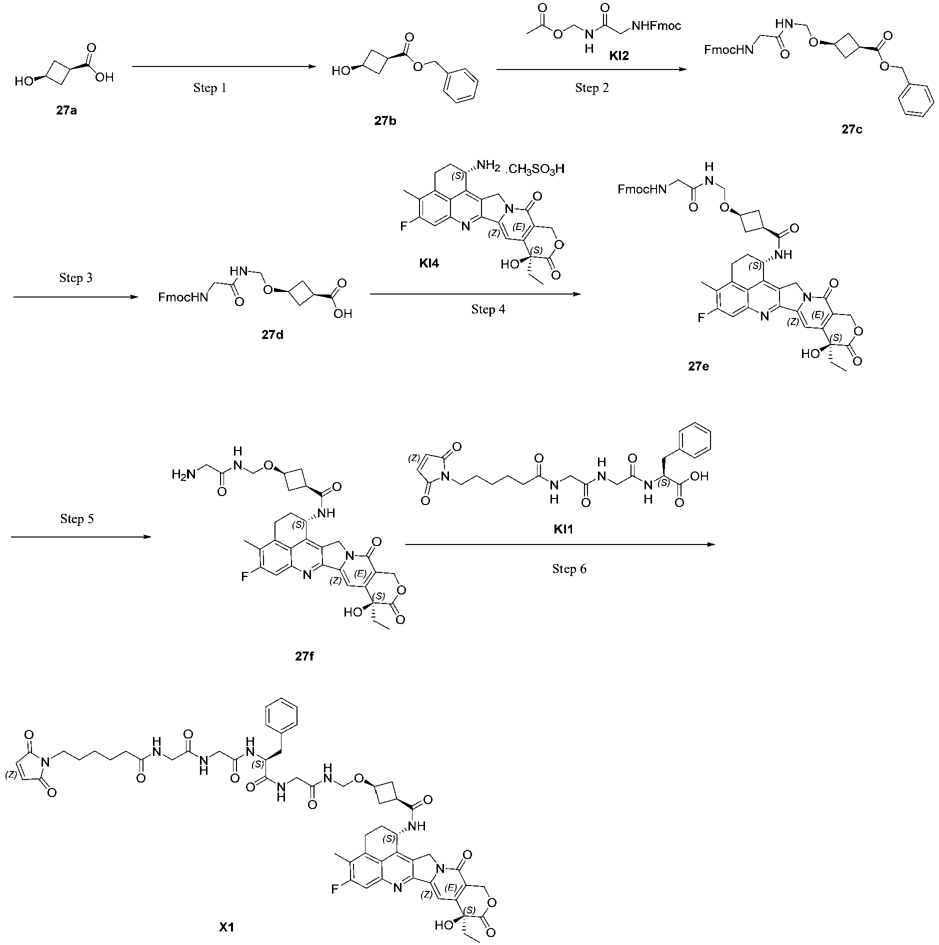

### Step 1

Benzyl bromide (11.0 g, 64.6 mmol) was added dropwise to a solution of 27a (5.00 g, 43.0 mmol) and NaHCO₃ (10.9 g, 129 mmol) in DMF (50 mL) under a nitrogen atmosphere, and the mixture was allowed to react at 25 °C for 17 h. After the reaction was completed as detected by TLC (PE/EA = 2/1), the reaction solution was added to water (500 mL) and extracted twice with EA (250 mL). The organic phase was separated, washed with a saturated aqueous sodium chloride solution (500 mL), dried over anhydrous Na₂SO₄, and concentrated, and the residue was subjected to column chromatography (PE:EA = 3:2) to give a colorless liquid (5.1 g, yield: 57.1%).

### Step 2

A solution of 27b (4.50 g, 21.8 mmol) in THF (10 mL) was added dropwise to a solution of KI2 (4.00 g, 10.9 mmol) and TsOH (800 mg, 4.65 mmol) in THF (30 mL) at 0 °C under a nitrogen atmosphere, and the mixture was allowed to react at 25 °C for 2 h. After the reaction was completed as detected by TLC (PE/EA = 1/2), the reaction solution was added to water (200 mL) and extracted twice with EA (200 mL). The organic phase was separated, dried over anhydrous Na₂SO₄, and concentrated, and the residue was subjected to column chromatography (PE/EA = 3/2) to give a white solid (1.56 g, yield: 26%).

### Step 3

Pd/C (80 mg) was added to a mixed solution of 27c (800 mg, 1.55 mmol) in EtOH (8 mL) and EA (8 mL) at 0 °C under a hydrogen atmosphere, and the mixture was stirred at 0 °C for 2.5 h. After the reaction was completed as detected by LCMS, the reaction solution was filtered through celite, and the filter cake was washed with EA (200 mL). The filtrate was concentrated, and then the residue was dissolved in THF (20 mL). The resulting solution was concentrated to dryness by rotary evaporation to give a white solid (600 mg, yield: 91%).

### Step 4

DIEA (152 mg, 1.18 mmol) was added to a solution of 27d (220 mg, 0.515 mmol), HY-13631A (250 mg, 0.47 mmol) and HATU (214 mg, 0.56 mmol) in DMF (6 mL) at 0 °C under a nitrogen atmosphere, and the mixture was allowed to react at 0 °C for 2 h. After the reaction was completed as detected by LCMS, the reaction solution was added to an aqueous citric acid solution (pH = 4) (150 mL), and the mixture was filtered. The filter cake was washed with water (175 mL), dried by filtration, and dried with an oil pump to give a brown solid (260 mg, yield: 66%).

### Step 5

Diethylamine (8 mL) was added dropwise to a solution of 27e (260 mg, 0.309 mmol) in DCM (30 mL) at 0 °C under a nitrogen atmosphere, and the mixture was allowed to react at 0 °C for 3 h. After the reaction was completed as detected by LCMS, the reaction solution was added to a petroleum ether solution (600 mL) at 0 °C, and a solid was precipitated. The resulting mixture was left to stand until the solid was adsorbed on the bottom of the flask, and then the solution was poured out. The residue was dried with an oil pump to give a brown solid (90 mg, yield: 47.1%).

### Step 6

HATU (74 mg, 0.19 mmol) was added to a solution of 27f (90 mg, 0.13 mmol), KI1 (92 mg, 0.19 mmol) and DIEA (50 mg, 0.39 mmol) in DMF (2.5 mL) at 0 °C under a nitrogen atmosphere, and the mixture was allowed to react at 0 °C for 2 h. After the reaction was substantially completed as detected by LCMS, the reaction solution was added to an aqueous citric acid solution (30 mL) at pH 4 at 0 °C, and a flocculent solid was precipitated. The resulting mixture was filtered, and subjected to preparative chromatography (DCM/MecOH = 10/1) to give X1 as a pale yellow solid (9.2 mg, yield: 6%).
MS m/z (ESI): 1074 [M+1]
H-NMR (400 MHz, MeOD): 7.65 (d, 1H), 7.62 (s, 1H), 7.30-7.21(m, 5H), 6.79 (s, 2H), 5.69-5.65 (m, 1 H), 5.57 (d, 1H), 5.43-5.10 (m, 3H), 4.70 (d, 2H), 4.48-4.39 (m, 2H), 4.10-4.05 (m, 1H), 4.01-3.75 (m, 5H),3.46 (t, 2H), 3.22-3.15 (m, 2H), 3.07-3.00 (m, 1H), 2.75 (m, 1H), 2.62 (m, 1H), 2.45 (s, 3H), 2.37-2.20 (m, 6H), 2.10-2.02 (m, 2H), 2.00-1.92 (m, 2H) 1.68-1.57 (m, 6H), 1.01 (t, 3H)

### Preparation of linker-payload X2

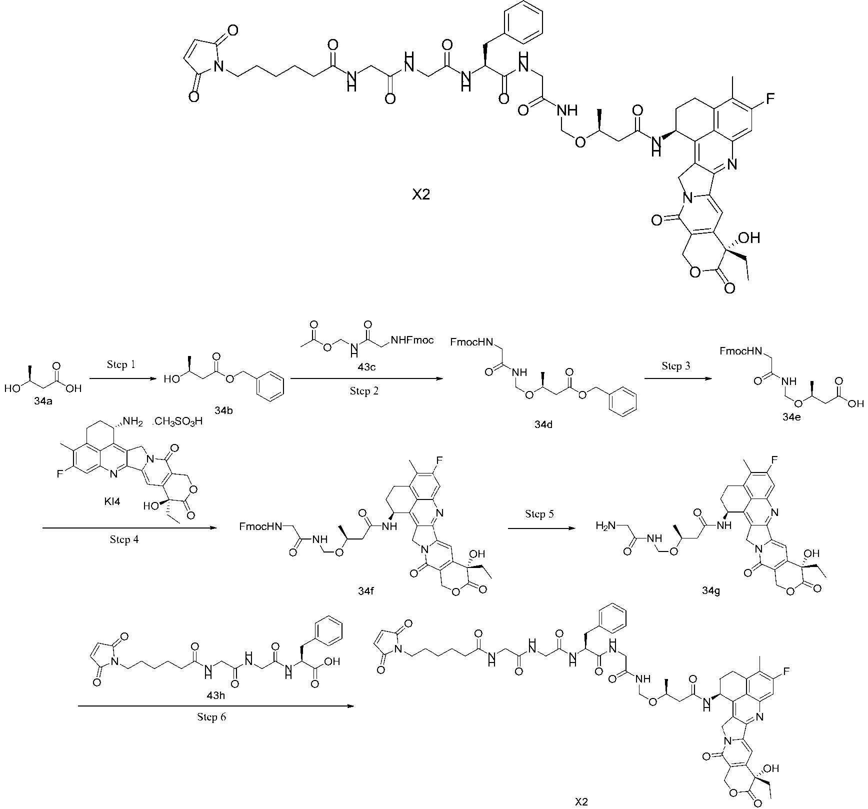

### Step 1

34a (5 g, 48.0 mmol) and K₂CO₃ (19.9 g, 144.0 mmol) were dissolved in DMF (20 mL), and benzyl bromide (12.3 g, 72.0 mmol) was added dropwise to the resulting solution. The mixture was allowed to react at 25 °C for 17 h. After the starting materials were completely consumed as detected by TLC (PE/EA = 3/1), the reaction solution was added to water (200 mL) and extracted with EA (250 mL). The organic phase was separated, washed with saturated NaCl, dried over anhydrous Na₂SO₄, and then concentrated, and the residue was subjected to column chromatography (PE:EA = 2:1) to give 34b as a colorless liquid (8.7 g, yield 93%). MS-ESI: m/z 195.1 [M+H]+.

### Step 2

34c (7.3 g, 19.8 mmol) and TsOH (1.46 g, 8.5 mmol) were dissolved in THF (20 mL), and the mixture was cooled to 0 °C under a nitrogen atmosphere. A solution of 43b (7.7 g, 39.6 mmol) in THF (10 mL) was added dropwise, and after the addition, the mixture was allowed to react at 0 °C for 2 h. After most of the starting materials were consumed as detected by TLC (PE/EA = 2/1), the reaction solution was poured into water (100 mL) and extracted with DCM (100 mL). The organic phase was separated, washed with saturated NaCl, dried over anhydrous Na₂SO₄, and then subjected to column chromatography (PE/EA = 1/1) to give 34d as a colorless sticky substance (3.9 g, yield: 39%). MS-ESI: m/z 503.3 [M+H]+.

### Step 3

Pd/C (1 g, 10 wt.%) was added to a mixed solution of 34d (1.9 g, 3.78 mmol) in EtOH (100 mL) and EA (100 mL) at 0 °C under a hydrogen atmosphere, and the mixture was allowed to react at 0 °C for 3 h. After the reaction was completed as detected by TLC (PE/EA = 2/1), the reaction solution was filtered through celite, and the filter cake was washed with EA/EtOH (1:1, 100 mL × 3). The filtrate was concentrated. The residue was dissolved in THF (50 mL × 3), and the resulting solution was concentrated to dryness by rotary evaporation; the procedure was repeated three times to give 34e as a gray solid (1 g, yield: 64%). MS-ESI: m/z 435.2 [M+Na]+.

### Step 4

DIEA (303 mg, 2.35 mmol) was added dropwise to a solution of 34e (426 mg, 1.03 mmol), KI4 (500 mg, 0.94 mmol) and HATU (429 mg, 1.13 mmol) in DMF (20 mL) at 0 °C under a nitrogen atmosphere, and after the addition, the mixture was allowed to react at 0 °C for 2 h. After the reaction was completed as detected by LCMS, the reaction solution was added dropwise to water (300 mL). The mixture was stirred, then left to stand for 5 min, and filtered, and the filter cake was dissolved in DCM/MeOH (10:1, 100 mL) solution. The resulting solution was dried and concentrated to dryness by rotary evaporation, and the residue was mixed with silica gel and subjected to column chromatography (EA:MeOH = 30:1) to give 34f as a yellow solid (600 mg, yield: 77%). MS-ESI: m/z 830.3 [M+H]+.

### Step 5

Diethylamine (5 mL) was added dropwise to a solution of 34f (150 mg, 0.18 mmol) in DCM (5 mL) at 0 °C under a nitrogen atmosphere, and the mixture was allowed to react at 0 °C for 2 h. After the reaction was completed as detected by LCMS, a petroleum ether solution (100 mL × 6) was added to the reaction solution, and a solid precipitated. The resulting mixture was left to stand until the solid settled, and then the solution was poured out. The residue was dried with an oil pump to give 34g as a white powder (120 mg, yield: 76%), with a product content of 70% as determined by LCMS. MS-ESI: m/z 608.3 [M+H]+.

### Step 6

A solution of HATU (45 mg, 0.118 mmol) in DMF (1 mL) was added to a solution of 34g (60 mg, 0.099 mmol), 43h (51 mg, 0.108 mmol) and DIEA (32 mg, 0.25 mmol) in DMF (1 mL) at 0 °C under a nitrogen atmosphere, and the mixture was allowed to react at 0 °C for 2 h. After the starting materials were completely consumed as detected by LCMS, the reaction solution was directly subjected to reversed-phase column chromatography (eluent: (MeCN/MeOH = 1/1):H₂O = 60%:40%) to give X2 as a yellow solid (14.8 mg, 14% yield).

MS-ESI: m/z 1062.4 [M+H]+.

1H NMR (400 MHz, Methanol-d4) δ 7.69 - 7.61 (m, 2H), 7.22 - 7.16 (m, 2H), 7.16 - 7.09 (m, 3H), 6.76 (s, 2H), 5.70 - 5.64 (m, 1H), 5.60 (d, J = 16.4 Hz, 1H), 5.40 - 5.31 (m, 2H), 5.26 (d, J = 19.0 Hz, 1H), 4.65 - 4.50 (m, 7H), 4.25 - 4.16 (m, 1H), 3.87 (d, J = 16.7 Hz, 1H), 3.83 - 3.76 (m, 3H), 3.72 (d, J = 17.0 Hz, 2H), 3.44 (t, J = 7.1 Hz, 2H), 3.25 - 3.17 (m, 2H), 3.10 - 3.02 (m, 1H), 2.92 - 2.83 (m, 1H), 2.45 - 2.39 (m, 5H), 2.32 - 2.20 (m, 5H), 1.97 - 1.89 (m, 2H), 1.63 - 1.50 (m, 4H), 1.34 - 1.20 (m, 6H), 0.99 (t, J = 7.3 Hz, 3H).

### Preparation of linker-payload X3

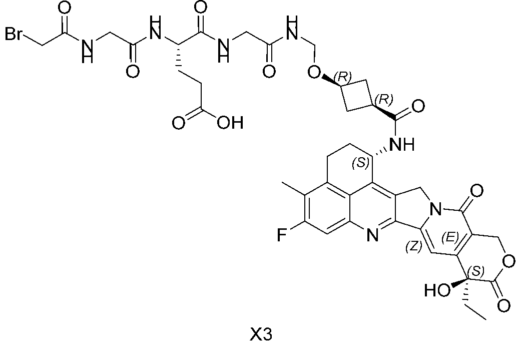

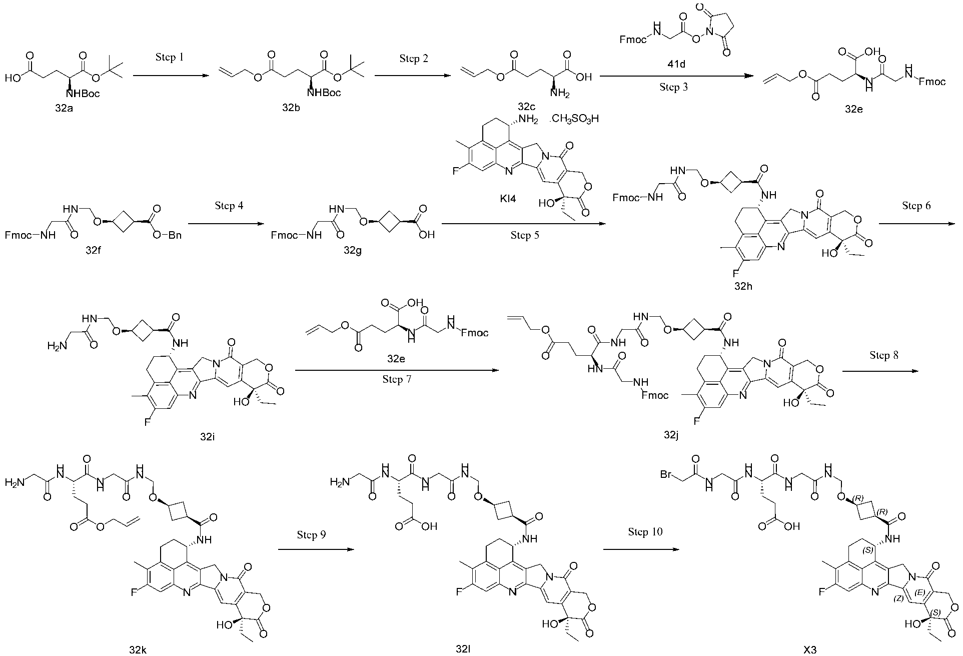

### Step 1

Bromopropene (960 mg, 7.92 mmol) was added to 32a (2.00 g, 6.6 mmol) and K₂CO₃ (1.82 g, 13.2 mmol) in MeCN (20 mL), and the mixture was stirred at 20 °C for 5 h. After the reaction was completed as detected by TLC (PE/EA = 1/2), the reaction solution was poured into water (100 mL), adjusted to pH 5, and extracted three times with EA (100 mL). The organic phase was dried over anhydrous sodium sulfate and concentrated to dryness by rotary evaporation, and the residue was purified by column chromatography (PE/EA = 2/1) to give 32b as a white solid (1.83 g, yield: 81%).

### Step 2

TFA (10 mL) was added to 32b (1.38 g, 4.02 mmol) in DCM (10 mL), and the mixture was stirred at 25 °C for 17 h. After the reaction was completed as detected by TLC (PE/EA = 1/3), the reaction solution was concentrated to dryness by rotary evaporation to give 32c as a yellow sticky substance (0.91 g, yield not calculated).

### Step 3

41d (1.92 g, 4.87 mmol) was added to 32c (910 mg, 4.87 mmol) and NaHCO₃ (613 mg, 7.3 mmol) in DME/H₂O (20 mL/10 mL), and the mixture was stirred at 25 °C for 3 h. After the reaction was completed as detected by TLC (DCM/MeOH = 1/1), the reaction solution was poured into water (100 mL), adjusted to pH 5 with aq. HCl (1 N), and extracted twice with EA (150 mL). The organic phase was dried over anhydrous sodium sulfate and concentrated to dryness by rotary evaporation, and the residue was purified by column chromatography (DCM/MeOH = 20/1) to give 32e as a white solid (1.53 g, yield: 67%). MS-ESI: m/z 467.4 [M+H]+.

### Step 4

Pd/C (600 mg) was added to 32f (3 g, 5.83 mmol) in MeOH (50 mL), and the mixture was stirred under a hydrogen atmosphere at 25 °C for 5 h. After the reaction was completed as detected by TLC (EA), the reaction solution was filtered, and the filtrate was concentrated to dryness by rotary evaporation to give 32g as a white solid (1.9 g, yield: 77%).

### Step 5

HATU (707 mg, 1.86 mmol) was added to 32g (789 mg, 1.86 mmol), KI4 (900 mg, 1.69 mmol) and triethylamine (342 mg, 3.38 mmol) in DMF (10 mL), and the mixture was stirred at 0 °C for 3.5 h. After the reaction was completed as detected by TLC (EA), the reaction solution was poured into H₂O (80 mL) and extracted twice with EA (100 mL). The organic phase was dried over anhydrous sodium sulfate and concentrated to dryness by rotary evaporation, and the residue was purified by column chromatography (EA) to give 32h as a white solid (1.186 g, yield: 83%). MS-ESI: m/z 842.3 [M+H]+.

### Step 6

32h (1.186 g, 1.41 mmol) in DCM/diethylamine (20 mL, 20/1) was stirred at 25 °C for 17 h. After the reaction was completed as detected by TLC (DCM/MeOH = 10/1), the reaction solution was poured into petroleum ether (200 mL). The resulting mixture was filtered to give 32i as a white solid (768 mg, yield: 88%). MS-ESI: m/z 620.3 [M+H]+.

### Step 7

HATU (414 mg, 1.09 mmol) was added to 32i (676 mg, 1.09 mmol), 32e (508 mg, 1.09 mmol) and DIEA (423 mg, 3.27 mmol) in DMF (10 mL), and the mixture was stirred at 20 °C for 17 h. After the reaction was completed as detected by TLC (PE/EA = 1/5), the reaction solution was poured into water (30 mL). The resulting mixture was filtered, and the filter cake was purified by column chromatography (DCM/MeOH = 50/1) to give 32j as a white solid (511 mg, yield: 44%). MS-ESI: m/z 1068.3 [M+H]+.

### Step 8

A solution of 32j (482 mg, 0.451 mmol) in diethylamine/DCM (10 mL, 1/5) was stirred at 10 °C for 17 h. After the reaction was completed as detected by TLC (EA), the reaction solution was poured into PE (300 mL). The resulting mixture was filtered to give 32k as a white solid (301 mg, yield not calculated).

### Step 9

Morpholine (93 mg, 1.07 mmol) was added to 32k (301 mg, 0.356 mmol) and Pd(PPh3)4 (82 mg, 0.071 mmol) in THF (5 mL), and the mixture was stirred at 25 °C for 5 h. After the reaction was completed as detected by LCMS, the reaction solution was subjected to preparative chromatography to give 321 as a white solid (108 mg, yield: 38%). MS-ESI: m/z 806.3 [M+H]+.

### Step 10

Bromoacetyl bromide (27 mg, 0.134 mmol) was added to 321 (108 mg, 0.134 mmol) and triethylamine (41 mg, 0.402 mmol) in THF (2 mL) and DMF (2 mL), and the mixture was stirred at 0 °C for 1 h. After the reaction was completed as detected by TLC (DCM/MeOH = 10/1), the reaction solution was directly subjected to preparative chromatography to give X3 as a white solid (15 mg, yield: 12%).

MS-ESI: m/z 926.3 [M+H]+.

1H NMR (400 MHz, DMSO-d6) δ 12.11 (s, 1H), 8.54 - 8.42 (m, 3H), 8.27 - 8.16 (m, 2H), 7.78 (d, J = 11.0 Hz, 1H), 7.30 (s, 1H), 6.53 (s, 1H), 5.61 - 5.51 (m, 1H), 5.42 (s, 2H), 5.20 - 5.05 (m, 2H), 4.56 - 4.42 (m, 2H), 4.32 - 4.22 (m, 1H), 3.96 - 3.87 (m, 3H), 3.79 (d, J = 5.6 Hz, 2H), 3.70 (d, J = 5.9 Hz, 2H), 3.25 - 3.08 (m, 2H), 2.61 - 2.53 (m, 2H), 2.45 - 2.36 (m, 4H), 2.36 - 2.22 (m, 3H), 2.20 - 2.03 (m, 4H), 1.99 - 1.68 (m, 4H), 0.87 (t, J = 7.3 Hz, 3H).

### Preparation of linker-payload X4

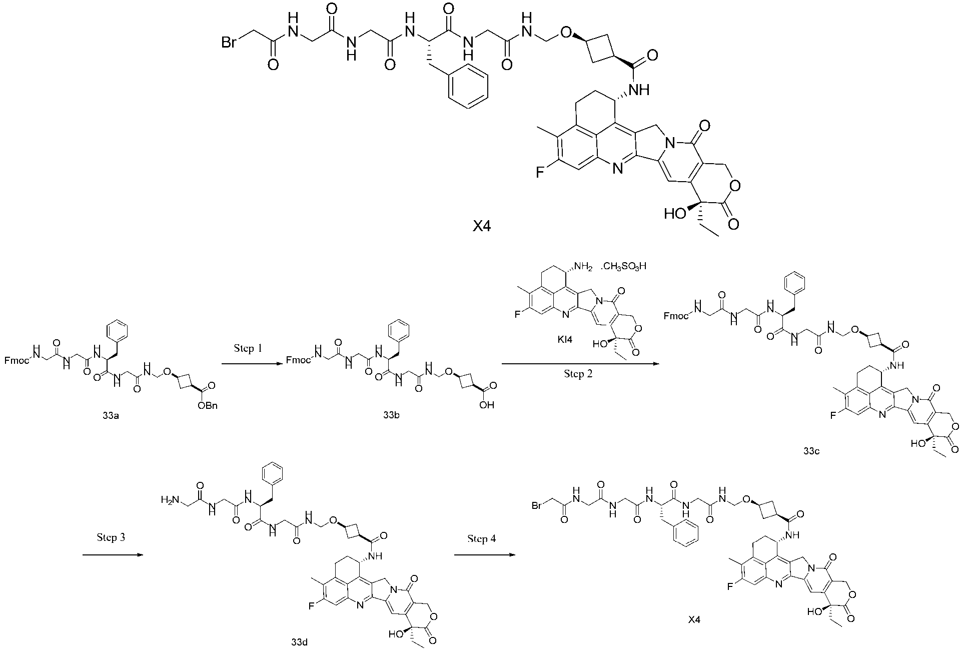

### Step 1

Pd/C (400 mg, 10 wt.%) was added to 33a (2.00 g, 2.58 mmol) in MeOH (20 mL), and the mixture was stirred at 20 °C for 5 h. After the reaction was completed as detected by TLC (EA), the reaction solution was filtered, and the filtrate was concentrated to dryness by rotary evaporation to give 33b as a white solid (1.3 g, yield: 74%).

### Step 2

HATU (305 mg, 0.802 mmol) was added to 33b (0.55 g, 0.802 mmol), KI4 (427 mg, 0.802 mmol) and DIPEA (310 mg, 2.40 mmol) in DMF (5 mL), and the mixture was stirred at 0 °C for 2 h. After the reaction was completed as detected by TLC (DCM/MeOH = 1/10), the reaction solution was poured into water (40 mL). The resulting mixture was filtered to give a crude product, which was then purified by column chromatography (DCM/MeOH = 20/1) to give 33c as a yellow solid (360 mg, yield: 41%).

### Step 3

Diethylamine (2 mL) was added to 33c (360 mg, 0.326 mmol) in DCM (10 mL), and the mixture was stirred at 25 °C for 17 h. After the reaction was completed as detected by TLC (DCM/MeOH = 5/1), the reaction solution was poured into PE (100 mL). The resulting mixture was filtered to give 33d as a white solid (205 mg, yield: 71%). MS-ESI: m/z 881.3 [M+H]+.

### Step 4

A solution of bromoacetyl bromide (94 mg, 0.446 mmol) in THF (2 mL) was added to 33d (205 mg, 0.233 mmol) and triethylamine (118 mg, 1.17 mmol) in DMF (1 mL) and water (1 mL), and the mixture was stirred at 0 °C for 1 h. The reaction solution was directly subjected to preparative chromatography to give X4 as a white solid (15 mg, yield: 6%).

MS-ESI: m/z 1001.2 [M+H]+.

1H NMR (400 MHz, DMSO-d6) δ 8.57 - 8.50 (m, 1H), 8.50 - 8.43 (m, 2H), 8.35 - 8.29 (m, 1H), 8.19 - 8.12 (m, 2H), 7.80 (d, J = 10.8 Hz, 1H), 7.27 - 7.14 (m, 7H), 6.53 (s, 1H), 5.59 - 5.51 (m, 1H), 5.44 - 5.39 (m, 2H), 5.20 - 5.07 (m, 2H), 4.56 - 4.44 (m, 3H), 3.92 (s, 3H), 3.80 - 3.68 (m, 5H), 3.41 (s, 1H), 3.21 - 3.12 (m, 2H), 2.83 - 2.74 (m, 1H), 2.58 - 2.55 (m, 3H), 2.39 (s, 4H), 2.18 - 2.03 (m, 4H), 1.93 - 1.78 (m, 2H), 0.87 (t, J = 7.3 Hz, 3H).

### 6.2 Preparation of antibody-drug conjugates

**Table 8**

| Molecule name | Antibody clone | Linker-cytotoxin | DAR |
|---|---|---|---|
| ADC-1 | Zalutumumab | X2 | 8 |
| ADC-2 | hu3F8 clone 2 | X2 | 8 |
| ADC-3 | Cetuximab | X2 | 8 |
| ADC-4 | DBXT001-01 | X2 | 4 |
| ADC-5 | DBXT001-01 | X2 | 6 |
| ADC-6 | DBXT001-01 | X2 | 8 |
| ADC-7 | DBXT001-01 | X1 | 4 |
| ADC-8 | DBXT001-01 | X1 | 6 |
| ADC-9 | DBXT001-01 | X1 | 8 |
| ADC-10 | SI-1X6.4 | X2 | 8 |
| ADC-11 | Isotype IgG1 | X2 | 8 |
| ADC-12 | Isotype IgG1 | X1 | 8 |
| ADC-13 | DBXT005-01 | X2 | 6 |
| ADC-14 | SI-1X6.4 | Ed-04 | 8 |

| | | | |
|---|---|---|---|
| Isotype IgG1 source: purchased from Biointron (Taicang). | | | |

### Preparation of DBXT001-01-X1

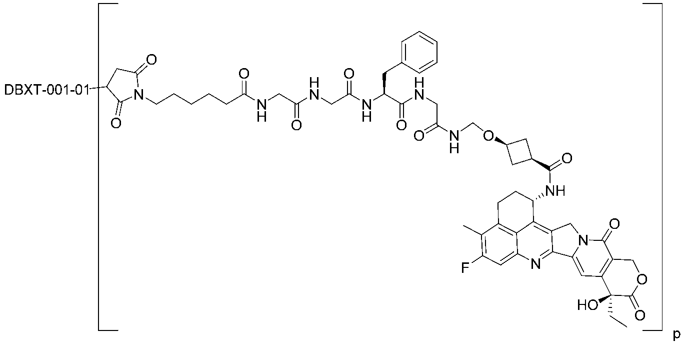

### ADC-7 (DBXT001-01-X1-DAR4)

To a buffer solution of the antibody DBXT001-01 (PBS pH 7.2; 70 mg, 8.52 mg/mL, 0.47 µmol) were added a 10 mM EDTA solution (0.875 mL) and a prepared tris(2-carboxyethyl)phosphine hydrochloride solution (3.48 mM, 0.379 mL, 1.32 µmol). The mixture was placed in a constant-temperature stirrer at a stirring speed of 60 rpm and allowed to react at 22 °C for 2 h, and then the reaction was stopped.

X1 (2.61 mg, 2.43 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 53 mL; eluent: 30 mM histidine-hydrochloric acid pH 5.5), and subjected to ultrafiltration concentration using a 30 KD ultrafiltration tube to give a solution of an exemplary product ADC-7 (30 mM histidine-hydrochloric acid pH 5.5; 68 mg, 22 mg/mL, yield: 97%), which was then stored at -80 °C. HIC DAR analysis was performed, and the calculated DAR value n = 4.06; the SEC main peak purity was 98.2%.

### ADC-8 (DBXT001-01-X1-DAR6)

To a buffer solution of the antibody DBXT001-01 (PBS pH 7.2; 70 mg, 8.52 mg/mL, 0.47 µmol) were added a 10 mM EDTA solution (0.875 mL) and a prepared tris(2-carboxyethyl)phosphine hydrochloride solution (3.48 mM, 0.598 mL, 2.08 µmol). The mixture was placed in a constant-temperature stirrer at a stirring speed of 60 rpm and allowed to react at 22 °C for 2 h, and then the reaction was stopped.

X1 (4.30 mg, 4.00 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 53 mL; eluent: 30 mM histidine-hydrochloric acid pH 5.5), and subjected to ultrafiltration concentration using a 30 KD ultrafiltration tube to give a solution of an exemplary product ADC-8 (30 mM histidine-hydrochloric acid pH 5.5; 69 mg, 23 mg/mL, yield: 99%), which was then stored at -80 °C. HIC DAR analysis was performed, and the calculated DAR value n = 6.11; the SEC main peak purity was 99.2%.

### ADC-9 (DBXT001-01-X1-DAR8)

To a buffer solution of the antibody DBXT001-01 (PBS pH 7.2; 70 mg, 8.52 mg/mL, 0.47 µmol) were added a 10 mM EDTA solution (0.875 mL) and a prepared tris(2-carboxyethyl)phosphine hydrochloride solution (3.48 mM, 1.12 mL, 3.89 µmol). The mixture was placed in a constant-temperature stirrer at a stirring speed of 60 rpm and allowed to react at 22 °C for 2 h, and then the reaction was stopped.

X1 (5.05 mg, 4.70 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 53 mL; eluent: 30 mM histidine-hydrochloric acid pH 5.5), and subjected to ultrafiltration concentration using a 30 KD ultrafiltration tube to give a solution of an exemplary product ADC-9 (30 mM histidine-hydrochloric acid pH 5.5; 68 mg, 22.4 mg/mL, yield: 97%), which was then stored at - 80 °C.

HIC DAR analysis was performed, and the calculated DAR value n = 7.99; the SEC main peak purity was 99.0%.

### Preparation of DBXT001-01-X2

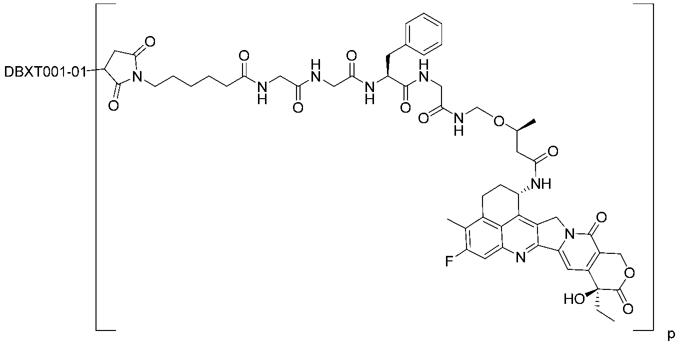

### ADC-4 (DBXT001-01-X2-DAR4)

To a buffer solution of the antibody DBXT001-01 (PBS pH 7.4; 70 mg, 8.52 mg/mL, 0.47 µmol) were added a 10 mM EDTA solution (0.875 mL) and a prepared tris(2-carboxyethyl)phosphine hydrochloride solution (3.48 mM, 0.379 mL, 1.32 µmol). The mixture was placed in a constant-temperature stirrer at a stirring speed of 60 rpm and allowed to react at 37 °C for 2 h, and then the reaction was stopped.

X2 (2.75 mg, 2.59 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 53 mL; eluent: 30 mM histidine-hydrochloric acid pH 5.5), and subjected to ultrafiltration concentration using a 30 KD ultrafiltration tube to give a solution of an exemplary product ADC-4 (30 mM histidine-hydrochloric acid pH 5.5; 67.2 mg, 22 mg/mL, yield: 96%), which was then stored at - 80 °C.

HIC DAR analysis was performed, and the calculated DAR value n = 4.10; the SEC main peak purity was 98.4%.

### ADC-5 (DBXT001-01-X2-DAR6)

To a buffer solution of the antibody DBXT001-01 (PBS pH 7.4; 70 mg, 8.52 mg/mL, 0.47 µmol) were added a 10 mM EDTA solution (0.875 mL) and a prepared tris(2-carboxyethyl)phosphine hydrochloride solution (3.48 mM, 0.598 mL, 2.08 µmol). The mixture was placed in a constant-temperature stirrer at a stirring speed of 60 rpm and allowed to react at 37 °C for 2 h, and then the reaction was stopped.

X2 (4.58 mg, 4.31 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 3 mL; eluent: 30 mM histidine-hydrochloric acid pH 5.5), and subjected to ultrafiltration concentration using a 30 KD ultrafiltration tube to give a solution of an exemplary product ADC-5 (30 mM histidine-hydrochloric acid pH 5.5; 66.5 mg, 25 mg/mL, yield: 95%), which was then stored at - 80 °C.

HIC DAR analysis was performed, and the calculated DAR value n = 6.05; the SEC main peak purity was 98.9%.

### ADC-6 (DBXT001-01-X2-DAR8)

To a buffer solution of the antibody DBXT001-01 (PBS pH 7.2; 70 mg, 8.52 mg/mL, 0.47 µmol) were added a 10 mM EDTA solution (0.875 mL) and a prepared tris(2-carboxyethyl)phosphine hydrochloride solution (3.48 mM, 1.12 mL, 3.89 µmol). The mixture was placed in a constant-temperature stirrer at a stirring speed of 60 rpm and allowed to react at 22 °C for 2 h, and then the reaction was stopped.

X2 (5.49 mg, 5.17 µmol) was dissolved in 1.75 mL of DMA, and the resulting solution was added to the above solution. The resulting mixture was placed in a constant-temperature stirrer at a stirring speed of 60 rpm and allowed to react at 22 °C for 2 h with shaking, and then the reaction was stopped. After filtration with activated carbon, the reaction solution was desalted and purified on AKTA using a G-25 gel column (desalting column: HiPrep 26/10 Desalting column, 53 mL; eluent: 30 mM histidine-hydrochloric acid pH 5.5), and subjected to ultrafiltration concentration using a 30 KD ultrafiltration tube to give a solution of an exemplary product ADC-6 (30 mM histidine-hydrochloric acid pH 5.5; 67.9 mg, 29.2 mg/mL, yield: 97%), which was then stored at - 80 °C.

HIC DAR analysis was performed, and the calculated DAR value n = 7.98; the SEC main peak purity was 99.0%.

### ADC-13 (DBXT005-01-X2, DAR6)

In PBS 7.4 buffer (PBS 7.4, 2 mM EDTA, pH 7.4), the antibody DBXT005-01 was reduced with 20 equivalents of tris(2-carboxyethyl)phosphine (TCEP). The reaction was carried out at 22 °C for 3 h with shaking on a shaker (at a shaking speed of 60 rpm).

After intermediate monitoring, 5 equivalents of tris(2-carboxyethyl)phosphine (TCEP) were supplemented, and the mixture was allowed to react at 22 °C for 1 h with shaking on a shaker. The reaction solution was used directly for the next conjugation reaction without removing the excess TCEP.

To the reduced antibody were slowly added 200 mM histidine-hydrochloric acid pH 6.0 (to achieve a final concentration of 20 mM histidine-hydrochloric acid in the conjugation reaction solution) in dimethyl sulfoxide containing 0.1% v/v acetic acid, and a linker-cytotoxin (linker-payload) solution (10 equivalents, 10 mM X2 solution (in dimethyl sulfoxide containing 0.1% v/v acetic acid)) at room temperature. The reaction system contained 3% v/v dimethyl sulfoxide (DMSO) containing 0.1% v/v acetic acid. After thoroughly mixing, the conjugation reaction solution was allowed to react at 22 °C for 1.5 h with shaking on a shaker (at a shaking speed of 60 rpm).

After intermediate monitoring, linker-cytotoxin (linker-payload) (2 equivalents, 10 mM X2 solution (in dimethyl sulfoxide containing 0.1% v/v acetic acid)) was supplemented. After thoroughly mixing, the conjugation reaction solution was further allowed to react at 22 °C for 2 h with shaking on a shaker (at a shaking speed of 60 rpm).

After the reaction was completed, the ADC solution was buffer-exchanged into a storage buffer (20 mM histidine-hydrochloric acid, pH 6.0) using a desalting column (40 K). A sample was taken and sent for detection, resulting in an ADC product.

The detection results are as follows:

| Name | DAR value (mass spectrometry detection) | SEC aggregation (%) | Free small molecule (mol%) | Endotoxin (EU/mg) |
|---|---|---|---|---|
| ADC-13 | 5.99 | 2.39 | <2.02% | <0.400 |

ADC-14 (BL-B01D1, DAR8) was prepared with reference to the patent WO2023083381A1, and the details are as follows:
In PBS 7.4 buffer (PBS 7.4, 2 mM EDTA, pH 7.4), the antibody SI-1X6.4 was reduced with 15 equivalents of tris(2-carboxyethyl)phosphine (TCEP). The reaction was carried out at 22 °C for 17.5 h with shaking on a shaker (at a shaking speed of 60 rpm). The reaction solution was used directly for the next conjugation reaction without removing the excess TCEP.

To the reduced antibody were slowly added dimethyl sulfoxide (DMSO) and linker-cytotoxin (linker-payload) (10 equivalents, 10 mM solution of Ed-04 in DMSO) at room temperature. The reaction system contained 10% v/v dimethyl sulfoxide (DMSO). After thoroughly mixing, the conjugation reaction solution was allowed to react at 22 °C for 2 h with shaking on a shaker (at a shaking speed of 60 rpm).

After intermediate monitoring, linker-cytotoxin (linker-payload) (2 equivalents, 10 mM solution of Ed-04 in DMSO) was supplemented. After thoroughly mixing, the conjugation reaction solution was further allowed to react at 22 °C for 1.5 h with shaking on a shaker (at a shaking speed of 60 rpm).

After the reaction was completed, 200 mM histidine-hydrochloric acid pH 5.5 was added to the reaction solution to achieve a final concentration of 20 mM histidine-hydrochloric acid in the reaction solution. Then, the reaction solution was filtered using a 0.22 µm PES syringe filter. Subsequently, the ADC solution was buffer-exchanged into a storage buffer (20 mM histidine-hydrochloric acid, pH 5.5) using AKTA with a desalting column (HiPrep^{™} 26/10 Desalting). The reaction solution was concentrated using Amicon (30 K MWCO), and finally filtered using a 0.22 µm PES syringe filter to give an ADC product. A sample was taken and sent for detection.

The detection results are as follows:

| Name | DAR value (mass spectrometry detection) | SEC aggregation (%) | Free small molecule (mol%) | Endotoxin (EU/mg) |
|---|---|---|---|---|
| ADC-14 | 8 | 1.73 | <0.97% | <0.208 |

ADC-1 to 3, ADC-10, and ADC-11 were prepared by referring to the preparation process of DBXT001-01-X2, except that the corresponding antibody clones in Table 8 were replaced.

By replacing the antibody clone DBXT001-01 in Table 8 with DBXT001-02 to 08, DBXT003-01 to 08, and DBXT004-01 to 08, corresponding bispecific antibody-drug conjugates can be prepared by referring to the experimental procedures described above.

### Example 7: Evaluation of In Vivo Efficacy of Antibody-Drug Conjugates in Mice Bearing Human Esophageal Cancer Cell Line OE-19 with Low EGFR Expression and Medium HER3 Expression

In order to study the inhibitory effect of ADC-6 on *in vivo* tumor formation induced by a human esophageal cancer cell line, after subcutaneous ectopic inoculation of OE-19 was performed in mice to form xenograft tumors, the anti-tumor effect of ADC-6 was evaluated and compared with the anti-tumor effects of parent mAb ADCs.
1. Test drugs and materials
   Blank control group (control group): normal saline
   ADC-1 (treatment group): day 0: 1 mg/kg, day 12: 3 mg/kg
   ADC-2 (treatment group): day 0: 1 mg/kg, day 12: 3 mg/kg
   ADC-6 (treatment group): day 0: 1 mg/kg, day 12: 3 mg/kg
2. Preparation method: All samples were prepared by dilution with normal saline.
3. Experimental animals: 6- to 7-week-old female BALB/c Nude mice, purchased from Jiangsu GemPharmatech Co., Ltd.
4. Experimental procedures:
   OE-19 (CL-00806) cells were cultured in an RPMI1640 medium containing 10% fetal bovine serum and 2 mM L-Glutamine. OE-19 cells in the exponential phase were harvested and resuspended in PBS to achieve an appropriate concentration for subcutaneous tumor inoculation in mice.

The experimental mice were inoculated subcutaneously on the right side of the back with 5 × 10⁶ OE-19 cells (the cells were resuspended in a 1:1 mixture of PBS and Matrigel), with 0.1 mL per mouse. Tumor growth was observed periodically.

When the tumors grew to about 140.15 mm³, the tumor-bearing mice were randomized into groups using StudyDirectorTM. Intravenous (i.v.) injection of the test samples was started on the day of grouping (day 0), and a total of 2 injections were administered, once on day 0 and once on day 12. The treatment groups received a dose of 1 mg/kg on day 0 and 3 mg/kg on day 12. The experimental endpoint was on day 25 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 9. Evaluation of anti-tumor efficacy of test substances in OE-19 cell subcutaneous xenograft tumor model**

| (calculations based on tumor volumes on day 25 after administration) | | | |
|---|---|---|---|
| Group | Tumor volume (mm³) ¹ | T/C (%)² | TGI (%) ² |
| Blank control group | 2193.72 + 468.29 | - | - |
| ADC-1 | 1978.95 + 332.17 | 90.21 | 9.79 |
| ADC-2 | 1267.10 + 413.32 | 57.76 | 42.24 |
| ADC-6 | 902.53 + 567.80 | 41.14 | 58.86 |

| | | | |
|---|---|---|---|
| 1. Tumor volume was expressed as mean + standard error. 2. Tumor growth inhibition is reflected by T/C (T/C (%) = T₂₅/V₂₅ × 100) and TGI (TGI (%) = [1 - (T₂₅ - T₀)/(V₂₅ - V₀)] × 100). | | | |

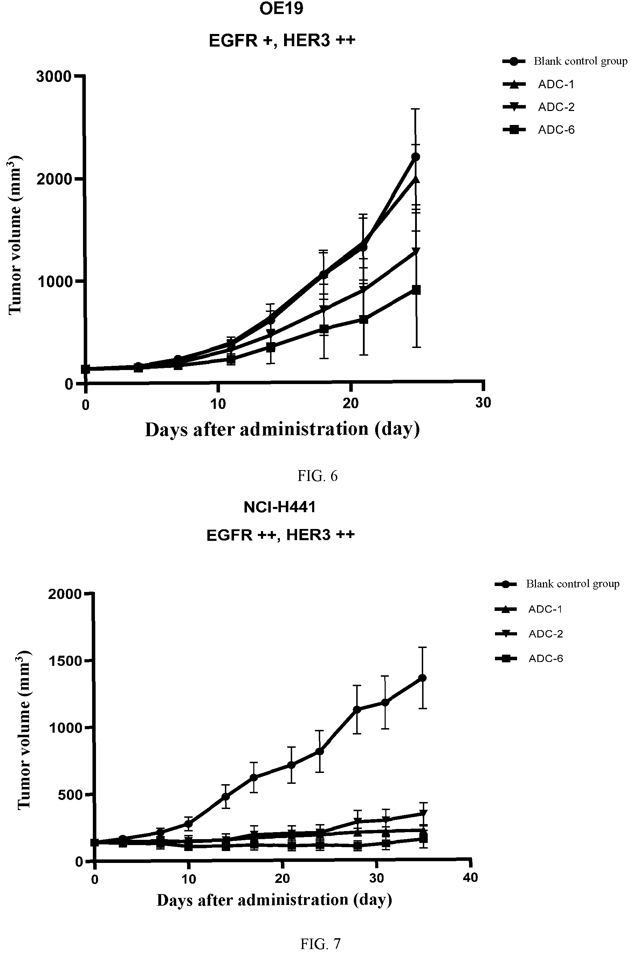

The experimental results are shown in FIG. 6 and Table 9, indicating that in the OE-19 model, the mAb ADC-1 (EGFR-ADC) failed to inhibit tumor growth, and the BsAb ADC-6 (EGFR-HER3 ADC) had a better anti-tumor effect as compared to the mAb ADC-2 (HER3-ADC).

### Experimental conclusion:

For the EGFR ADC-resistant model, the BsAb ADC had a better anti-tumor effect as compared to the mAb ADCs.

### Example 8: Evaluation of In Vivo Efficacy of Antibody-Drug Conjugates in Mice Bearing Human Non-Small Cell Lung Cancer Cell Line NCI-H441 with Medium EGFR Expression and Medium HER3 Expression

In order to study the inhibitory effect of ADC-6 on *in vivo* tumor formation induced by a human non-small cell lung cancer cell line, after subcutaneous ectopic inoculation of NCI-H441 was performed in mice to form xenograft tumors, the anti-tumor effect of ADC-6 was evaluated and compared with the anti-tumor effects of parent mAb ADCs.
1. Test drugs and materials
   Blank control group (control group): normal saline
   ADC-1 (treatment group): 0.5 mg/kg
   ADC-2 (treatment group): 0.5 mg/kg
   ADC-6 (treatment group): 0.5 mg/kg
2. Preparation method: All samples were prepared by dilution with normal saline.
3. Experimental animals: 6- to 7-week-old male NU/NU mice, purchased from Beijing Vital River Laboratory Animal Technology Co., Ltd.
4. Experimental procedures:
   NCI-H441 (CL-00759) cells were cultured in an RPMI1640 medium containing 10% fetal bovine serum. NCI-H441 cells in the exponential phase were harvested and resuspended in PBS to achieve an appropriate concentration for subcutaneous tumor inoculation in mice.

The experimental mice were inoculated subcutaneously on the right side of the back with 5 × 10⁶ NCI-H441 cells (the cells were resuspended in a 1:1 mixture of PBS and Matrigel), with 0.1 mL per mouse. Tumor growth was observed periodically.

When the tumors grew to about 139.12 mm³, the tumor-bearing mice were randomized into groups using StudyDirectorTM. Intravenous (i.v.) injection of the test samples was started on the day of grouping (day 0), and a total of 1 injection was administered. The treatment groups received a dose of 0.5 mg/kg on day 0. The experimental endpoint was on day 35 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 10. Evaluation of anti-tumor efficacy of test substances in NCI-H441 cell subcutaneous xenograft tumor model**

| (calculations based on tumor volumes on day 35 after administration) | | | | |
|---|---|---|---|---|
| Group | Tumor volume (mm³) ¹ | T/C (%)² | TGI (%) ² | p value³ |
| Blank control group, | 1355.83 + 228.07 | - | - | - |
| ADC-1 | 218.89 + 33.69 | 16.14 | 83.86 | ** |
| ADC-2 | 341.42 + 83.18 | 25.18 | 74.82 | ns |
| ADC-6 | 152.85 + 65.97 | 11.27 | 88.73 | *** |

| | | | | |
|---|---|---|---|---|
| 1. Tumor volume was expressed as mean + standard error. 2. Tumor growth inhibition is reflected by T/C (T/C (%) = T₃₅/V₃₅ × 100) and TGI (TGI (%) = [1 - (T₃₅ - T₀)/(V₃₅ - V₀)] × 100). 3*. p* values were calculated based on the tumor volumes (* *indicates p < 0.05,* ** *indicates p < 0.01,* *** *indicates p < 0.001, and ns indicates no significant significance).* | | | | |

The experimental results are shown in FIG. 7 and Table 10, indicating that in the NCI-H441 model, ADC-6 had a better anti-tumor effect as compared to ADC-2.

### Experimental conclusion:

In the model with comparable levels of EGFR and HER3 expression, the BsAb ADC had a better anti-tumor effect as compared to the mAb ADCs.

### Example 9: Evaluation of In Vivo Efficacy of Antibody-Drug Conjugates in Mice Bearing Human Oral Squamous Cell Carcinoma Cell Line CAL-27 with High EGFR Expression and Low HER3 Expression

In order to study the inhibitory effect of ADC-6 on *in vivo* tumor formation induced by a human oral squamous cell carcinoma cell line, after subcutaneous ectopic inoculation of CAL-27 was performed in mice to form xenograft tumors, the anti-tumor effect of ADC-6 was evaluated and compared with the anti-tumor effects of parent mAb ADCs and an anti-EGFR mAb.
1. Test drugs and materials
   Blank control group (control group): normal saline
   ADC-1 (treatment group): 3 mg/kg
   ADC-2 (treatment group): 3 mg/kg
   ADC-6 (treatment group): 3 mg/kg
   Anti-EGFR mAb zalutumumab (treatment group): 3 mg/kg
2. Preparation method: All samples were prepared by dilution with normal saline.
3. Experimental animals: 6- to 7-week-old female NOD/Scid mice, purchased from Jiangsu GemPharmatech Co., Ltd.
4. Experimental procedures:
   CAL-27 (CL-00599) cells were cultured in a DMEM medium containing 10% fetal bovine serum. CAL-27 cells in the exponential phase were harvested and resuspended in PBS to achieve an appropriate concentration for subcutaneous tumor inoculation in mice.

The experimental mice were inoculated subcutaneously on the right side of the back with 1 × 10⁷ CAL-27 cells (the cells were resuspended in a 1:1 mixture of PBS and Matrigel), with 0.1 mL per mouse. Tumor growth was observed periodically.

When the tumors grew to about 144.75 mm³, the tumor-bearing mice were randomized into groups using StudyDirectorTM. Intravenous (i.v.) injection of the test samples was started on the day of grouping (day 0), and a total of 2 injections were administered, once on day 0 and once on day 14. The treatment groups received a dose of 3 mg/kg. The experimental endpoint was on day 27 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 11. Evaluation of anti-tumor efficacy of test substances in CAL-27 cell subcutaneous xenograft tumor model**

| (calculations based on tumor volumes on day 27 after administration) | | | | |
|---|---|---|---|---|
| Group | Tumor volume (mm³) ¹ | T/C (%) ² | TGI (%) ² | *p* value³ |
| Blank control group | 1193.68 ± 152.76 | - | - | - |
| ADC-1 | 63.57 ± 10.46 | 5.33 | 94.67 | *** |
| ADC-2 | 1171.19 ± 213.03 | 98.12 | 1.88 | ns |
| ADC-6 | 158.74 ± 68.86 | 13.30 | 86.70 | *** |
| Anti-EGFR mAb | 689.00 ± 235.58 | 57.72 | 42.28 | ns |

| | | | | |
|---|---|---|---|---|
| 1. Tumor volume was expressed as mean + standard error. 2. Tumor growth inhibition is reflected by T/C (T/C (%) = T₂₇/V₂₇ × 100) and TGI (TGI (%) = [1 - (T₂₇ - T₀)/(V₂₇ - V₀)] × 100). 3. *p* values were calculated based on the tumor volumes (* *indicates p < 0.05,* ** *indicates p < 0.01,* *** *indicates p < 0.001, and ns indicates no significant significance).* | | | | |

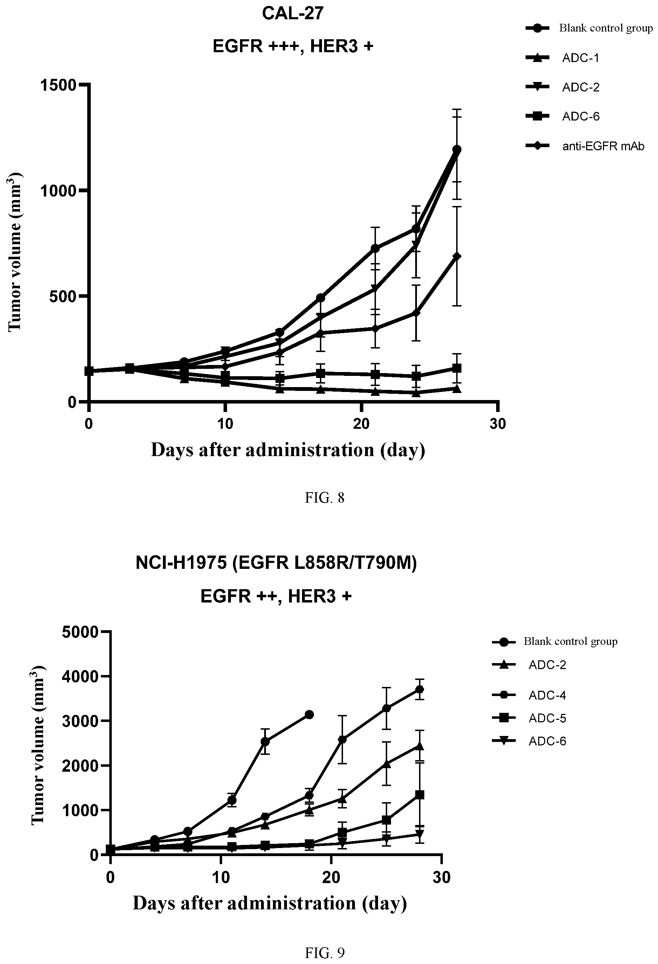

The experimental results are shown in FIG. 8 and Table 11, indicating that in the CAL-27 model, ADC-1 and ADC-6 both had significant anti-tumor effects, and were superior to ADC-2 and the anti-EGFR mAb. Experimental conclusion:
In the model with high EGFR expression and low HER3 expression that is sensitive to EGFR targeting, the BsAb ADC had a better anti-tumor effect as compared to the anti-EGFR mAb or HER3 ADC, and was not inferior to the EGFR ADC.

### Example 10: Evaluation of In Vivo Efficacy of Antibody-Drug Conjugates with Different DAR Values in NCI-H1975 and OE-19 Tumor-Bearing Mice

In order to study the inhibitory effects of conjugates of DBXT001-01 with linker-cytotoxin X2 with different DAR values on *in vivo* tumor formation induced by the human non-small cell lung cancer cell line **NCI-H1975** and human esophageal cancer cell line OE-19, after subcutaneous ectopic inoculation of **NCI-H1975** or OE-19 was performed on the right side of the back of mice to form xenograft tumors, the anti-tumor effects of the BsAb-toxin conjugates with different DAR values were evaluated.

The test drugs and administration regimens are shown in Tables 12 and 13.

**Table 12. Administration routes, doses and regimens in human lung cancer NCI-H1975 subcutaneous xenograft model**

| Group | Number of animals | Administration group | Dose (mg/kg) | Administration route | Administration cycle |
|---|---|---|---|---|---|
| 1 | 5 | Blank control group | - | i.v. | Q2W × 2 |
| 2 | 5 | ADC-2 | 10 | i.v. | Q2W × 3 |
| 3 | 5 | ADC-4 | 3 | i.v. | Q2W × 2 |
| 4 | 5 | ADC-5 | 3 | i.v. | Q2W × 3 |
| 5 | 5 | ADC-6 | 3 | i.v. | Q2W × 3 |

**Table 13. Administration routes, doses and regimens in human esophageal cancer OE-19 cell line subcutaneous xenograft model**

| Group | Number of animals | Administration group | Dose (mg/kg) | Administration route | Administration cycle |
|---|---|---|---|---|---|
| 1 | 5 | Blank control group | - | i.v. | Q2W × 2 |
| 2 | 5 | ADC-1 | 6 | i.v. | Q2W × 2 |
| 3 | 5 | ADC-2 | 6 | i.v. | Q2W × 2 |
| 4 | 5 | ADC-4 | 6 | i.v. | Q2W × 2 |
| 5 | 5 | ADC-5 | 6 | i.v. | Q2W × 2 |
| 6 | 5 | ADC-6 | 6 | i.v. | Q2W × 2 |

| | | | | | |
|---|---|---|---|---|---|
| 2. Preparation method: All samples were prepared by dilution with normal saline. 3. Experimental animals: 6- to 8-week-old female Balb/c nude mice, purchased from Jiangsu GemPharmatech Co., Ltd. 4. Experimental procedures: NCI-H1975 (CL-00650) cells were cultured in an RPMI1640 medium containing 10% fetal bovine serum, and OE-19 (CL-00806) cells were cultured in an RPMI1640 medium containing 10% fetal bovine serum and 2 mM L-Glutamine. Cells in the exponential phase were harvested and resuspended in PBS to achieve an appropriate concentration for subcutaneous tumor inoculation in nude mice. | | | | | |

The experimental mice were inoculated subcutaneously on the right side of the back with 5 × 10⁶ NCI-H1975 cells (the cells were resuspended in PBS), with 0.1 mL per mouse. Tumor growth was observed periodically. The experimental mice were inoculated subcutaneously on the right side of the back with 5 × 10⁶ OE-19 cells (the cells were resuspended in a 1:1 mixture of PBS and Matrigel), with 0.1 mL per mouse. Tumor growth was observed periodically.

When the tumors grew to about 121.71 mm³ (NCI-H1975) or 139.93 mm³ (OE-19), the tumor-bearing mice were randomized into groups using StudyDirectorTM. The day of grouping was designated as day 0. The specific administration regimens are shown in Tables 12 and 13. The experimental endpoint was on day 31 after grouping (NCI-H1975), or day 35 after grouping (OE-19). The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 14. Evaluation of anti-tumor efficacy of test substances in NCI-H1975 cell subcutaneous xenograft tumor model**

| (calculations based on tumor volumes on day 18¹ after administration) | | | | |
|---|---|---|---|---|
| Group | Tumor volume (mm³) ² | T/C (%)³ | TGI (%) ³ | *p* value⁴ |
| Blank control group | 3138.18 + 34.92 | - | - | - |
| ADC-2 | 1007.29 + 132.59 | 32.10 | 67.90 | *** |
| ADC-4 | 1331.77 + 150.56 | 42.44 | 57.56 | * |
| ADC-5 | 243.30 + 80.55 | 7.75 | 92.25 | *** |
| ADC-6 | 212.18 + 106.87 | 6.76 | 93.24 | *** |

**Table 15. Evaluation of anti-tumor efficacy of test substances in OE-19 cell subcutaneous xenograft tumor model**

| (calculations based on tumor volumes on day 21¹ after administration) | | | | |
|---|---|---|---|---|
| Group | Tumor volume (mm³) ² | T/C (%)³ | TGI (%) ³ | *p* value⁴ |
| Blank control group | 1734.6 + 617.12 | - | - | - |
| ADC-1 | 1078.01 + 263.36 | 62.15 | 37.85 | ns |
| ADC-2 | 741.6 + 195.8 | 42.75 | 57.25 | ns |
| ADC-4 | 595.89 + 141.93 | 34.35 | 65.65 | ns |
| ADC-5 | 744.13 ± 229.18 | 42.90 | 57.10 | ns |
| ADC-6 | 305.00 + 56.98 | 17.58 | 82.42 | * |

1. Since some mice in the NCI-H1975 blank control group reached euthanasia criteria after 18 days, the statistical analysis data were incomplete, so that statistical analysis was performed on day 18 with the complete number of animals. Similarly, some mice in the OE-19 blank control group reached euthanasia criteria after 21 days, the statistical analysis data were incomplete, so that statistical analysis was performed on day 21 with the complete number of animals.
2. Tumor volume was expressed as mean + standard error.
3. Tumor growth inhibition is reflected by T/C (T/C (%) = T_{D}/V_{D} × 100) and TGI (TGI (%) = [1 - (T_{D} - T₀)/(V_{D} - V₀)] × 100).
4. *p* values were calculated based on the tumor volumes *(* indicates p < 0.05,* ** *indicates p < 0.01,* *** *indicates p < 0.001, and ns indicates no significant significance).*

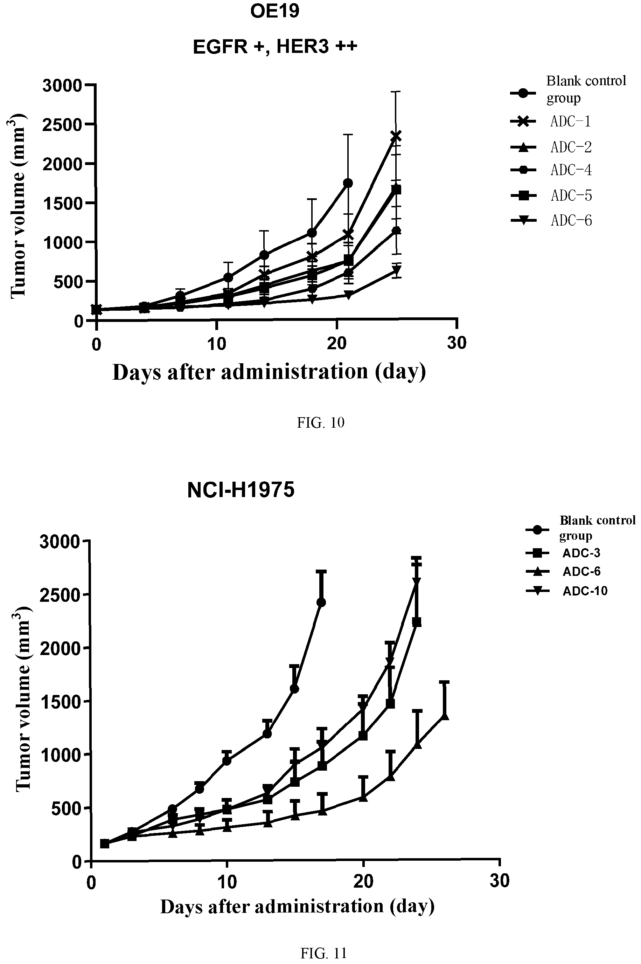

The experimental results are shown in FIGs. 9 and 10 and Tables 14 and 15, indicating that in the administration regimens of the NCI-H1975 model, ADC-2, ADC-4, ADC-5, and ADC-6 all had anti-tumor effects.

### Experimental conclusion:

The conjugate of the BsAb targeting EGFR and HER3 of the present disclosure with linker-cytotoxin X2 had an anti-tumor effect, and the anti-tumor effect was enhanced along with the increase of the DAR value.

### Example 11: Comparison of In Vivo Efficacy of Antibody-Drug Conjugate Disclosed Herein and Control Antibody Conjugates in NCI-H1975 Tumor-Bearing Mice

In order to compare the inhibitory effects of a conjugate of DBXT001-01 with linker-cytotoxin X2 and a conjugate of a control antibody SI-1X6.4 with linker-cytotoxin X2 on *in vivo* tumor formation induced by the human non-small cell lung cancer cell line NCI-H1975, after subcutaneous ectopic inoculation of NCI-H1975 was performed on the right side of the back of mice to form xenograft tumors, the anti-tumor effects of the two conjugates were evaluated.

The test drugs and administration regimens are shown in Table 16.

**Table 16. Administration routes, doses and regimens in human lung cancer NCI-H1975 subcutaneous xenograft model**

| Group | Number of animals | Administration group | Dose (mg/kg) | Administration route | Administration cycle |
|---|---|---|---|---|---|
| 1 | 5 | Blank control group | - | Intravenous injection | Single injection |
| 2 | 5 | ADC-3 | 3 | Intravenous injection | Single injection |
| 3 | 5 | ADC-6 | 3 | Intravenous injection | Single injection |
| 4 | 5 | ADC-10 | 4^{a} | Intravenous injection | Single injection |

| | | | | | |
|---|---|---|---|---|---|
| a. Molar conversion was performed according to the molecular weight, so as to ensure consistent molar concentrations across all treatment groups. 1. Preparation method: All samples were prepared by dilution with normal saline. 2. Experimental animals: 6- to 8-week-old female Balb/c nude mice, purchased from Jiangsu GemPharmatech Co., Ltd. 3. Experimental procedures: NCI-H1975 (CL-00650) cells were cultured in an RPMI1640 medium containing 10% fetal bovine serum. | | | | | |

Cells in the exponential phase were harvested and resuspended in PBS to achieve an appropriate concentration for subcutaneous tumor inoculation in nude mice.

The experimental mice were inoculated subcutaneously on the right side of the back with 5 × 10⁶ NCI-H1975 cells (the cells were resuspended in a 1:1 mixture of DPBS and Matrigel), with 0.2 mL per mouse. Tumor growth was observed periodically.

When the tumors grew to about 164 mm³ (NCI-H1975), the tumor-bearing mice were randomized into groups using StudyDirectorTM. The day of grouping was designated as day 0. The specific administration regimens are shown in Table 16. The experimental endpoint was on day 26 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a* and *b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 17. Evaluation of anti-tumor efficacy of test substances in NCI-H1975 cell subcutaneous xenograft tumor model**

| (calculations based on tumor volumes on day 17¹ after administration) | | | | |
|---|---|---|---|---|
| Group | Tumor volume (mm³) ² | T/C (%)³ | TGI (%) ³ | *p* value⁴ |
| Blank control group | 2414.60 + 288.22 | - | - | - |
| ADC-3 | 880.55 + 227.95 | 34.67 | 68.16 | *** |
| ADC-6 | 461.66 + 158.24 | 16.73 | 86.82 | *** |
| ADC-10 | 1054.42 + 175.66 | 40.51 | 60.45 | *** |

1. Since some mice in the NCI-H1975 blank control group reached euthanasia criteria after 17 days, the statistical analysis data were incomplete, so that statistical analysis was performed on day 17 with the complete number of animals.
2. Tumor volume was expressed as mean + standard error.
3. Tumor growth inhibition is reflected by T/C (T/C (%) = T₁₇/V₁₇ × 100) and TGI (TGI (%) = [1 - (T₁₇ - T₀)/(V₁₇ - V₀)] × 100).
4. *p* values were calculated based on the tumor volumes (* *indicates p < 0.05,* ** *indicates p < 0.01,* *** *indicates p < 0.001, and ns indicates no significant significance).*

The experimental results are shown in FIG. 11 and Table 17, indicating that in the administration regimens of the NCI-H1975 model, ADC-3, ADC-6, and ADC-10 all had significant anti-tumor effects, among which ADC-6 showed the strongest anti-tumor effect, and ADC-3 and ADC-10 had comparable efficacy.

### Experimental conclusion:

The conjugate of the BsAb targeting EGFR and HER3 of the present disclosure with linker-cytotoxin X2 (DAR8) had a stronger anti-tumor effect as compared to the conjugate of the control antibody SI-1X6.4 with linker-cytotoxin X2 (DAR8).

### Example 12: Comparison of In Vivo Efficacy of Antibody-Drug Conjugate Disclosed Herein and Control Antibody Conjugates in OE-19 Tumor-Bearing Mice

In order to compare the inhibitory effects of a conjugate of DBXT001-01 with linker-cytotoxin X2 or linker-cytotoxin X1 and a conjugate of a control antibody SI-1X6.4 with linker-cytotoxin X2 on *in vivo* tumor formation induced by the human esophageal cancer cell line OE-19, after subcutaneous ectopic inoculation of OE-19 was performed on the right side of the back of mice to form xenograft tumors, the *in vivo* anti-tumor effects were evaluated.

The test drugs and administration regimens are shown in Table 18.

**Table 18. Administration routes, doses and regimens in human esophageal cancer OE-19 subcutaneous xenograft model**

| Group | Number of animals | Administration group | Dose (mg/kg) | Administration route | Administration cycle |
|---|---|---|---|---|---|
| 1 | 5 | Blank control group | - | | |
| 2 | 5 | ADC-11 | 7.5 | Intravenous injection | Once a week, for a total of 3 times |
| 3 | 5 | ADC-3 | 7.5 | Intravenous injection | Once a week, for a total of 3 times |
| 4 | 5 | ADC-6 | 7.5 | Intravenous injection | Once a week, for a total of 3 times |
| 5 | 5 | ADC-10 | 10^{a} | Intravenous injection | Once a week, for a total of 3 times |
| 6 | 5 | ADC-9 | 7.5 | Intravenous injection | Once a week, for a total of 3 times |

| | | | | | |
|---|---|---|---|---|---|
| a. Molar conversion was performed according to the molecular weight, so as to ensure consistent molar concentrations across all treatment groups. 1. Preparation method: All samples were prepared by dilution with normal saline. 2. Experimental animals: 6- to 8-week-old female Balb/c nude mice, purchased from Jiangsu GemPharmatech Co., Ltd. 3. Experimental procedures: OE-19 (CL-00806) cells were cultured in an RPMI1640 medium containing 10% fetal bovine serum and 2 mM L-Glutamine. | | | | | |

The experimental mice were inoculated subcutaneously on the right side of the back with 5 × 10⁶ OE-19 cells (the cells were resuspended in a 1:1 mixture of PBS and Matrigel), with 0.1 mL per mouse. Tumor growth was observed periodically.

When the tumors grew to about 138 mm³, the tumor-bearing mice were randomized into groups using StudyDirectorTM. The day of grouping was designated as day 0. The specific administration regimens are shown in Table 18. The experimental endpoint was on day 21 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a* and *b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 19. Evaluation of anti-tumor efficacy of test substances in OE-19 cell subcutaneous xenograft tumor model**

| (calculations based on tumor volumes on day 21 after administration) | | | | |
|---|---|---|---|---|
| Group | Tumor volume (mm³) ¹ | T/C (%)² | TGI (%) ² | *p* value³ |
| Blank control group | 2621.07 + 438.01 | - | - | - |
| ADC-11 | 2486.00 + 454.45 | 94.85 | 5.15 | ns |
| ADC-3 | 1601.60 + 371.78 | 61.10 | 38.90 | ns |
| ADC-6 | 704.78 ± 121.82 | 26.89 | 73.11 | * |
| ADC-10 | 1646.57 + 368.12 | 62.82 | 37.18 | ns |
| ADC-9 | 1461.81 + 347.47 | 55.77 | 44.23 | ns |

| | | | | |
|---|---|---|---|---|
| 1. Tumor volume was expressed as mean + standard error. 2. Tumor growth inhibition is reflected by T/C (T/C (%) = T₂₁/V₂₁ × 100) and TGI (TGI (%) = [1 - (T₂₁ - T₀)/(V₂₁ - V₀)] × 100). *3. p* values were calculated based on the tumor volumes (* *indicates p < 0.05,* ** *indicates p < 0.01,* *** *indicates p < 0.001, and ns indicates no significant significance).* | | | | |

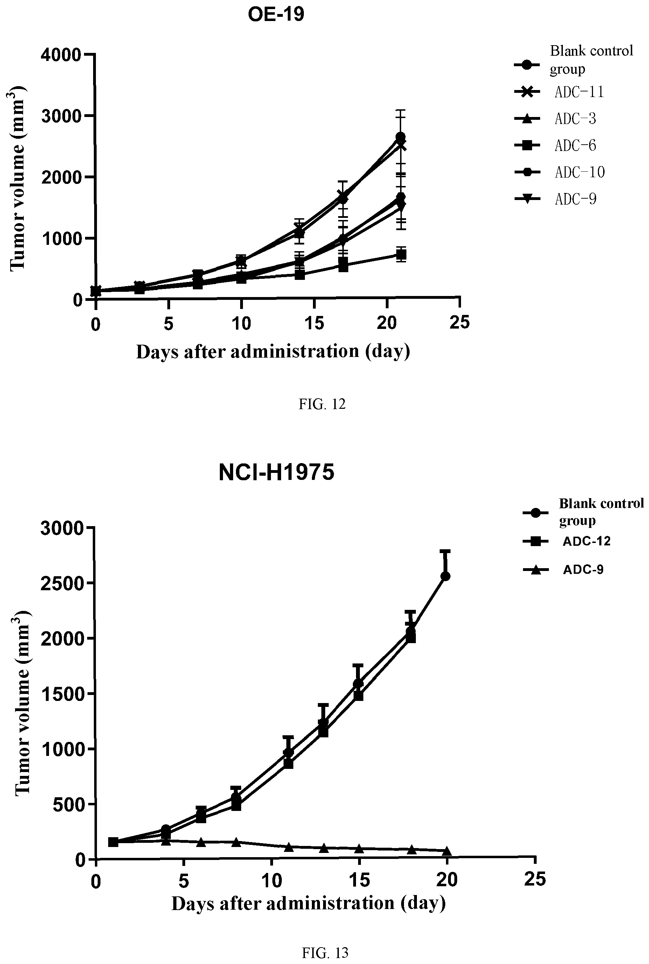

The experimental results are shown in FIG. 12 and Table 19, indicating that in the administration regimens of the OE-19 model, ADC-3, ADC-6, ADC-9, and ADC-10 all had anti-tumor effects, among which ADC-6 showed the strongest anti-tumor effect, and ADC-3, ADC-9 and ADC-10 had comparable efficacy.

### Experimental conclusion:

In the model resistant to EGFR-targeted therapy, the conjugate of the BsAb targeting EGFR and HER3 of the present disclosure with linker-cytotoxin X2 (DAR8) was able to overcome the EGFR resistance, and had a stronger anti-tumor effect as compared to the conjugate of the control antibody SI-1X6.4 with linker-cytotoxin X2 (DAR8).

### Example 13: Evaluation of In Vivo Efficacy of Conjugates of Antibody Drugs with Linker-Cytotoxin X1 in NCI-H1975 Tumor-Bearing Mice

In order to study the inhibitory effect of a conjugate of DBXT001-01 with linker-cytotoxin X1 (DAR8) on *in vivo* tumor formation induced by the human non-small cell lung cancer cell line NCI-H1975, after subcutaneous ectopic inoculation of NCI-H1975 was performed on the right side of the back of mice to form xenograft tumors, the anti-tumor effect of the antibody conjugate was evaluated.

The test drugs and administration regimens are shown in Table 20.

**Table 20. Administration routes, doses and regimens in human lung cancer NCI-H1975 subcutaneous xenograft model**

| Group | Number of animals | Administration group | Dose (mg/kg) | Administration route | Administration cycle |
|---|---|---|---|---|---|
| 1 | 5 | Blank control group | - | i.v. | QW × 4 |
| 2 | 5 | ADC-12 | 7.5 | i.v. | QW × 4 |
| 3 | 5 | ADC-9 | 7.5 | i.v. | QW × 4 |

2. Preparation method: All samples were prepared by dilution with normal saline.
3. Experimental animals: NCI-H1975: 6- to 8-week-old female CB-17 SCID, purchased from Beijing Vital River Laboratory Animal Technology Co., Ltd.
4. Experimental procedures:
   NCI-H1975 (Cell Bank of Chinese Academy of Sciences) cells were cultured in an RPMI1640 medium containing 10% fetal bovine serum. Cells in the exponential phase were harvested and resuspended in DPBS to achieve an appropriate concentration for subcutaneous tumor inoculation in nude mice.

The experimental mice were inoculated subcutaneously on the right side of the back with 5 × 10⁶ NCI-H1975 cells (the cells were resuspended in a 1:1 mixture of DPBS and BD Matrigel (Cat. No. 356234)), with 0.2 mL per mouse. Tumor growth was observed periodically.

When the tumors grew to about 201.31 mm³, the tumor-bearing mice were randomized into groups using StudyDirectorTM. The day of grouping was designated as day 0. The specific administration regimens are shown in Table 20. The experimental endpoint was on day 20 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a* and *b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the two groups of samples was performed using the independent sample T-Test, and the data was analyzed using SPSS. P < 0.05 indicates a significant difference. Plotting was performed using the GraphPad Prism software.

**Table 21. Evaluation of anti-tumor efficacy of test substances in NCI-H1975 cell subcutaneous xenograft tumor model**

| (calculations based on tumor volumes on day 18¹ after administration) | | | | |
|---|---|---|---|---|
| Group | Tumor volume (mm³) | T/C (%)³ | TGI (%) ³ | *p* value⁴ |
| Blank control group | 2044.16+176.43 | - | - | - |
| ADC-12 | 1981.68+132.22 | 97.22 | 3.29 | ns |
| ADC-9 | 72.36+19.18 | 3.51 | 104.42 | *** |

| | | | | |
|---|---|---|---|---|
| 1. Since some mice in the NCI-H1975 blank control group reached euthanasia criteria after 18 days, the statistical analysis data were incomplete, so that statistical analysis was performed on day 18 with the complete number of animals. 2. Tumor volume was expressed as mean + standard error. 3. Tumor growth inhibition is reflected by T/C (T/C (%) = T₁₈/V₁₈ × 100) and TGI (TGI (%) = [1 - (T₁₈ - T₀)/(V₁₈ - Vo)] × 100). 4. *p* values were calculated based on the tumor volumes (* *indicates p < 0.05,* ** *indicates p < 0.01,* *** *indicates p < 0.001, and ns indicates no significant significance).* | | | | |

The experimental results are shown in FIG. 13 and Table 21, indicating that in the administration regimens of the NCI-H1975 model, ADC-9 had a significant anti-tumor effect, while the blank control group and ADC-12 showed no anti-tumor effect.

### Experimental conclusion:

The antibody-drug conjugate of the BsAb targeting EGFR and HER3 of the present disclosure with linker-cytotoxin X1 had a significant inhibitory effect on tumor growth.

### Example 14: Comparison of In Vivo Efficacy of BsAb ADC Disclosed Herein and Control ADC in EGFR ADC-Resistant Mouse Model

In order to compare the *in vivo* efficacy of a DBXT005-01 ADC (ADC-13) and a control ADC (BL-B01D1, ADC-14) in an EGFR ADC-resistant model, after subcutaneous ectopic inoculation of OE-19 was performed in mice to form xenograft tumors, the anti-tumor effects of the two ADCs were compared.
1. The test drugs and administration regimens are shown in Table 22.

**Table 22. Administration routes, doses and regimens in human esophageal cancer OE-19 subcutaneous xenograft model**

| Group | Number of animals | Administration group | Dose (mg/kg) | Administration route | Administration cycle |
|---|---|---|---|---|---|
| 1 | 5 | Blank control group | - | Intravenous injection | Single injection |
| 2 | 5 | ADC-13 | 5 | Intravenous injection | Single injection |
| 3 | 5 | ADC-14 | 8 | Intravenous injection | Single injection |

2. Preparation method: All samples were prepared by dilution with normal saline.

3. Experimental animals: 6- to 7-week-old female BALB/c Nude mice, purchased from Jiangsu GemPharmatech Co., Ltd.

### 4. Experimental procedures:

OE-19 (CL-00806) cells were cultured in an RPMI1640 medium containing 10% fetal bovine serum and 2 mM L-Glutamine. OE-19 cells in the exponential phase were harvested and resuspended in PBS to achieve an appropriate concentration for subcutaneous tumor inoculation in mice.

The experimental mice were inoculated subcutaneously on the right side of the back with 5 × 10⁶ OE-19 cells (the cells were resuspended in a 1:1 mixture of PBS and Matrigel), with 0.1 mL per mouse. Tumor growth was observed periodically.

When the tumors grew to about 151 mm³, the tumor-bearing mice were randomized into groups using StudyDirectorTM. Intravenous (i.v.) injection of the test samples was started on the day of grouping (day 1), and a total of 1 injection was administered. The experimental endpoint was on day 23 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using T/C (%). The percentage value of T/C (%) is an index that reflects the tumor growth inhibition, where T and C represent the mean tumor volume of the administration group and the control group, respectively, on a certain day. The tumor growth inhibition rate was calculated using the following formula: TGI (%) = [1 - (Tᵢ - T₀)/(Vᵢ - V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the sample groups was performed using ONE-WAY ANOVA. P < 0.05 indicates a significant difference. Plotting and statistical analysis were performed using the GraphPad Prism software.

**Table 23. Evaluation of anti-tumor efficacy of test substances in OE-19 cell subcutaneous xenograft tumor model**

| (calculations based on tumor volumes on day 23 after administration) | | | | |
|---|---|---|---|---|
| Group | Tumor volume (mm³) ¹ | T/C (%) ² | TGI (%) ² | *p* value³ |
| Blank control group | 1837.21 + 66.04 | - | - | - |
| ADC-13 | 791.04 + 110.20 | 42.73 | 62.05 | * |
| ADC-14 | 1165.86 + 129.79 | 63.41 | 39.83 | ns |

| | | | | |
|---|---|---|---|---|
| 1. Tumor volume was expressed as mean + standard error. 2. Tumor growth inhibition is reflected by T/C (T/C (%) = T₂₃/V₂₃ × 100) and TGI (TGI (%) = [1 - (T₂₃ - T₀)/(V₂₃ - V₀)] × 100). 3. *p* values were calculated based on the tumor volumes (* *indicates p < 0.05,* ** *indicates p < 0.01,* *** *indicates p < 0.001, and ns indicates no significant significance).* | | | | |

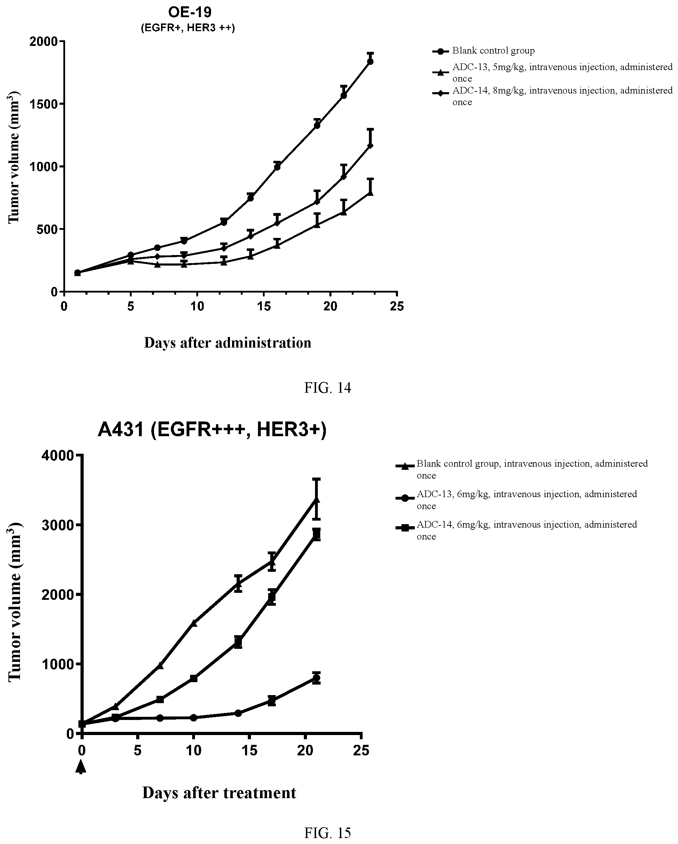

The experimental results are shown in FIG. 14 and Table 23, indicating that in the EGFR ADC-resistant model, the antibody conjugate of the present disclosure had a better anti-tumor effect as compared to the control ADC-14.

### Example 15: Comparison of In Vivo Efficacy of BsAb ADC Disclosed Herein and Control ADC in Mouse Model with High EGFR Expression and Low HER3 Expression

In order to compare the inhibitory effects of a DBXT005-01 ADC (ADC-13) and a control ADC (BL-B01D1, ADC-14) on *in vivo* tumor formation induced by a cell line with high EGFR expression and low HER3 expression, after subcutaneous ectopic inoculation of A431 was performed in mice to form xenograft tumors, the anti-tumor effects of the two ADCs were compared.
1. The test drugs and administration regimens are shown in Table 244.

**Table 24. Administration routes, doses and regimens in human skin cancer A431 subcutaneous xenograft model**

| Group | Number of animals | Administration group | Dose (mg/kg) | Administration route | Administration cycle |
|---|---|---|---|---|---|
| 1 | 5 | Adjuvant solvent group | - | Intravenous injection | Single injection |
| 2 | 5 | ADC-13 | 6 | Intravenous injection | Single injection |
| 3 | 5 | ADC-14 | 6 | Intravenous injection | Single injection |

2. Preparation method: All samples were prepared by dilution with an adjuvant solvent.

3. Experimental animals: 6- to 8-week-old female NCG mice, purchased from Jiangsu GemPharmatech Co., Ltd.

### 4. Experimental procedures:

A431 (CRL-1555) cells were cultured in a DMEM medium containing 10% fetal bovine serum and 2 mM L-Glutamine. A431 cells in the exponential phase were harvested and resuspended in PBS to achieve an appropriate concentration for subcutaneous tumor inoculation in mice.

The experimental mice were inoculated subcutaneously on the right side of the back with 5 × 10⁶ A431 cells. Tumor growth was observed periodically.

When the tumors grew to about 137 mm³, the tumor-bearing mice were randomized into groups. Intravenous (i.v.) injection of the test samples was started on the day of grouping (day 0), and a total of 1 injection was administered. The experimental endpoint was on day 21 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using TGI (%). The tumor growth inhibition rate was calculated using the following formula: TGI (%) = [1 - (Tᵢ/T₀)/(Vᵢ/V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the sample groups was performed using ONE-WAY ANOVA. P < 0.05 indicates a significant difference. Plotting and statistical analysis were performed using the GraphPad Prism software.

**Table 25. Evaluation of anti-tumor efficacy of test substances in A431 cell subcutaneous xenograft tumor model**

| (calculations based on tumor volumes on day 21 after administration) | | | | |
|---|---|---|---|---|
| Group | Tumor volume (mm³) ¹ | TGI (%) ² | *p* value³ | *p* value⁴ |
| Blank control group | 3367.45+287.92 | - | - | - |
| ADC-13 (6 mg/kg) | 799.85+75.63 | 76.28 | ** | *** |
| ADC-14 (6 mg/kg) | 2860.17+76.43 | 15.47 | ns | - |

| | | | | |
|---|---|---|---|---|
| 1. Tumor volume was expressed as mean + standard error. 2. Tumor growth inhibition is reflected by T/C (T/C (%) = T₂₁/V₂₁ × 100) and TGI (TGI (%) = [1 - (T₂₁/T₀)/(V₂₁/V₀)] × 100). 3. *p* values were calculated based on the tumor volumes *(* indicates p < 0.05,* ** *indicates p < 0.01,* *** *indicates p < 0.001, and ns indicates no significant significance).* The tumor volumes in each experimental group and the blank control group were compared. 4. *p* values were calculated based on the tumor volumes *(* indicates p < 0.05,* ** *indicates p < 0.01,* *** *indicates p < 0.001, and ns indicates no significant significance).* The tumor volumes in the experimental groups of the BsAb ADCs and control ADC in this example were compared. | | | | |

The experimental results are shown in FIG. 15 and Table 25, indicating that in the tumor model with high EGFR expression and low HER3 expression, the antibody conjugate of the present disclosure had a better anti-tumor effect as compared to the control ADC at the same dose. The control ADC failed to inhibit tumor growth at this administration concentration. Mice in each administration group showed good tolerance, with no obvious weight loss.

### Example 16: Comparison of Efficacy of BsAb ADC and Control ADC in This Example in Human Colon Cancer Cell Line Xenograft Tumor-Bearing Mouse Model That Does Not Express EGFR but Only Expresses HER3

In order to compare the inhibitory effects of a DBXT005-01 ADC (ADC-13) and a control ADC (BL-B01D1, ADC-14) on *in vivo* tumor formation induced by a human colon cancer cell line that does not express EGFR but only expresses HER3, after subcutaneous ectopic inoculation of SW620 was performed in mice to form xenograft tumors, the anti-tumor effects of the two ADCs were compared.
1. The test drugs and administration regimens are shown in Table 26.

**Table 26. Administration routes, doses and regimens in human colon cancer SW620 subcutaneous xenograft model**

| Group | Number of animals | Administration group | Dose (mg/kg) | Administration route | Administration cycle |
|---|---|---|---|---|---|
| 1 | 5 | Adjuvant solvent group | - | Intravenous injection | Once every three weeks, for a total of two injections |
| 2 | 5 | ADC-13 | 10 | Intravenous injection | Once every three weeks, for a total of two injections |
| 3 | 5 | ADC-14 | 10 | Intravenous injection | Once every three weeks, for a total of two injections |

2. Preparation method: All samples were prepared by dilution with an adjuvant solvent.

3. Experimental animals: 6- to 8-week-old female NCG mice, purchased from Jiangsu GemPharmatech Co., Ltd.

### 4. Experimental procedures:

SW620 (CCL-227) cells were cultured in a DMEM medium containing 10% fetal bovine serum and 2 mM L-Glutamine. SW620 cells in the exponential phase were harvested and resuspended in PBS to achieve an appropriate concentration for subcutaneous tumor inoculation in mice.

The experimental mice were inoculated subcutaneously on the right side of the back with 5 × 10⁶ SW620 cells. Tumor growth was observed periodically.

When the tumors grew to about 131 mm³, the tumor-bearing mice were randomized into groups. Intravenous (i.v.) injection of the test samples was started on the day of grouping (day 0). The experimental endpoint was on day 59 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using TGI (%). The tumor growth inhibition rate was calculated using the following formula: TGI (%) = [1 - (Tᵢ/T₀)/(Vᵢ/V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the sample groups was performed using ONE-WAY ANOVA. P < 0.05 indicates a significant difference. Plotting and statistical analysis were performed using the GraphPad Prism software.

**Table 27. Evaluation of anti-tumor efficacy of test substances in SW620 cell subcutaneous xenograft tumor model**

| Group | Tumor volume on day 31 (mm³) ¹ | TGI (%) ² | *p* value³ | *p* value⁴ | Tumor volume on day 59 (mm³) ¹ | *p* value⁴ |
|---|---|---|---|---|---|---|
| Blank control group | 2619.88+293.60 | - | - | - | N/A⁵ | - |
| ADC-13 (10mg/kg) | 5.01±1.81 | 99.81% | *** | *** | 0.00+0.00 | *** |
| ADC-14 (10mg/kg) | 109.34+19.03 | 95.86% | *** | - | 958.00+79.34 | - |

| | | | | | | |
|---|---|---|---|---|---|---|
| 1. Tumor volume was expressed as mean + standard error. 2. Tumor growth inhibition is reflected by TGI (TGI (%) = [1 - (T₃₁/T₀)/(V₃₁/V₀)] × 100). 3*. p* values were calculated based on the tumor volumes *(* indicates p < 0.05, ** indicates p < 0.01,* *** *indicates p < 0.001, and ns indicates no significant significance).* The tumor volumes in each experimental group and the blank control group were compared. 4. *p* values were calculated based on the tumor volumes *(* indicates p < 0.05,* ** *indicates p < 0.01,* *** *indicates p < 0.001, and ns indicates no significant significance).* The tumor volumes in the experimental groups of the BsAb ADCs and control ADC in this example were compared. 5. N/A indicates that the mice in the control group were euthanized on day 31 according to animal welfare principles. | | | | | | |

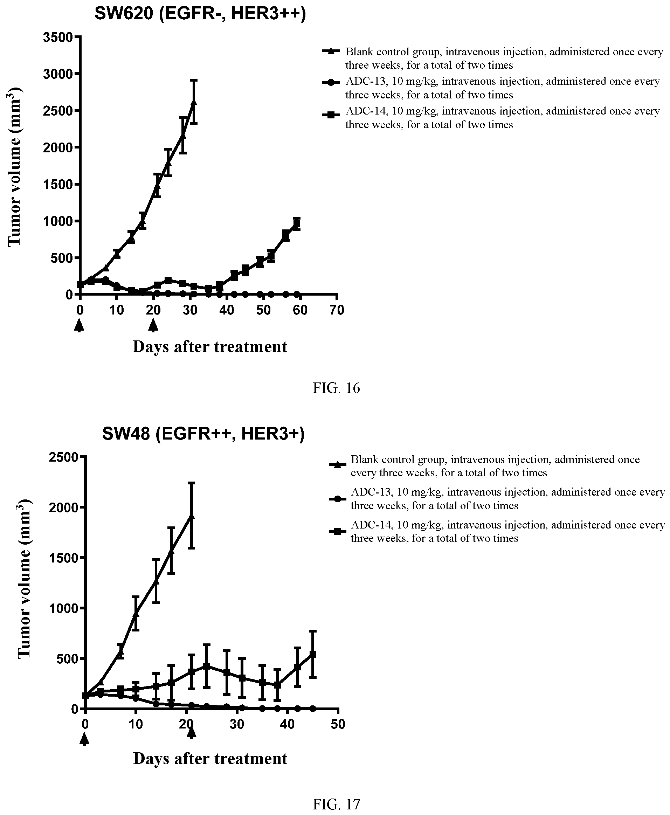

The experimental results are shown in FIG. 16 and Table 27, indicating that in the tumor model with no EGFR expression and medium HER3 expression, all experimental groups had significant inhibitory effects on tumor growth as compared to the blank control group; at the same dose, the antibody conjugate of the present disclosure had a better anti-tumor effect as compared to the control ADC, which could achieve complete tumor regression and sustain tumor growth inhibition until day 59; the control ADC failed to achieve complete tumor regression and failed to inhibit tumor regrowth. Mice in each administration group showed good tolerance, with no obvious weight loss.

### Example 17: Comparison of Efficacy of BsAb ADC Disclosed Herein and Control ADC in Human Colon Cancer Cell Line Xenograft Tumor-Bearing Mouse Model with Medium EGFR Expression and Low HER3 Expression

In order to compare the inhibitory effects of a DBXT005-01 ADC (ADC-13) and a control ADC (BL-B01D1, ADC-14) on *in vivo* tumor formation induced by a human colon cancer cell line with medium EGFR expression and low HER3 expression, after subcutaneous ectopic inoculation of SW48 was performed in mice to form xenograft tumors, the anti-tumor effects of the two ADCs were compared.
1. The test drugs and administration regimens are shown in Table 28.

**Table 28. Administration routes, doses and regimens in human colon cancer SW48 subcutaneous xenograft model**

| Group | Number of animals | Administration group | Dose (mg/kg) | Administration route | Administration cycle |
|---|---|---|---|---|---|
| 1 | 5 | Adjuvant solvent group | - | Intravenous injection | Once every three weeks, for a total of two injections |
| 2 | 5 | ADC-13 | 10 | Intravenous injection | Once every three weeks, for a total of two injections |
| 3 | 5 | ADC-14 | 10 | Intravenous injection | Once every three weeks, for a total of two injections |

2. Preparation method: All samples were prepared by dilution with an adjuvant solvent.

3. Experimental animals: 6- to 8-week-old female NCG mice, purchased from Jiangsu GemPharmatech Co., Ltd.

### 4. Experimental procedures:

SW48 (CCL-231) cells were cultured in a DMEM medium containing 10% fetal bovine serum and 2 mM L-Glutamine. SW48 cells in the exponential phase were harvested and resuspended in PBS to achieve an appropriate concentration for subcutaneous tumor inoculation in mice.

The experimental mice were inoculated subcutaneously on the right side of the back with 1 × 10⁷ SW48 cells. Tumor growth was observed periodically.

When the tumors grew to about 131 mm³, the tumor-bearing mice were randomized into groups. Intravenous (i.v.) injection of the test samples was started on the day of grouping (day 0). The experimental endpoint was on day 45 after grouping. The tumor volume and body weight were measured twice a week, and the data were recorded.

There were 5 mice in each of the control and treatment groups. Tumor inhibition rates were calculated by measuring tumor volumes.

The tumor volume was calculated using the following formula: V = 0.5 *a* × *b²,* where *a and b* represent the long diameter and short diameter of the tumor, respectively. The anti-tumor efficacy of the compounds was evaluated using TGI (%). The tumor growth inhibition rate was calculated using the following formula: TGI (%) = [1 - (Tᵢ/T₀)/(Vᵢ/V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the sample groups was performed using ONE-WAY ANOVA. P < 0.05 indicates a significant difference. Plotting and statistical analysis were performed using the GraphPad Prism software.

**Table 29. Evaluation of anti-tumor efficacy of test substances in SW48 cell subcutaneous xenograft tumor model**

| Group | Tumor volume on day 21 (mm³) ¹ | TGI (%) ² | *p* value³ | *p* value⁴ | Tumor volume on day 45 (mm³) ¹ | *p* value⁴ |
|---|---|---|---|---|---|---|
| Blank control group | 1918.13+324.23 | - | - | - | N/A⁵ | |
| ADC-13 (10 mg/kg) | 35.45±2.67 | 98.14 | *** | *** | 1.44+0.94 | *** |
| ADC-14 (10 mg/kg) | 366.30+167.82 | 81.06 | *** | - | 541.01±229.49 | - |

| | | | | | | |
|---|---|---|---|---|---|---|
| 1. Tumor volume was expressed as mean + standard error. 2. Tumor growth inhibition is reflected by TGI (TGI (%) = [1 - (T₂₁ - T₀)/(V₂₁ - V₀)] × 100). 3*. p* values were calculated based on the tumor volumes *(* indicates p < 0.05, ** indicates p < 0.01,* *** *indicates p < 0.001, and ns indicates no significant significance).* The tumor volumes in each experimental group and the blank control group were compared. 4*. p* values were calculated based on the tumor volumes *(* indicates p < 0.05,* ** *indicates p < 0.01,* *** *indicates p < 0.001, and ns indicates no significant significance).* The tumor volumes in the experimental groups of the BsAb ADCs and control ADC in this example were compared. 5. N/A indicates that the mice in the control group were euthanized on day 21 according to animal welfare principles. | | | | | | |

The experimental results are shown in FIG. 17 and Table 29, indicating that in the tumor model with medium EGFR expression and low HER3 expression, all experimental groups had significant inhibitory effects on tumor growth as compared to the blank control group; at the same dose, the antibody conjugate of the present disclosure had a better anti-tumor effect as compared to the control ADC, which could achieve complete tumor regression in some mice and sustain tumor growth inhibition until day 45; the control ADC failed to achieve complete tumor regression and failed to inhibit tumor recurrence. Mice in each administration group showed good tolerance, with no obvious weight loss.

### Example 18: Comparison of Efficacy of BsAb ADC Disclosed Herein and Control ADC in Human Non-Small Cell Lung Cancer Cell Line Xenograft Tumor-Bearing Mouse Model Resistant to Osimertinib

In order to compare the inhibitory effects of a DBXT005-01 ADC (ADC-13) and a control ADC (BL-B01D1, ADC-14) on in vivo tumor formation induced by an osimertinib-resistant non-small cell lung cancer cell line, after subcutaneous ectopic inoculation of NCI-H1975 (EGFR L858R/T790M/C797S triple mutation) was performed in mice to form xenograft tumors, the anti-tumor effects of the two ADCs were compared.
1. The test drugs and administration regimens are shown in Table 30.

**Table 30. Administration routes, doses and regimens in human non-small cell lung cancer NCI-H1975**

| (EGFR L858R/T790M/C797S triple mutation) subcutaneous xenograft model | | | | | |
|---|---|---|---|---|---|
| Group | Number of animals | Administration group | Dose (mg/kg) | Administration route | Administration cycle |
| 1 | 6 | Adjuvant solvent group | - | Intravenous injection | Once every three weeks, for a total of two injections |
| 2 | 6 | ADC-13 | 6 | Intravenous injection | Once every three weeks, for a total of two injections |
| 3 | 6 | ADC-14 | 6 | Intravenous injection | Once every three weeks, for a total of two injections |

2. Preparation method: All samples were prepared by dilution with an adjuvant solvent.

3. Experimental animals: 6- to 7-week-old female NOD/SCID mice, purchased from Jiangsu GemPharmatech Co., Ltd.

### 4. Experimental procedures:

NCI-H1975 EGFR L858R/T790M/C797S (CL-01195) cells were cultured in an RPMI1640 medium containing 10% fetal bovine serum and 100 µg/mL Hygromycin. NCI-H1975 cells in the exponential phase were harvested and resuspended in PBS to achieve an appropriate concentration for subcutaneous tumor inoculation in NOD/SCID mice. The experimental mice were inoculated subcutaneously on the right side of the back with 1 × 10⁷ NCI-H1975 EGFR L858R/T790M/C797S cells (the cells were resuspended in a 1:1 mixture of PBS and Matrigel), with 0.2 mL per mouse. Tumor growth was observed periodically.

When the tumors grew to about 169.46 mm³, the tumor-bearing mice were randomized into groups. Intravenous (i.v.) injection of the test samples was started on the day of grouping (day 0). The experimental endpoint was on day 35 after grouping. The tumor volume and body weight were measured twice a week, and the data were recorded.

There were 6 mice in each of the control and treatment groups. Tumor inhibition rates were calculated by measuring tumor volumes.

The tumor volume was calculated using the following formula: V = 0.5 *a* × *b²,* where *a* and *b* represent the long diameter and short diameter of the tumor, respectively. The anti-tumor efficacy of the compounds was evaluated using TGI (%). The tumor growth inhibition rate was calculated using the following formula: TGI (%) = [1 - (Tᵢ/T₀)/(Vᵢ/V₀)] × 100, where Tᵢ is the mean tumor volume of a certain administration group on a certain day, T₀ is the mean tumor volume of the administration group at the start of administration, Vᵢ is the mean tumor volume of the vehicle control group on a certain day (same day as Tᵢ), and V₀ is the mean tumor volume of the vehicle control group at the start of administration.

The comparison between the sample groups was performed using ONE-WAY ANOVA. P < 0.05 indicates a significant difference. Plotting and statistical analysis were performed using the GraphPad Prism software.

**Table 31. Evaluation of anti-tumor efficacy of test substances in NCI-H1975 EGFR L858R/T790M/C797S cell subcutaneous xenograft tumor model**

| (calculations based on tumor volumes on day 35 after administration) | | | | |
|---|---|---|---|---|
| Group | Tumor volume (mm³) 1 | TGI (%)² | *p* value³ | *p* value⁴ |
| Blank control group | 1615.42 + 190.27 | - | - | - |
| ADC-13 (6 mg/kg) | 31.02 + 4.45 | 98.09 | *** | *** |
| ADC-14 (6 mg/kg) | 490.04 + 67.43 | 68.37 | ** | - |

| | | | | |
|---|---|---|---|---|
| 1. Tumor volume was expressed as mean + standard error. 2. Tumor growth inhibition is reflected by TGI (TGI (%) = [1 - (T₃₅/T₀)/(V₃₅/V₀)] × 100). 3. *p* values were calculated based on the tumor volumes *(* indicates p < 0.05, ** indicates p < 0.01,* *** *indicates p < 0.001, and ns indicates no significant significance).* The tumor volumes in each experimental group and the blank control group were compared. 4. *p* values were calculated based on the tumor volumes *(* indicates p < 0.05,* ** *indicates p < 0.01,* *** *indicates p < 0.001, and ns indicates no significant significance).* The tumor volumes in the experimental groups of the BsAb ADCs and control ADC in this example were compared. | | | | |

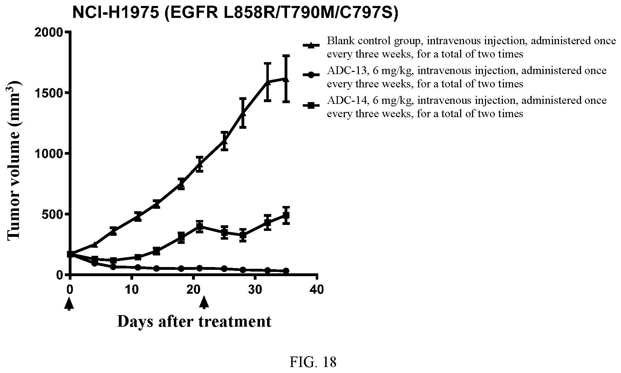

The experimental results are shown in FIG. 18 and Table 31, indicating that in the non-small cell lung cancer tumor model resistant to the third-generation TKI osimertinib, all experimental groups had significant inhibitory effects on tumor growth as compared to the blank control group; at the same dose, the antibody conjugate of the present disclosure had a better anti-tumor effect as compared to the control ADC; mice in each administration group showed good tolerance, with no obvious weight loss.

### Sequence

| SEQ ID NO: | Name | Sequence |
|---|---|---|
| 1 | H1CDR1 | TYGMH |
| 2 | H1CDR2 | VIWDDGSYKYYGDSVKG |
| 3 | H1CDR3 | DGITMVRGVMKDYFDY |
| 4 | L1CDR1 | RASQDISSALV |
| 5 | L1CDR2 | DASSLES |
| 6 | L1CDR3 | QQFNSYPLT |
| 7 | H2CDR1 | SAYYWN |
| 8 | H2CDR2 | YISYDGRNNXNPSLKN, where X is Y or F |
| 9 | H2CDR3 | DGDYDYFDY |
| 10 | L2CDR1 | RASQDISNYLN |
| 11 | L2CDR2 | YTSILHS |
| 12 | L2CDR3 | QQGDTLPPT |
| 13 | H1FR1 | QVQLVESGGGVVQPGRSLRLSCAASGFTFS |
| 14 | H1FR2 | WVRQAPGKGLEWVA |
| 15 | H1FR3 | RFTISRDNSKNTLYLQMNSLRAEDTAVYYCAR |
| 16 | H1FR4 | WGQGTLVTVSS |
| 17 | L1FR1 | AIQLTQSPSSLSASVGDRVTITC |
| 18 | L1FR2/ L2FR2 | WYQQKPGKAPKLLIY |
| 19 | L1FR3 | GVPSRFSGSESGTDFTLTISSLQPEDFATYYC |
| 20 | L1FR4 | FGGGTKVEIK |
| 21 | H2FR1 | QVQLQESGPGLVKPSETLSLTCTVSGYSIT |
| 22 | H2FR2 | WIRQPFGKGLEWMG |
| 23 | H2FR3 | RVTISRDTSKNQFSLKLSSVTAADTAVYYCAR |
| 24 | H2FR4 | WGQGTTVTVSS |
| 25 | L2FR1 | DIQMTQSPSSLSASVGDRVTITC |
| 26 | L2FR3 | GVPSRFSGSGSGTDYTFTISSLQPEDIATYFC |
| 27 | L2FR4 | FGGGTKLEIK |
| 28 | VH1 | |
| 29 | VL1 | |
| 30 | VH2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSS |
| 31 | VL2 | DIQMTQSPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIK |
| 32 | CL1 | |
| 33 | CL2 | SSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKV DKKVEPKSC |
| 34 | C1H1 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSC |
| 35 | C2H1 | ASVAAPSVFIFPPSDEQLKSGTASVVCLLNNFYPREAKVQWKVDNAL |
| | | QSGNSQESVTEQDSKDSTYSLSSTLTLSKADYEKHKVYACEVTHQGL SSPVTKSFNRGEC |
| 36 | Fcl/Fc2 | SVFLFPPKPKDTLMISRTPEVTCVVVDVSHEDPEVKFNWYVDGVEVH NAKTKPREEQYNSTYRVVSVLTVLHQDWLNGKEYKCKVSNKALPAP IEKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| 37 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASTKGPSVFPLAP SSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSRDELTKNQVSLWCL VKGFYPSDIAVEWESNGQPENNYKTTPPVLDSDGSFFLYSKLTVDKSR WQQGNVFSCSVMHEALHNHYTQKSLSLSPGK |
| 38 | Light chain L1 | AIQLTQSPSSLSASVGDRVTITCRASQDISSALVWYQQKPGKAPKLLIY DASSLESGVPSRFSGSESGTDFTLTISSLQPEDFATYYCQQFNSYPLTFG GGTKVEIKRTVAAPSVFIFPPSDEQLKSGTASVVCLLNNFYPREAKVQ WKVDNALQSGNSQESVTEQDSKDSTYSLSSTLTLSKADYEKHKVYA CEVTHQGLSSPVTKSFNRGEC |
| 39 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGEC DKTHTCPPCPAPELLGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSRDELTKNQ VSLSCAVKGFYPSDIAVEWESNGQPENNYKTTPPVLDSDGSFFLVSKL TVDKSRWQQGNVFSCSVMHEALHNHYTQKSLSLSPGK |
| 40 | Light chain L2 | DIQMTQSPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIKSSASTKGPSVFPLAPSSKSTSGGTAALGCLVKDYFPEPVT VSWNSGALTSGVHTFPAVLQSSGLYSLSSVVTVPSSSLGTQTYICNVN HKPSNTKVDKKVEPKSC |
| 41 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASTKGPSVFPLAP SSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPCRDELTKNQVSLWCL VKGFYPSDIAVEWESNGQPENNYKTTPPVLDSDGSFFLYSKLTVDKSR WQQGNVFSCSVMHEALHNHYTQKSLSLSPGK |
| 42 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK |
| | | VSNKALPAPIEKTISKAKGQPREPQVCTLPPSRDELTKNQVSLSCAVKG FYPSDIAVEWESNGQPENNYKTTPPVLDSDGSFFLVSKLTVDKSRWQQ GNVFSCSVMHEALHNHYTQKSLSLSPGK |
| 43 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASTKGPSVFPLAP SSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVYTLPPSRDELTKNQVSLSCAV KGFYPSDIAVEWESNGQPENNYKTTPPVLDSDGSFFLVSKLTVDKSRW QQGNVFSCSVMHEALHNHYTQKSLSLSPGK |
| 44 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 45 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASTKGPSVFPLAP SSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKF NWYVDGVEVHNAKTKPREEQYNSTYRVVSVLTVLHQDWLNGKEYK CKVSNKALPAPIEKTISKAKGQPREPQVCTLPPSRDELTKNQVSLSCAV KGFYPSDIAVEWESNGQPENNYKTTPPVLDSDGSFFLVSKLTVDKSRW QQGNVFSCSVMHEALHNHYTQKSLSLSPGK |
| 46 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 47 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASVAAPSVFIFPPS DEQLKSGTASVVCLLNNFYPREAKVQWKVDNALQSGNSQESVTEQD SKDSTYSLSSTLTLSKADYEKHKVYACEVTHQGLSSPVTKSFNRGECD KTHTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSRDELTKNQV SLWCLVKGFYPSDIAVEWESNGQPENNYKTTPPVLDSDGSFFLYSKLT VDKSRWQQGNVFSCSVMHEALHNHYTQKSLSLSPGK |
| 48 | Light chain L1 | AIQLTQSPSSLSASVGDRVTITCRASQDISSALVWYQQKPGKAPKLLIY |
| | | DASSLESGVPSRFSGSESGTDFTLTISSLQPEDFATYYCQQFNSYPLTFG GGTKVEIKSSASTKGPSVFPLAPSSKSTSGGTAALGCLVKDYFPEPVT VSWNSGALTSGVHTFPAVLQSSGLYSLSSVVTVPSSSLGTQTYICNVN HKPSNTKVDKKVEPKSC |
| 49 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 50 | Light chain L2 | DIQMTQSPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGEC |
| 51 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASVAAPSVFIFPPS DEQLKSGTASVVCLLNNFYPREAKVQWKVDNALQSGNSQESVTEQD SKDSTYSLSSTLTLSKADYEKHKVYACEVTHQGLSSPVTKSFNRGECD KTHTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPCRDELTKNQV SLWCLVKGFYPSDIAVEWESNGQPENNYKTTPPVLDSDGSFFLYSKLT VDKSRWQQGNVFSCSVMHEALHNHYTQKSLSLSPGK |
| 52 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVCTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 53 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASVAAPSVFIFPPS DEQLKSGTASVVCLLNNFYPREAKVQWKVDNALQSGNSQESVTEQD SKDSTYSLSSTLTLSKADYEKHKVYACEVTHQGLSSPVTKSFNRGECD KTHTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPSRDELTKNQV SLSCAVKGFYPSDIAVEWESNGQPENNYKTTPPVLDSDGSFFLVSKLT VDKSRWQQGNVFSCSVMHEALHNHYTQKSLSLSPGK |
| 54 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG |
| | | GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLWCLVKGFYPSDIA VEWESNGQPENNYKTTPPVLDSDGSFFLYSKLTVDKSRWQQGNVFSC SVMHEALHNHYTQKSLSLSPGK |
| 55 | Heavy chain H1 | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSSASVAAPSVFIFPPS DEQLKSGTASVVCLLNNFYPREAKVQWKVDNALQSGNSQESVTEQD SKDSTYSLSSTLTLSKADYEKHKVYACEVTHQGLSSPVTKSFNRGECD KTHTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVCTLPPSRDELTKNQV SLSCAVKGFYPSDIAVEWESNGQPENNYKTTPPVLDSDGSFFLVSKLT VDKSRWQQGNVFSCSVMHEALHNHYTQKSLSLSPGK |
| 56 | Heavy chain H2 | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCRDELTKNQVSLWCLVKGFYPSDI AVEWESNGQPENNYKTTPPVLDSDGSFFLYSKLTVDKSRWQQGNVFS CSVMHEALHNHYTQKSLSLSPGK |
| 57 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLSCAVK GFYPSDIAVEWESNGQPENNYKTTPPVLDSDGSFFLVSKLTVDKSRWQ QGNVFSCSVMHEALHNHYTQKSLSLSPGK |
| 58 | Light chain L2 | DIQMTQSPDSLSASLGDRVTITCRASQDISNYLNWYQQKPGKAPKLLI YYTSILHSGVPSRFSGSGSGTDYTFTISSLQPEDIATYFCQQGDTLPPTF GGGTKLEIKSSASTKGPSVFPLAPSSKSTSGGTAALGCLVKDYFPEPVT VSWNSGALTSGVHTFPAVLQSSGLYSLSSVVTVPSSSLGTQTYICNVN HKPSNTKVDKKVEPKSC |
| 59 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVCTLPPSRDELTKNQVSLSCAVKG FYPSDIAVEWESNGQPENNYKTTPPVLDSDGSFFLVSKLTVDKSRWQQ GNVFSCSVMHEALHNHYTQKSLSLSPGK |
| 60 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS |
| | | STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 61 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 62 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 63 | Light chain L2 | DIQMTQSPDSLSASLGDRVTITCRASQDISNYLNWYQQKPGKAPKLLI YYTSILHSGVPSRFSGSGSGTDYTFTISSLQPEDIATYFCQQGDTLPPTF GGGTKLEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGEC |
| 64 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVCTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 65 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLWCLVKGFYPSDIA VEWESNGQPENNYKTTPPVLDSDGSFFLYSKLTVDKSRWQQGNVFSC SVMHEALHNHYTQKSLSLSPGK |
| 66 | Heavy chain H2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSSASTKGPSVFPLAPSSKSTSGGTA |
| | | ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCRDELTKNQVSLWCLVKGFYPSDI AVEWESNGQPENNYKTTPPVLDSDGSFFLYSKLTVDKSRWQQGNVFS CSVMHEALHNHYTQKSLSLSPGK |
| 67 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLSCAVK GFYPSDIAVEWESNGQPENNYKTTPPVLDSDGSFFLVSKLTVDKSRWQ QGNVFSCSVMHEALHNHYTQKSLSLSPGK |
| 68 | Light chain L2 | DIQMTQEPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIKSSASTKGPSVFPLAPSSKSTSGGTAALGCLVKDYFPEPVT VSWNSGALTSGVHTFPAVLQSSGLYSLSSVVTVPSSSLGTQTYICNVN HKPSNTKVDKKVEPKSC |
| 69 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVCTLPPSRDELTKNQVSLSCAVKG FYPSDIAVEWESNGQPENNYKTTPPVLDSDGSFFLVSKLTVDKSRWQQ GNVFSCSVMHEALHNHYTQKSLSLSPGK |
| 70 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 71 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASVAAPSVFIFPPSDEQLKSGTA SVVCLLNNFYPREAKVQWKVDNALQSGNSQESVTEQDSKDSTYSLS STLTLSKADYEKHKVYACEVTHQGLSSPVTKSFNRGECDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCRDELTKNQVSLWCLVK GFYPSDIAVEWESNGQPENNYKTTPPVLDSDGSFFLYSKLTVDKSRWQ QGNVFSCSVMHEALHNHYTQKSLSLSPGK |
| 72 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY |
| | | YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 73 | Light chain L2 | DIQMTQEPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGEC |
| 74 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVCTLPPSRDELTKNQVSLSCAVKGFYPSDIA VEWESNGQPENNYKTTPPVLDSDGSFFLVSKLTVDKSRWQQGNVFSC SVMHEALHNHYTQKSLSLSPGK |
| 75 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLWCLVKGFYPSDIA VEWESNGQPENNYKTTPPVLDSDGSFFLYSKLTVDKSRWQQGNVFSC SVMHEALHNHYTQKSLSLSPGK |
| 76 | Heavy chain H2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCRDELTKNQVSLWCLVKGFYPSDI AVEWESNGQPENNYKTTPPVLDSDGSFFLYSKLTVDKSRWQQGNVFS CSVMHEALHNHYTQKSLSLSPGK |
| 77 | H2CDR2 | YISYDGRNNYNPSLKN |
| 78 | H2CDR2 | YISYDGRNNFNPSLKN |
| 79 | VH2 | QVQLQESGPGLVKPSDTLSLTCTVSGYSITSAYYWNWIRQPFGKGLE WMGYISYDGRNNFNPSLKNRVTISRDTSKNQFSLKLDSVTAADTAVY YCARDGDYDYFDYWGQGTTVTVSS |
| 80 | VH2 | QVQLQESGPGLVKPSDTLSLTCTVSGYSITSAYYWNWIRQPFGKGLE WMGYISYDGRNNYNPSLKNRVTISRDTSKNQFSLKLDSVTAADTAVY YCARDGDYDYFDYWGQGTTVTVSS |
| 81 | VL2 | DIQMTQSPDSLSASLGDRVTITCRASQDISNYLNWYQQKPGKAPKLLI YYTSILHSGVPSRFSGSGSGTDYTFTISSLQPEDIATYFCQQGDTLPPTF |
| | | GGGTKLEIK |
| 82 | VL2 | DIQMTQEPSSLSASVGDRVTITCRASQDISNYLNWYQQKPGKAPKLLI YYTSILHSGVPSRFSGSGSGTDYTFTISSLQPEDIATYFCQQGDTLPPTF GGGTKLEIK |
| 83 | Anti-EGFR H chain | QVQLVESGGGVVQPGRSLRLSCAASGFTFSTYGMHWVRQAPGKGLE WVAVIWDDGSYKYYGDSVKGRFTISRDNSKNTLYLQMNSLRAEDTA VYYCARDGITMVRGVMKDYFDYWGQGTLVTVSS ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSR EEMTKNQVSLTCLVKGFYPSDIAVEWESNGQPENNYKTTPPVLDSDG SFFLYSKLTVDKSRWQQGNVFSCSVMHEALHNHYTQKSLSLSPGK |
| 84 | Anti-EGFR L chain | AIQLTQSPSSLSASVGDRVTITCRASQDISSALVWYQQKPGKAPKLLIY DASSLESGVPSRFSGSESGTDFTLTISSLQPEDFATYYCQQFNSYPLTFG GGTKVEIK RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 85 | Anti-HER3 H chain | QVQLQESGPGLVKPSETLSLTCTVSGYSITSAYYWNWIRQPFGKGLE WMGYISYDGRNNFNPSLKNRVTISRDTSKNQFSLKLSSVTAADTAVY YCARDGDYDYFDYWGQGTTVTVSSASTKGPSVFPLAPSSKSTSGGTA ALGCLVKDYFPEPVTVSWNSGALTSGVHTFPAVLQSS--- GLYSLSSVVTVPSSS- LGTQTYICNVNHKPSNTKVDKKVEPKSCDKTHTCPPCPAPELLGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPIE KTISKAKGQPREPQVYTLPPSRDELTKNQVSLTCLVKGFYPSDIAVEW ESNGQPENNYKTTPPVLDSDGSFFLYSKLTVDKSRWQQGNVFSCSVM HEALHNHYTQKSLSLSPGK |
| 86 | Anti-HER3 L chain | DIQMTQSPSSLSASVGDRVTITCRASQDISNYLNWYQQKPGKAPKLLI YYTSILHSGVPSRFSGSGSGTDYTFTISSLQPEDIAT--- YFCQQGDTLPPTFGGGTKLEIKRTVAAPSVFIFPPSDEQLKSGTASVVC LLNNFYPREAKVQWKVDNALQSGNSQESVTEQDSKDSTYSLSSTLTL SKADYEKHKVYACEVTHQGLSSPVTKSFNRGEC |
| 87 | Cetuximab heavy chain | QVQLKQSGPGLVQPSQSLSITCTVSGFSLTNYGVHWVRQSPGKGLEW LGVIWSGGNTDYNTPFTSRLSINKDNSKSQVFFKMNSLQSNDTAIYYC ARALTYYDYEFAYWGQGTLVTVSAASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK |
| 88 | Cetuximab light chain | DILLTQSPVILSVSPGERVSFSCRASQSIGTNIHWYQQRTNGSPRLLIKY ASESISGIPSRFSGSGSGTDFTLSINSVESEDIADYYCQQNNNWPTTFG AGTKLELKRTVAAPSVFIFPPSDEQLKSGTASVVCLLNNFYPREAKVQ WKVDNALQSGNSQESVTEQDSKDSTYSLSSTLTLSKADYEKHKVYA CEVTHQGLSSPVTKSFNRGEC |
| 89 | Hinge region | DKTHTCPPCPAPELLGGP |
| 90 | Heavy chain H2 | DIQMTQSPSSLSASVGDRVTITCRASQDISNYLNWYQQKPGKAPKLLI |
| | | YYTSILHSGVPSRFSGSGSGTDYTFTISSLQPEDIATYFCQQGDTLPPTF GGGTKLEIKGGGGSGGGGSGGGGSGGGGSQVQLQESGPGLVKPSETL SLTCTVSGYSITSAYYWNWIRQPFGKGLEWMGYISYDGRNNFNPSLK NRVTISRDTSKNQFSLKLSSVTAADTAVYYCARDGDYDYFDYWGQG TTVTVSSEPKSSDKTHTCPPCPAPELLGGPSVFLFPPKPKDTLMISRTPE VTCVVVDVSHEDPEVKFNWYVDGVEVHNAKTKPREEQYNSTYRVV SVLTVLHQDWLNGKEYKCKVSNKALPAPIEKTISKAKGQPREPQVYT LPPSRDELTKNQVSLSCAVKGFYPSDIAVEWESNGQPENNYKTTPPVL DSDGSFFLVSKLTVDKSRWQQGNVFSCSVMHEALHNHYTQKSLSLSP GK |
| 91 | SI-1X6.4 heavy chain | QVQLKQSGPGLVQPSQSLSITCTVSGFSLTNYGVHWVRQSPGKGLEW LGVIWSGGNTDYNTPFTSRLSINKDNSKSQVFFKMNSLQSNDTAIYYC ARALTYYDYEFAYWGQGTLVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALPA PIEKTISKAKGQPREPQVYTLPPSRDELTKNQVSLTCLVKGFYPSDIAV EWESNGQPENNYKTTPPVLDSDGSFFLYSKLTVDKSRWQQGNVFSCS VMHEALHNHYTQKSLSLSPGGGGGSGGGGSQVQLQESGGGLVKPGG SLRLSCAASGFTFSSYWMSWVRQAPGKGLEWVANINRDGSASYYVD SVKGRFTISRDDAKNSLYLQMNSLRAEDTAVYYCARDRGVGYFDLW GRGTLVTVSSGGGGSGGGGSGGGGSQSALTQPASVSGSPGQSITISCT GTSSDVGGYNFVSWYQQHPGKAPKLMIYDVSDRPSGVSDRFSGSKS GNTASLIISGLQADDEADYYCSSYGSSSTHVIFGGGTKVTVL |
| 92 | SI-1X6.4 light chain | DILLTQSPVILSVSPGERVSFSCRASQSIGTNIHWYQQRTNGSPRLLIKY ASESISGIPSRFSGSGSGTDFTLSINSVESEDIADYYCQQNNNWPTTFG AGTKLELKRTVAAPSVFIFPPSDEQLKSGTASVVCLLNNFYPREAKVQ WKVDNALQSGNSQESVTEQDSKDSTYSLSSTLTLSKADYEKHKVYA CEVTHQGLSSPVTKSFNRGEC |
| 93 | Fc1 | SVFLFPPKPKDTLMISRTPEVTCVVVDVSHEDPEVKFNWYVDGVEVH NAKTKPREEQYNSTYRVVSVLTVLHQDWLNGKEYKCKVSNKALPAP IEKTISKAKGQPREPQVYTLPPSRDELTKNQVSLWCLVKGFYPSDIAVE WESNGQPENNYKTTPPVLDSDGSFFLYSKLTVDKSRWQQGNVFSCSV MHEALHNHYTQKSLSLSPGK |
| 94 | Fc2 | SVFLFPPKPKDTLMISRTPEVTCVVVDVSHEDPEVKFNWYVDGVEVH NAKTKPREEQYNSTYRVVSVLTVLHQDWLNGKEYKCKVSNKALPAP IEKTISKAKGQPREPQVYTLPPSRDELTKNQVSLSCAVKGFYPSDIAVE WESNGQPENNYKTTPPVLDSDGSFFLVSKLTVDKSRWQQGNVFSCSV MHEALHNHYTQKSLSLSPGK |
| 95 | Hinge region | GGGGSGGGGSGGGGSGGGGS |
| 96 | Hinge region | EPKSSDKTHTCPPCPAPELLGGP |

Although specific embodiments of the present disclosure have been described above, it should be understood by those skilled in the art that these embodiments are merely illustrative and that many changes or modifications can be made to these embodiments without departing from the principles and spirit of the present disclosure. The protection scope of the present disclosure is therefore defined by the appended claims.

## Claims

1. A bispecific antibody comprising an EGFR-binding domain and an HER3-binding domain, the EGFR-binding domain comprising a heavy chain variable region VH1 and a light chain variable region VL1, and the HER3-binding domain comprising a heavy chain variable region VH2 and a light chain variable region VL2, wherein the VH1 comprises H1CDR1, H1CDR2, and H1CDR3 with the amino acid sequences set forth in SEQ ID NO: 1, SEQ ID NO: 2, and SEQ ID NO: 3, respectively; the VL1 comprises L1CDR1, L1CDR2, and L1CDR3 with the amino acid sequences set forth in SEQ ID NO: 4, SEQ ID NO: 5, and SEQ ID NO: 6, respectively; the VH2 comprises H2CDR1, H2CDR2, and H2CDR3 with the amino acid sequences set forth in SEQ ID NO: 7, SEQ ID NO: 8, and SEQ ID NO: 9, respectively; and the VL2 comprises L2CDR1, L2CDR2, and L2CDR3 with the amino acid sequences set forth in SEQ ID NO: 10, SEQ ID NO: 11, and SEQ ID NO: 12, respectively.

2. The bispecific antibody according to claim 1, wherein the amino acid sequence of the H2CDR2 is set forth in SEQ ID NO: 77 or 78;
preferably, the VH1 comprises framework regions H1FR1, H1FR2, H1FR3, and H1FR4 with amino acid sequences set forth in or having at least 90%, 91%, 92%, 93%, 94%, 95%, 96%, 97%, 98%, or 99% identity to SEQ ID NO: 13, SEQ ID NO: 14, SEQ ID NO: 15, and SEQ ID NO: 16, respectively;
the VL1 comprises framework regions L1FR1, L1FR2, L1FR3, and H1FR4 with amino acid sequences set forth in or having at least 90%, 91%, 92%, 93%, 94%, 95%, 96%, 97%, 98%, or 99% identity to SEQ ID NO: 17, SEQ ID NO: 18, SEQ ID NO: 19, and SEQ ID NO: 20, respectively;
the VH2 comprises a framework region H2FR1 with an amino acid sequence set forth in or having at least 90%, 91%, 92%, 93%, 94%, 95%, 96%, 97%, 98%, or 99% identity to SEQ ID NO: 21, for example, having an E16D mutation on SEQ ID NO: 21; framework regions H2FR2 and H2FR4 with amino acid sequences set forth in or having at least 90%, 91%, 92%, 93%, 94%, 95%, 96%, 97%, 98%, or 99% identity to SEQ ID NO: 22 and SEQ ID NO: 24, respectively; and a framework region H2FR3 with an amino acid sequence set forth in or having at least 90%, 91%, 92%, 93%, 94%, 95%, 96%, 97%, 98%, or 99% identity to SEQ ID NO: 23, for example, having an S18D mutation on SEQ ID NO: 23;
the VL2 comprises a framework region L2FR1 with an amino acid sequence set forth in or having at least 90%, 91%, 92%, 93%, 94%, 95%, 96%, 97%, 98%, or 99% identity to SEQ ID NO: 25, for example, having S9D and V15L mutations, or an S7E mutation, on SEQ ID NO: 25; and framework regions L2FR2, L2FR3, and L2FR4 with amino acid sequences set forth in or having at least 90%, 91%, 92%, 93%, 94%, 95%, 96%, 97%, 98%, or 99% identity to SEQ ID NO: 18, SEQ 'ID NO: 26, and SEQ ID NO: 27, respectively.

3. The bispecific antibody according to claim 1 or 2, wherein an amino acid sequence of the VH1 is set forth in SEQ ID NO: 28, an amino acid sequence of the VL1 is set forth in SEQ ID NO: 29, an amino acid sequence of the VH2 is set forth in SEQ ID NO: 30, SEQ ID NO: 79, or SEQ ID NO: 80, and an amino acid sequence of the VL2 is set forth in SEQ ID NO: 31, SEQ ID NO: 81, or SEQ ID NO: 82;
preferably, the amino acid sequences of the VH1, the VL1, the VH2, and the VL2 of the bispecific antibody are set forth in SEQ ID NO: 28, SEQ ID NO: 29, SEQ ID NO: 30, and SEQ ID NO: 31, respectively, or set forth in SEQ ID NO: 28, SEQ ID NO: 29, SEQ ID NO: 79, and SEQ ID NO: 81, respectively, or set forth in SEQ ID NO: 28, SEQ ID NO: 29, SEQ ID NO: 79, and SEQ ID NO: 31, respectively, or set forth in SEQ ID NO: 28, SEQ ID NO: 29, SEQ ID NO: 80, and SEQ ID NO: 82, respectively.

4. The bispecific antibody according to any one of claims 1-3, wherein the EGFR-binding domain and the HER3-binding domain further each comprise a light chain constant region and a heavy chain constant region, the EGFR-binding domain comprising a light chain constant region CL1 and a heavy chain constant region HCl, and the HER3-binding domain comprising a light chain constant region CL2 and a heavy chain constant region HC2, wherein amino acid sequences of the CL1 and the CL2 are set forth in SEQ ID NO: 32 or SEQ ID NO: 33, respectively, or have at least 90%, 91%, 92%, 93%, 94%, 95%, 96%, 97%, 98%, or 99% identity thereto, and the amino acid sequences of the CL1 and the CL2 are not identical sequences; and/or the HC1 comprises C1H1 and Fc1, and the HC2 comprises C2H1 and Fc2, wherein amino acid sequences of the C1H1 and the C2H1 are set forth in SEQ ID NO: 34 or SEQ ID NO: 35, or have at least 90%, 91%, 92%, 93%, 94%, 95%, 96%, 97%, 98%, or 99% identity thereto, and the amino acid sequences of the C1H1 and the C2H1 are not identical sequences; amino acid sequences of the Fc1 and the Fc2 are variant sequences of the amino acid sequence set forth in SEQ ID NO: 36, or have at least 90%, 91%, 92%, 93%, 94%, 95%, 96%, 97%, 98%, or 99% identity thereto, for example, having a T146W mutation, or S134C and T146W mutations, or T146S, L148A and Y187V mutations, or Y349C, T366S, L368A and Y407V mutations, on SEQ ID NO: 36, and the amino acid sequences of the Fc1 and the Fc2 are not identical sequences;
preferably, the amino acid sequences of the CL1 and the CL2 are set forth in SEQ ID NO: 32 or SEQ ID NO: 33, respectively, the amino acid sequences of the C1H1 and the C2H1 are set forth in SEQ ID NO: 34 or SEQ ID NO: 35, respectively, and the amino acid sequences of the Fc1 and the Fc2 are variant sequences of the amino acid sequence set forth in SEQ ID NO: 36, for example, having a T146W mutation, or S134C and T146W mutations, or T146S, L148A and Y187V mutations, or Y349C, T366S, L368A and Y407V mutations, on SEQ ID NO: 36;
more preferably, the Fc1 and the Fc2 are connected by a disulfide bond in a hinge region and a knob-into-hole structure, wherein the Fc1 is knob-Fc and the Fc2 is hole-Fc, or the Fc2 is knob-Fc and the Fc1 is hole-Fc; further more preferably, the C1H1 and the Fc1, as well as the C2H1 and the Fc2, are connected by a hinge region, wherein an amino acid sequence of the hinge region is set forth in SEQ ID NO: 89.

5. The bispecific antibody according to any one of claims 1-3, wherein the EGFR-binding domain comprises a light chain constant region CL1 and a heavy chain constant region HC1, and the HER3-binding domain comprises a heavy chain constant region HC2, wherein an amino acid sequence of the CL1 is set forth in SEQ ID NO: 32, or has at least 90%, 91%, 92%, 93%, 94%, 95%, 96%, 97%, 98%, or 99% identity thereto; and/or the HC1 comprises C1H1 and Fc1, and the HC2 comprises Fc2, wherein an amino acid sequence of the C1H1 is set forth in SEQ ID NO: 34, or has at least 90%, 91%, 92%, 93%, 94%, 95%, 96%, 97%, 98%, or 99% identity thereto; amino acid sequences of the Fc1 and the Fc2 are variant sequences of the amino acid sequence set forth in SEQ ID NO: 36, or have at least 90%, 91%, 92%, 93%, 94%, 95%, 96%, 97%, 98%, or 99% identity thereto;
preferably, the amino acid sequence of the CL1 is set forth in SEQ ID NO: 32, the amino acid sequence of the C1H1 is set forth in SEQ ID NO: 34, and the amino acid sequences of the Fc1 and the Fc2 are set forth in SEQ ID NOs: 93 and 94, respectively;
more preferably, the Fc1 and the Fc2 are connected by a disulfide bond in a hinge region and a knob-into-hole structure, wherein the Fc1 is knob-Fc and the Fc2 is hole-Fc, or the Fc2 is knob-Fc and the Fc1 is hole-Fc; further more preferably, the C1H1 and the Fc1 are connected by a hinge region with the amino acid sequence set forth in SEQ ID NO: 89; the VL2 and the VH2 are connected by a hinge region with the amino acid sequence set forth in SEQ ID NO: 95; the VH2 and the Fc2 are connected by a hinge region with the amino acid sequence set forth in SEQ ID NO: 96.

6. The bispecific antibody according to any one of claims 1-3 or claim 5, comprising a heavy chain H1, a light chain L1, and a heavy chain H2, wherein an amino acid sequence of the H1 is set forth in SEQ ID NO: 37, or has at least 90%, 91%, 92%, 93%, 94%, 95%, 96%, 97%, 98%, or 99% identity thereto, and/or an amino acid sequence of the L1 is set forth in SEQ ID NO: 38, or has at least 90%, 91%, 92%, 93%, 94%, 95%, 96%, 97%, 98%, or 99% identity thereto, and/or an amino acid sequence of the H2 is set forth in SEQ ID NO: 90, or has at least 90%, 91%, 92%, 93%, 94%, 95%, 96%, 97%, 98%, or 99% identity thereto;
preferably, the amino acid sequences of the heavy chain H1, the light chain L1, and the heavy chain H2 are set forth in SEQ ID NO: 37, SEQ ID NO: 38, and SEQ ID NO: 90, respectively.

7. The bispecific antibody according to any one of claims 1-4, comprising a heavy chain H1, a light chain L1, a heavy chain H2, and a light chain L2 with the amino acid sequences set forth in SEQ ID NO: 37, SEQ ID NO: 38, SEQ ID NO: 39, and SEQ ID NO: 40, respectively; or
set forth in SEQ ID NO: 41, SEQ ID NO: 38, SEQ ID NO: 42, and SEQ ID NO: 40, respectively; or
set forth in SEQ ID NO: 43, SEQ ID NO: 38, SEQ ID NO: 44, and SEQ ID NO: 40, respectively; or
set forth in SEQ ID NO: 45, SEQ ID NO: 38, SEQ ID NO: 46, and SEQ ID NO: 40, respectively; or
set forth in SEQ ID NO: 47, SEQ ID NO: 48, SEQ ID NO: 49, and SEQ ID NO: 50, respectively; or
set forth in SEQ ID NO: 51, SEQ ID NO: 48, SEQ ID NO: 52, and SEQ ID NO: 50, respectively; or
set forth in SEQ ID NO: 53, SEQ ID NO: 48, SEQ ID NO: 54, and SEQ ID NO: 50, respectively; or
set forth in SEQ ID NO: 55, SEQ ID NO: 48, SEQ ID NO: 56, and SEQ ID NO: 50, respectively; or
set forth in SEQ ID NO: 37, SEQ ID NO: 38, SEQ ID NO: 57, and SEQ ID NO: 58, respectively; or
set forth in SEQ ID NO: 41, SEQ ID NO: 38, SEQ ID NO: 59, and SEQ ID NO: 58, respectively; or
set forth in SEQ ID NO: 43, SEQ ID NO: 38, SEQ ID NO: 60, and SEQ ID NO: 58, respectively; or
set forth in SEQ ID NO: 45, SEQ ID NO: 38, SEQ ID NO: 61, and SEQ ID NO: 58, respectively; or
set forth in SEQ ID NO: 47, SEQ ID NO: 48, SEQ ID NO: 62, and SEQ ID NO: 63, respectively; or
set forth in SEQ ID NO: 51, SEQ ID NO: 48, SEQ ID NO: 64, and SEQ ID NO: 63, respectively; or
set forth in SEQ ID NO: 53, SEQ ID NO: 48, SEQ ID NO: 65, and SEQ ID NO: 63, respectively; or
set forth in SEQ ID NO: 55, SEQ ID NO: 48, SEQ ID NO: 66, and SEQ ID NO: 63, respectively; or
set forth in SEQ ID NO: 37, SEQ ID NO: 38, SEQ ID NO: 57, and SEQ ID NO: 40, respectively; or
set forth in SEQ ID NO: 41, SEQ ID NO: 38, SEQ ID NO: 59, and SEQ ID NO: 40, respectively; or
set forth in SEQ ID NO: 43, SEQ ID NO: 38, SEQ ID NO: 60, and SEQ ID NO: 40, respectively; or
set forth in SEQ ID NO: 45, SEQ ID NO: 38, SEQ ID NO: 61, and SEQ ID NO: 40, respectively; or
set forth in SEQ ID NO: 47, SEQ ID NO: 48, SEQ ID NO: 62, and SEQ ID NO: 50, respectively; or
set forth in SEQ ID NO: 51, SEQ ID NO: 48, SEQ ID NO: 64, and SEQ ID NO: 50, respectively; or
set forth in SEQ ID NO: 53, SEQ ID NO: 48, SEQ ID NO: 65, and SEQ ID NO: 50, respectively; or
set forth in SEQ ID NO: 55, SEQ ID NO: 48, SEQ ID NO: 66, and SEQ ID NO: 50, respectively; or
set forth in SEQ ID NO: 37, SEQ ID NO: 38, SEQ ID NO: 67, and SEQ ID NO: 68, respectively; or
set forth in SEQ ID NO: 41, SEQ ID NO: 38, SEQ ID NO: 69, and SEQ ID NO: 68, respectively; or
set forth in SEQ ID NO: 43, SEQ ID NO: 38, SEQ ID NO: 70, and SEQ ID NO: 68, respectively; or
set forth in SEQ ID NO: 45, SEQ ID NO: 38, SEQ ID NO: 71, and SEQ ID NO: 68, respectively; or
set forth in SEQ ID NO: 47, SEQ ID NO: 48, SEQ ID NO: 72, and SEQ ID NO: 73, respectively; or
set forth in SEQ ID NO: 51, SEQ ID NO: 48, SEQ ID NO: 74, and SEQ ID NO: 73, respectively; or
set forth in SEQ ID NO: 53, SEQ ID NO: 48, SEQ ID NO: 75, and SEQ ID NO: 73, respectively; or
set forth in SEQ ID NO: 55, SEQ ID NO: 48, SEQ ID NO: 76, and SEQ ID NO: 73, respectively.

8. An isolated nucleic acid, encoding the bispecific antibody according to any one of claims 1-7.

9. A recombinant expression vector, comprising the nucleic acid according to claim 8.

10. A transformant, comprising the nucleic acid according to claim 8 or the recombinant expression vector according to claim 9 in a host cell, wherein
preferably, the host cell is a eukaryotic cell, preferably a mammalian cell, such as a CHO cell.

11. A method for preparing the bispecific antibody according to any one of claims 1-7, comprising culturing the transformant according to claim 10 to obtain the bispecific antibody.

12. A bispecific antibody-drug conjugate, or a tautomer, an enantiomer, a diastereoisomer or a mixture of isomers thereof, or a pharmaceutically acceptable salt thereof, the bispecific antibody-drug conjugate comprising the following fragments: the bispecific antibody according to any one of claims 1-7 or an antigenbinding fragment thereof, a linker unit L, and a cytotoxic drug.

13. The bispecific antibody-drug conjugate, or the tautomer, the enantiomer, the diastereoisomer or the mixture of isomers thereof, or the pharmaceutically acceptable salt thereof according to claim 12, wherein the cytotoxic drug is camptothecin and a derivative thereof;
preferably, the cytotoxic drug is of a structure of formula (A-1), or a tautomer, an enantiomer or a diastereoisomer thereof: 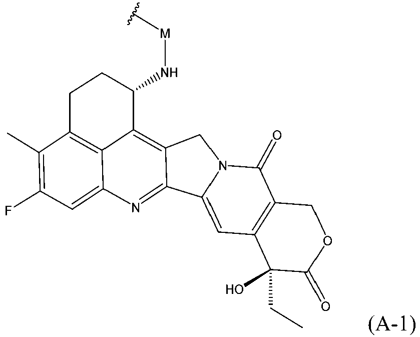
wherein
M is -L²-L¹-C(O)-;
L² is -NH-, O, or S, preferably -O- or -S-, and more preferably -O-, and L² is linked to the linker unit L;
L¹ is -(C(R^{1a})(R^{1b}))ₘ-CH₂-, saturated C₃-C₆ cycloalkylene, or saturated 3- to 6-membered heterocyclylene, wherein the saturated C₃-C₆ cycloalkylene and the saturated 3- to 6-membered heterocyclylene are each independently optionally substituted with one or more R^{2a};
m is selected from 1, 2, 3, and 4; each heteroatom in the saturated 3- to 6-membered heterocyclylene is independently N, O, or S, and the number of the heteroatom is 1, 2, or 3;
each R^{1a} and each R^{1b} are independently selected from hydrogen, halogen, hydroxyl, amino, and C₁-C₆ alkyl, the C₁-C₆ alkyl being optionally substituted with one or more halogens;
R^{2a} is selected from halogen, hydroxyl, amino, and C₁-C₆ alkyl, the C₁-C₆ alkyl being optionally substituted with one or more halogens.

14. The bispecific antibody-drug conjugate, or the tautomer, the enantiomer, the diastereoisomer or the mixture of isomers thereof, or the pharmaceutically acceptable salt thereof according to claim 13, wherein L¹ is - (C(R^{1a})(R^{1b}))ₘ-CH₂-; each R^{1a} is selected from: hydrogen, halogen, and C₁-C₆ alkyl; each R^{1b} is selected from: hydrogen, halogen, and C₁-C₆ alkyl; or
L¹ is -(C(R^{1a})(R^{1b}))ₘ-CH₂-, wherein R^{1a} is C₁-C₆ alkyl, preferably C₁-C₃ alkyl, and R^{1b} is selected from: hydrogen and C₁-C₆ alkyl, preferably selected from: hydrogen and C₁-C₃ alkyl; or L¹ is -(C(R^{1a})(R^{1b}))ₘ-CH₂-, wherein R^{1a} is -CH₃, and R^{1b} is selected from: hydrogen and -CH₃; or
L¹ is saturated C₃-C₆ cycloalkylene or saturated 3- to 6-membered heterocyclylene, preferably saturated C₃-C₆ cycloalkylene, wherein the saturated C₃-C₆ cycloalkylene and the saturated 3- to 6-membered heterocyclylene are each independently optionally substituted with one or more R^{2a}, each R^{2a} being independently halogen or C₁-C₆ alkyl; or
L¹ is cyclopropyl, cyclobutyl, cyclopentyl, or cyclohexyl, optionally substituted with one or more R^{2a}, each R^{2a} being independently selected from: halogen and C₁-C₆ alkyl; or
L¹ is 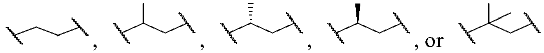 ; or
L¹ is

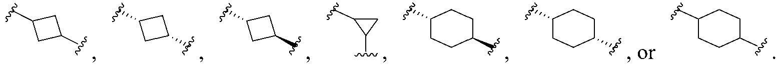

15. The bispecific antibody-drug conjugate, or the tautomer, the enantiomer, the diastereoisomer or the mixture of isomers thereof, or the pharmaceutically acceptable salt thereof according to claim 14, wherein in the structure of formula (A-1),
M is -L²-L¹-C(O)-;
L² is -O-;
L¹ is -(C(R^{1a})(R^{1b}))ₘ-CH₂- or saturated C₃-C₆ cycloalkylene, wherein the saturated C₃-C₆ cycloalkylene is optionally substituted with one or more R^{2a};
m is selected from 1 or 2;
R^{1a} and R^{1b} are each independently selected from hydrogen, halogen, and C₁-C₆ alkyl, the C₁-C₆ alkyl being optionally substituted with one or more halogens;
R^{2a} is selected from halogen and C₁-C₆ alkyl, the C₁-C₆ alkyl being optionally substituted with one or more halogens.

16. The bispecific antibody-drug conjugate, or the tautomer, the enantiomer, the diastereoisomer or the mixture of isomers thereof, or the pharmaceutically acceptable salt thereof according to any one of claims 13-15, wherein M is: 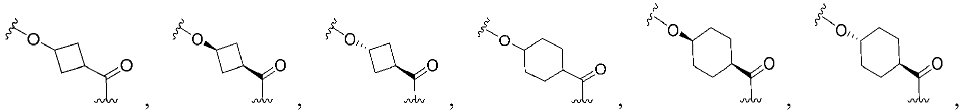 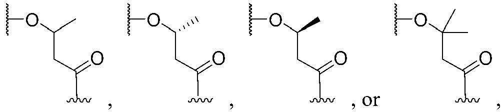 preferably

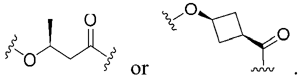

17. The bispecific antibody-drug conjugate, or the tautomer, the enantiomer, the diastereoisomer or the mixture of isomers thereof, or the pharmaceutically acceptable salt thereof according to any one of claims 12-16, wherein the cytotoxic drug is selected from any one of the following structures:

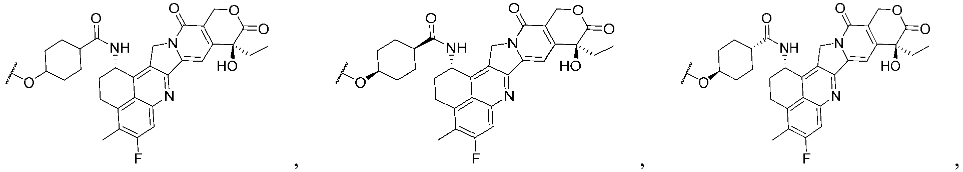

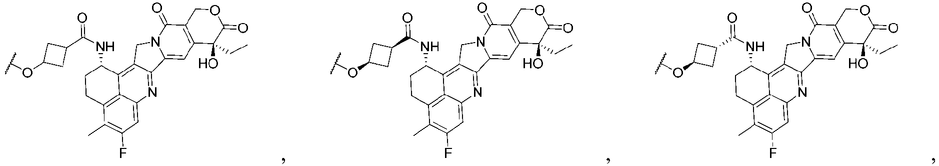

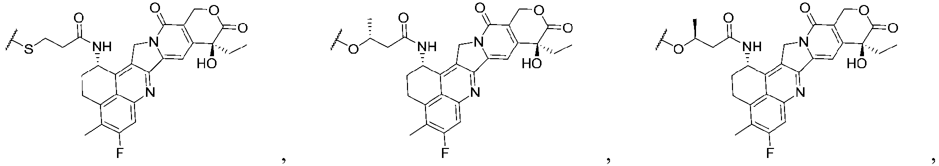

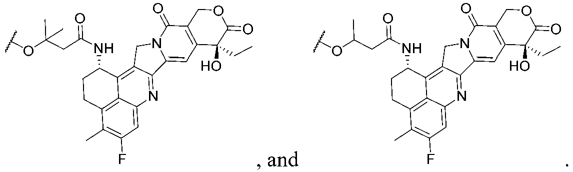

18. The bispecific antibody-drug conjugate, or the tautomer, the enantiomer, the diastereoisomer or the mixture of isomers thereof, or the pharmaceutically acceptable salt thereof according to any one of claims 12-17, wherein the linker unit L is -Lₐ-L_{b}-L_{c}-, wherein L_{c} is linked to the cytotoxic drug;
- Lₐ- is 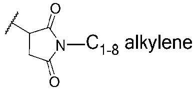 or -C₁₋₈ alkylene-C(O)-, preferably 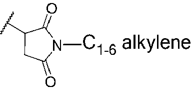 or -C₁₋₆ alkylene-C(O)-, more preferably 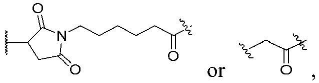 and further preferably 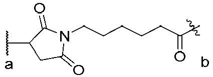 or 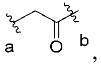 wherein the a end is connected to Ab, and the b end is connected to L_{b};
- L_{b}- is -(polypeptide of 1 to 6 natural amino acids)-NH-, preferably -(polypeptide of 2 to 4 natural amino acids)-NH-, more preferably selected from any one of the following structures: 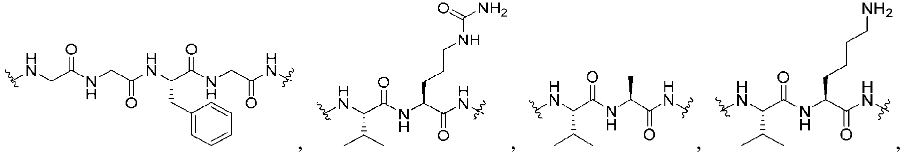 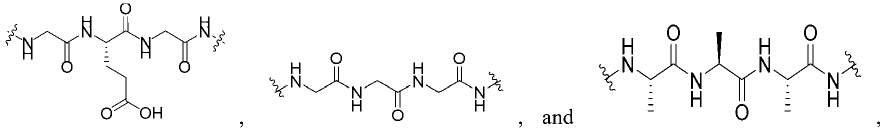 further preferably 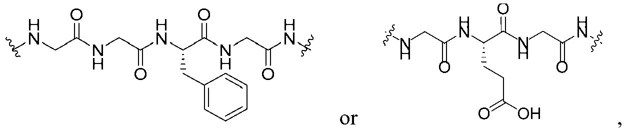 and more preferably 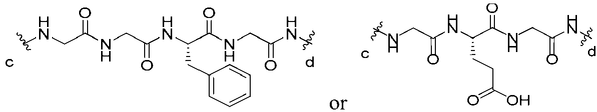 , wherein the c end is connected to Lₐ, and the d end is connected to L_{c};
- L_{c}- is C₁₋₆ alkylene, preferably C₁₋₃ alkylene, and more preferably

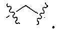

19. The bispecific antibody-drug conjugate, or the tautomer, the enantiomer, the diastereoisomer or the mixture of isomers thereof, or the pharmaceutically acceptable salt thereof according to claim 18, wherein the linker unit L is 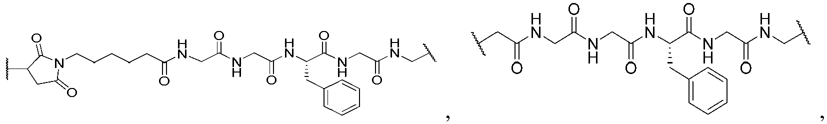 or 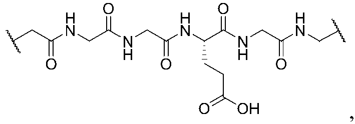 preferably

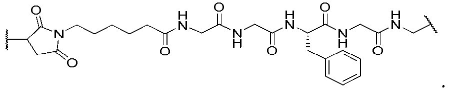

20. The bispecific antibody-drug conjugate, or the tautomer, the enantiomer, the diastereoisomer or the mixture of isomers thereof, or the pharmaceutically acceptable salt thereof according to any one of claims 12-19, wherein the bispecific antibody-drug conjugate has a structure of formula (A-2): 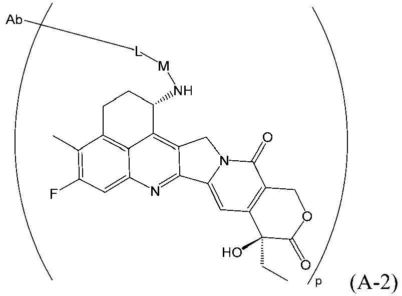
wherein p represents an average connection number, and p is any one of integers or decimals from 1 to 10,
preferably any one of integers or decimals from 3 to 9, such as 4, 4.06, 4.10, 6, 6.11, 6.05, 7.99, 7.98, or 8;
Ab is the bispecific antibody according to any one of claims 1-7 or an antigen-binding fragment thereof;
M is as defined in the antibody-drug conjugate according to any one of claims 13-17;
L is the linker unit L according to claim 18 or 19.

21. The bispecific antibody-drug conjugate, or the tautomer, the enantiomer, the diastereoisomer or the mixture of isomers thereof, or the pharmaceutically acceptable salt thereof according to any one of claims 12-20, wherein the bispecific antibody-drug conjugate has a structure of formula (A-2a) or (A-2b): 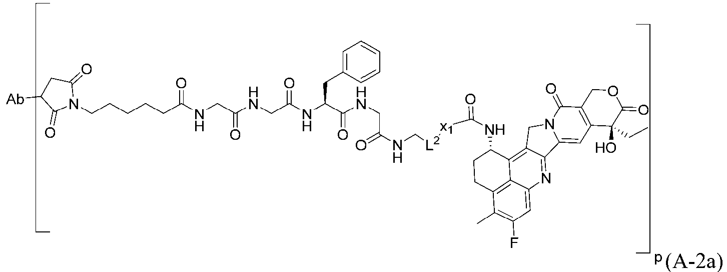 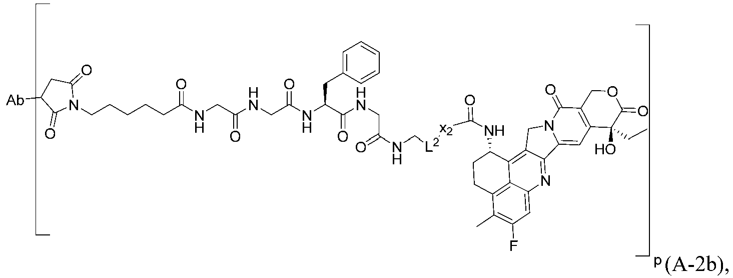 wherein
p represents an average connection number, and p is selected from any one of integers or decimals from 1 to 10, preferably any one of integers or decimals from 3 to 9, such as 4, 4.06, 4.10, 6, 6.11, 6.05, 7.99, 7.98, or 8;
Ab is the bispecific antibody according to any one of claims 1-7 or an antigen-binding fragment thereof;
L₂ is -NH-, O, or S, preferably -O- or -S-, and more preferably -O-;
X₁ is selected from saturated C₃-C₆ cycloalkylene optionally substituted with 1, 2, or 3 R^{2a};
X₂ is selected from -(C(R^{1a})(R^{1b}))ₘ-CH₂-;
m is selected from 1 or 2;
R^{1a} and R^{1b} are each independently hydrogen, halogen, or C₁-C₆ alkyl optionally substituted with 1, 2, or 3 halogens;
R^{2a} is selected from halogen, hydroxyl, amino, and C₁-C₆ alkyl, the C₁-C₆ alkyl being optionally substituted with one or more halogens;
preferably, the structure of the bispecific antibody-drug conjugate is selected from the following: 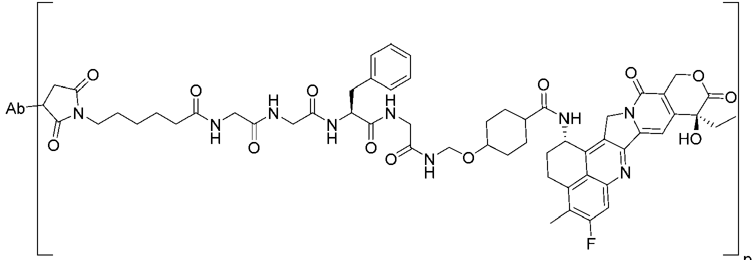 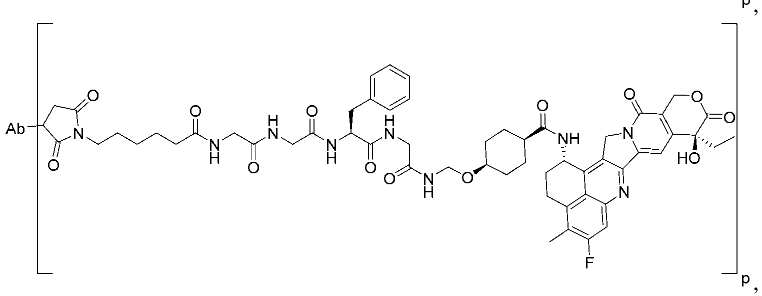 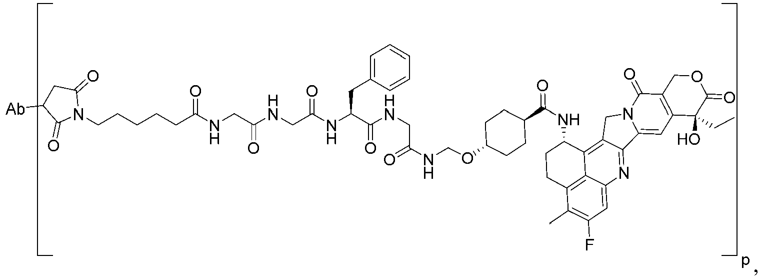 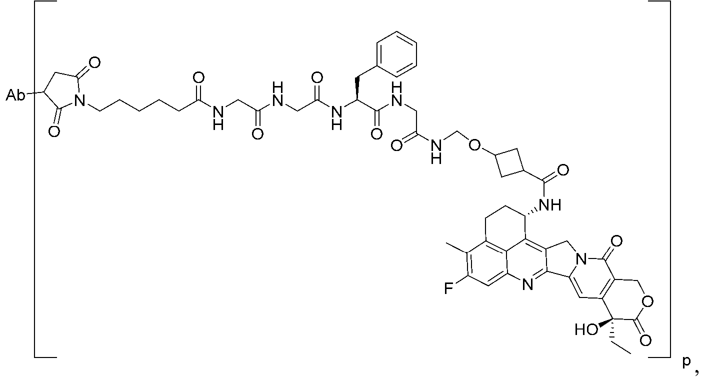 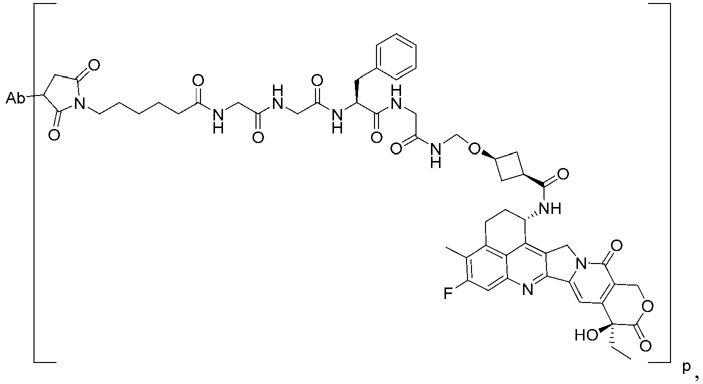 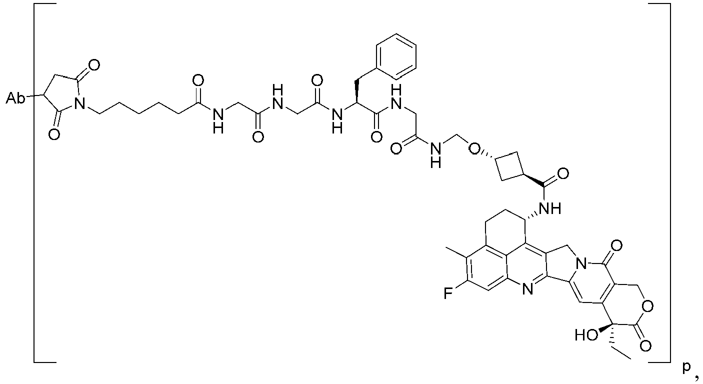 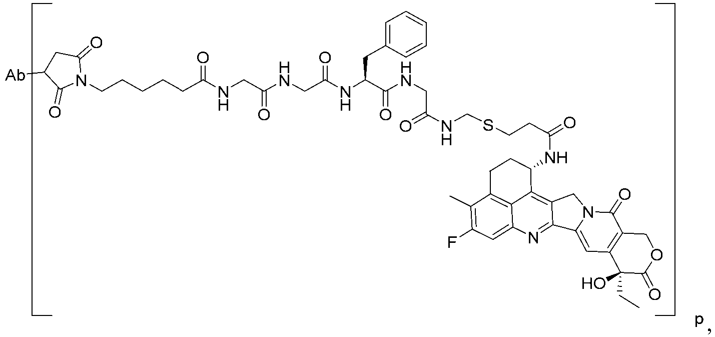 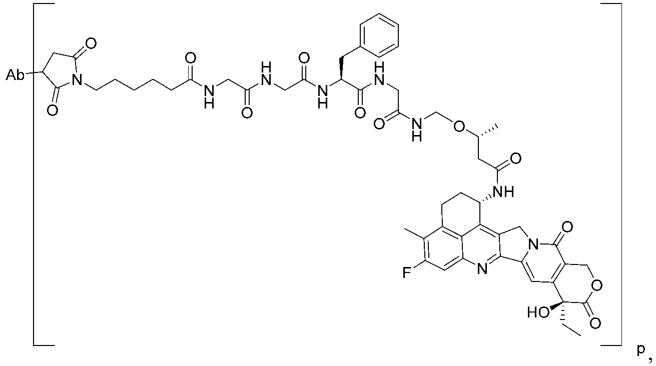 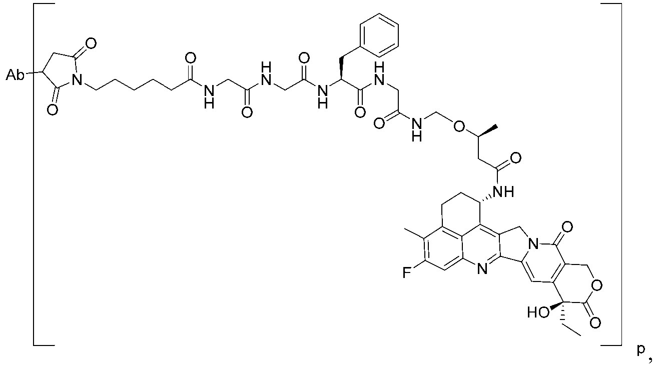 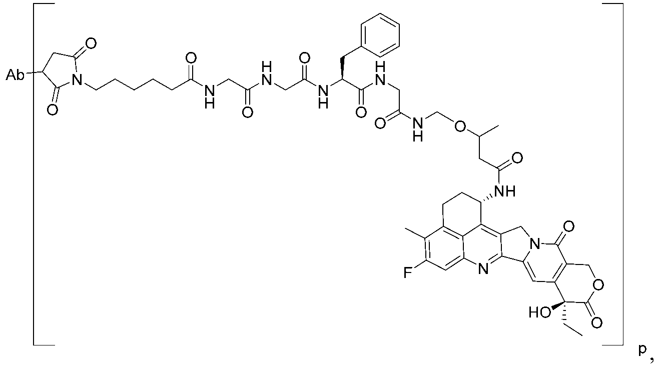 and 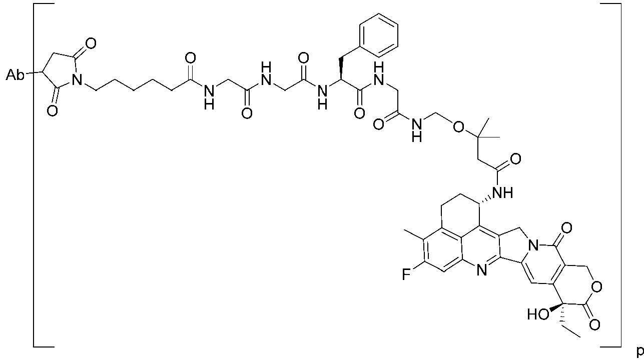 wherein
p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8;
Ab is the bispecific antibody according to any one of claims 1-7 or an antigen-binding fragment thereof; preferably, Ab is selected from bispecific antibodies of a DBXT001 series, a DBXT002 series, a DBXT003 series, and a DBXT004 series, preferably selected from bispecific antibodies of the DBXT001 series, the DBXT002 series, and DBXT005-01, more preferably selected from bispecific antibodies of the DBXT001 series and DBXT005-01, and further preferably selected from bispecific antibodies DBXT001-01 and DBXT005-01.

22. The bispecific antibody-drug conjugate, or the tautomer, the enantiomer, the diastereoisomer or the mixture of isomers thereof, or the pharmaceutically acceptable salt thereof according to claim 21, wherein the bispecific antibody-drug conjugate is selected from any one of the following: 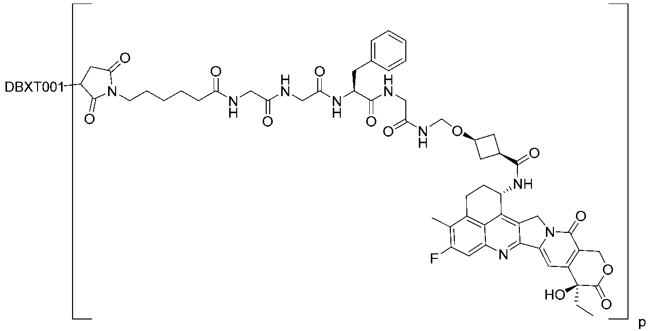 and 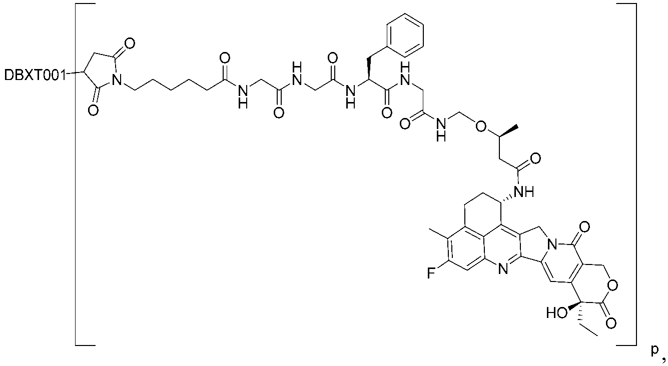 wherein
p represents an average connection number, and p is any one of integers or decimals from 1 to 10, preferably any one of integers or decimals from 3 to 9, such as 4, 4.06, 4.10, 6, 6.11, 6.05, 7.99, 7.98, or 8;
preferably, the bispecific antibody-drug conjugate is selected from any one of the following: 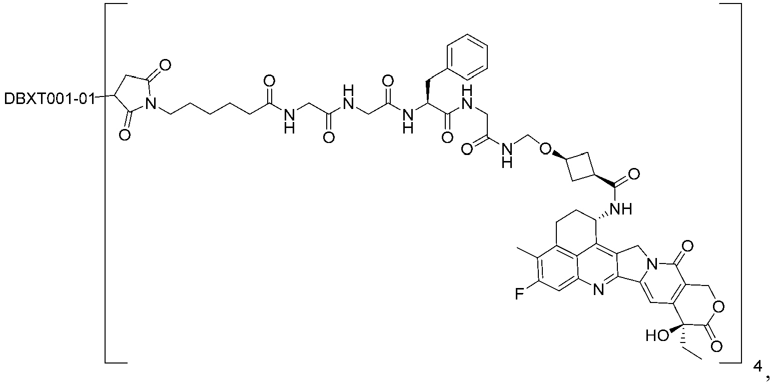 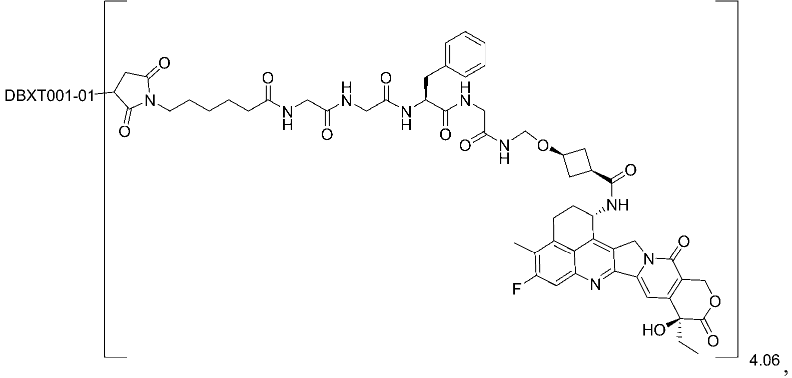 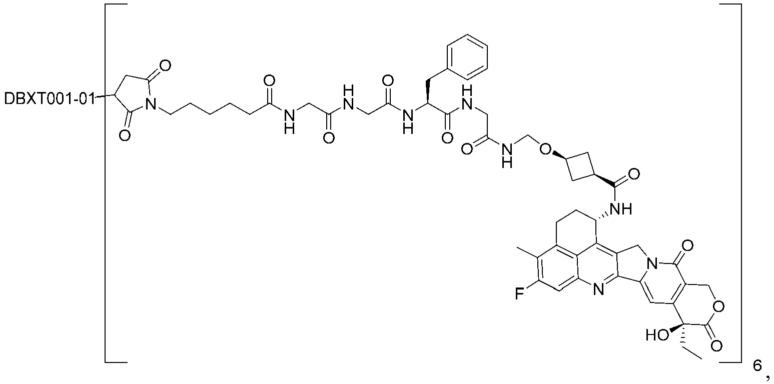 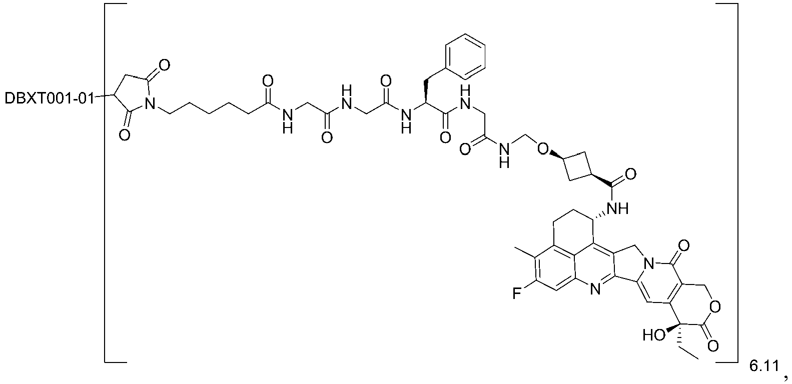 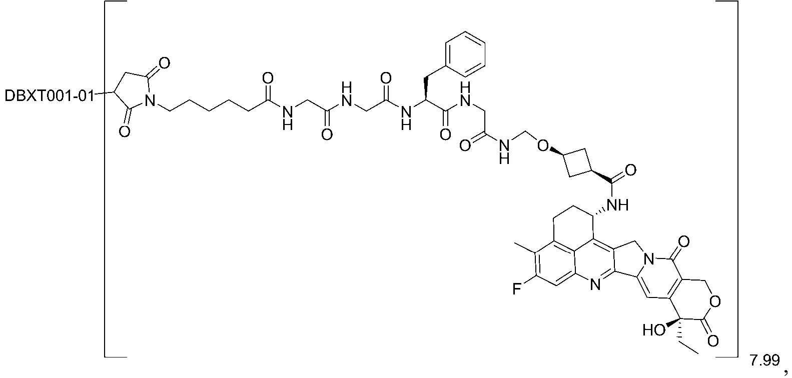 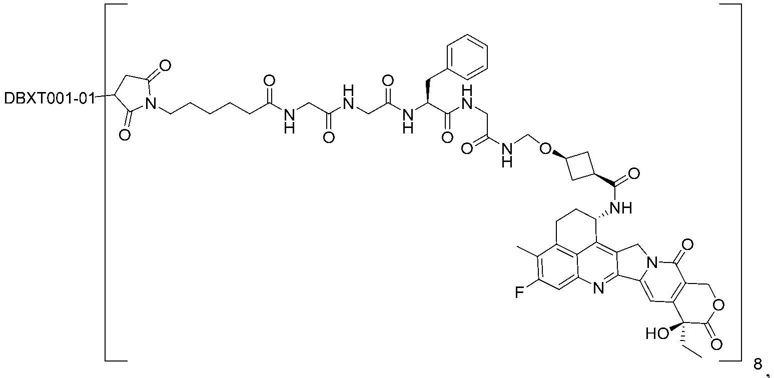 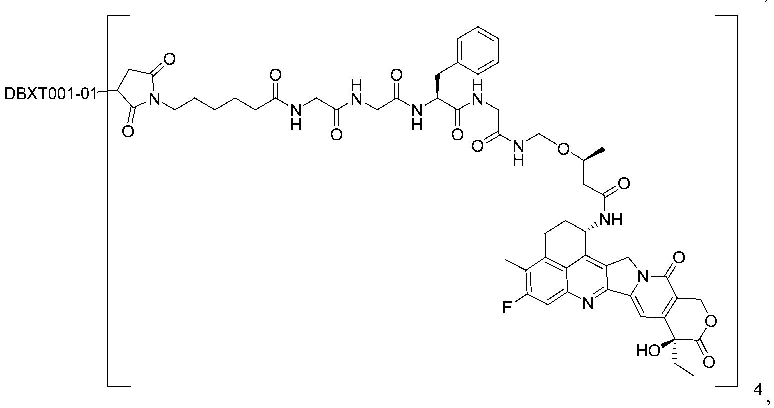 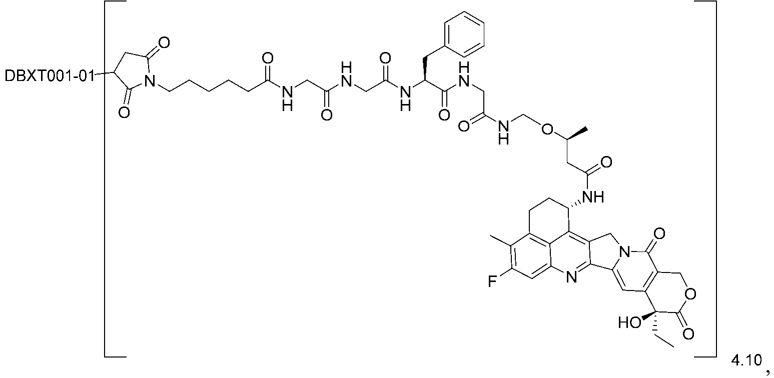 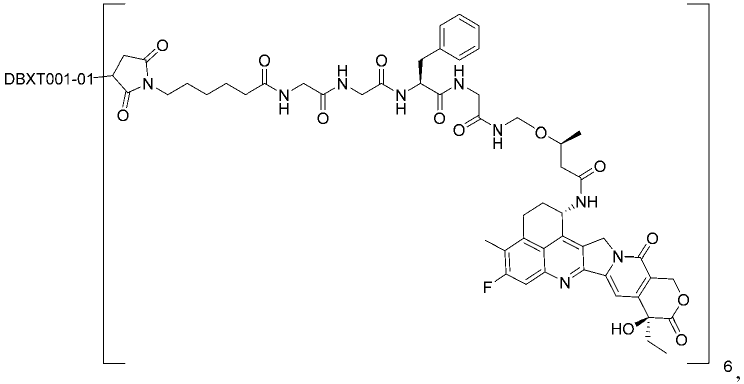 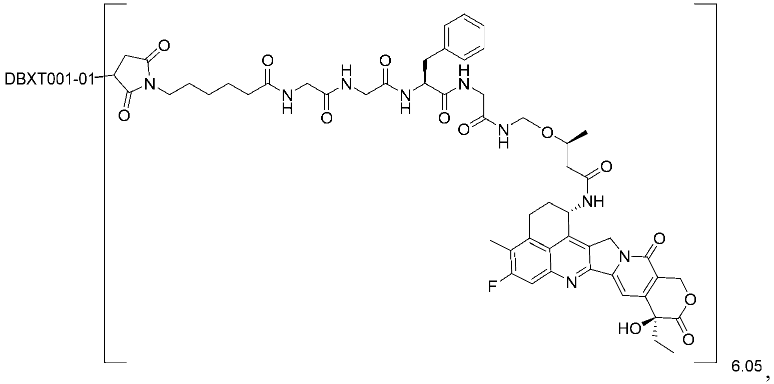 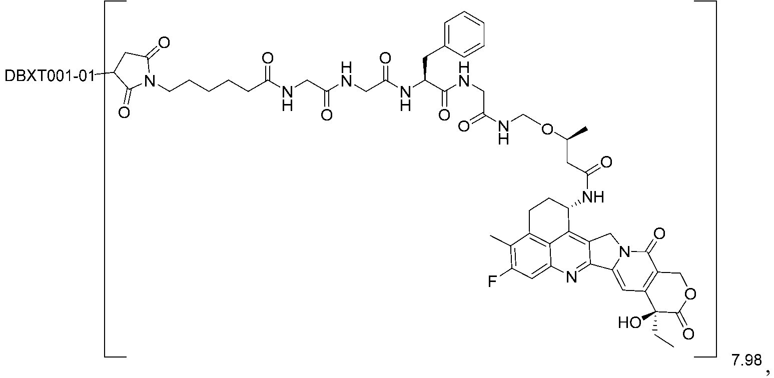 and 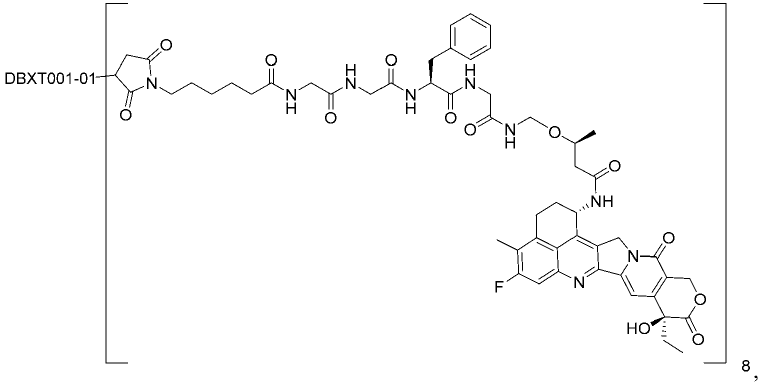 wherein
DBXT001-01 is an anti-EGFR/HER3 bispecific antibody comprising a heavy chain H1, a light chain L1, a heavy chain H2, and a light chain L2 with the amino acid sequences set forth in SEQ ID NO: 37, SEQ ID NO: 38, SEQ ID NO: 39, and SEQ ID NO: 40, respectively.

23. The bispecific antibody-drug conjugate, or the tautomer, the enantiomer, the diastereoisomer or the mixture of isomers thereof, or the pharmaceutically acceptable salt thereof according to any one of claims 12-21, wherein the bispecific antibody-drug conjugate is: 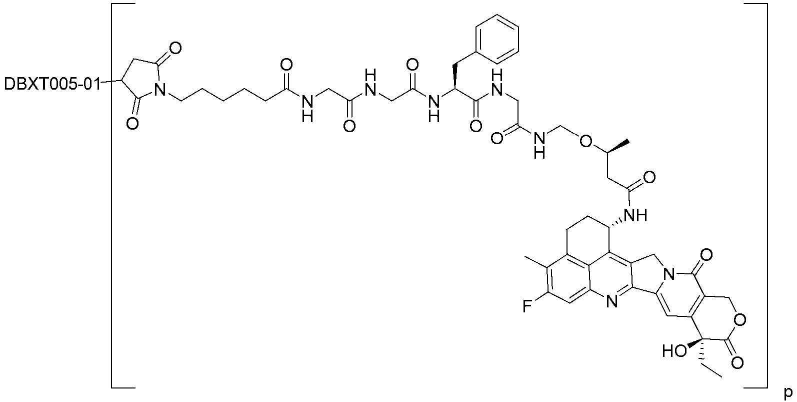
wherein p represents an average connection number, and p is any one of integers or decimals from 1 to 10, preferably any one of integers or decimals from 3 to 9, and more preferably any one of integers or decimals from 4 to 6, such as 5.99;
DBXT005-01 is an anti-EGFR/HER3 bispecific antibody, comprising a heavy chain H1 with the amino acid sequence set forth in SEQ ID NO: 37, a light chain L1 with the amino acid sequence set forth in SEQ ID NO: 38, and a heavy chain H2 with the amino acid sequence set forth in SEQ ID NO: 90.

24. The bispecific antibody-drug conjugate, or the tautomer, the enantiomer, the diastereoisomer or the mixture of isomers thereof, or the pharmaceutically acceptable salt thereof according to any one of claims 12-19, wherein the bispecific antibody-drug conjugate is: 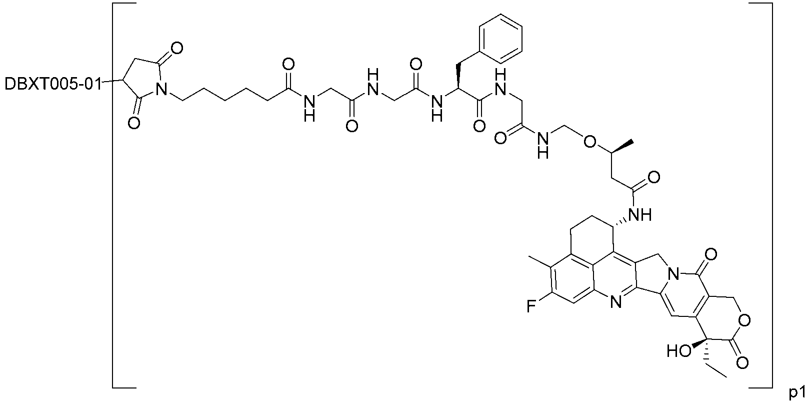
wherein p1 represents a connection number, and p1 is any one of integers from 1 to 10, preferably any one of integers from 3 to 9, and more preferably any one of integers from 4 to 6, such as 4, 5, or 6;
DBXT005-01 is an anti-EGFR/HER3 bispecific antibody, comprising a heavy chain H1 with the amino acid sequence set forth in SEQ ID NO: 37, a light chain L1 with the amino acid sequence set forth in SEQ ID NO: 38, and a heavy chain H2 with the amino acid sequence set forth in SEQ ID NO: 90.

25. A method for preparing the bispecific antibody-drug conjugate, or the tautomer, the enantiomer, the diastereoisomer or the mixture of isomers thereof, or the pharmaceutically acceptable salt thereof according to any one of claims 12-24, comprising the following step: under the action of a reducing agent, mixing the bispecific antibody dissolved in a buffer with the linker unit L-cytotoxic drug dissolved in a solvent to obtain the bispecific antibody-drug conjugate, wherein the reducing agent is preferably tris(2-carboxyethyl)phosphine hydrochloride, the buffer is preferably ethylenediaminetetraacetic acid, and the solvent is preferably dimethylacetamide.

26. The method according to claim 25, comprising reacting the anti-EGFR/HER3 bispecific antibody with a compound of formula X2, for example, reacting DBXT005-01 with a compound of formula X2.

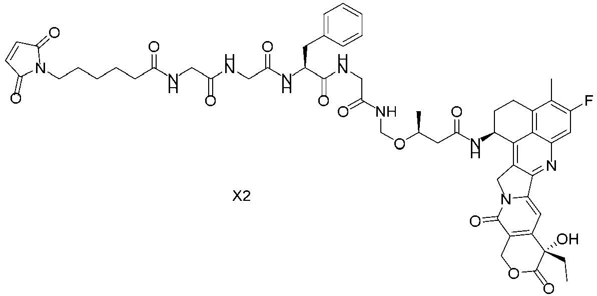

27. A pharmaceutical composition, comprising the bispecific antibody according to any one of claims 1-7, the isolated nucleic acid according to claim 8, the recombinant expression vector according to claim 9, the transformant according to claim 10, and/or the bispecific antibody-drug conjugate according to any one of claims 12-22, and a pharmaceutically acceptable carrier or excipient.

28. Use of the bispecific antibody according to any one of claims 1-7, the isolated nucleic acid according to claim 8, the recombinant expression vector according to claim 9, the transformant according to claim 10, the bispecific antibody-drug conjugate according to any one of claims 12-24, and/or the pharmaceutical composition according to claim 27 in the preparation of a medicament for treating and/or preventing a cancer, wherein preferably, the cancer is a cancer with positive expression of EGFR and/or Her3, for example, the cancer is selected from breast cancer, skin cancer, gastric cancer, colorectal cancer, lung cancer, esophageal cancer, biliary tract cancer, head and neck cancer, thyroid cancer, ovarian cancer, endometrial cancer, pancreatic cancer, prostate cancer, bladder cancer, gastrointestinal cancer, digestive tract cancer, cervical cancer, squamous cell carcinoma, peritoneal cancer, liver cancer, kidney cancer, thyroid cancer, leukemia, malignant lymphoma, plasmacytoma, myeloma, glioma, osteosarcoma, sarcoma, oral squamous cell carcinoma, and melanoma, preferably selected from breast cancer, colorectal cancer, skin cancer, lung cancer, esophageal cancer, and oral squamous cell carcinoma, wherein the lung cancer is preferably non-small cell lung cancer, the skin cancer is preferably cutaneous squamous cell carcinoma, and the colorectal cancer is preferably rectal cancer.

29. A method for treating and/or preventing a cancer, comprising administering to a subject in need the bispecific antibody according to any one of claims 1-7, the bispecific antibody-drug conjugate according to any one of claims 12-24, and/or the pharmaceutical composition according to claim 27, wherein preferably, the cancer is a cancer with positive expression of EGFR and/or Her3, for example, the cancer is selected from breast cancer, skin cancer, gastric cancer, colorectal cancer, lung cancer, esophageal cancer, biliary tract cancer, head and neck cancer, thyroid cancer, ovarian cancer, endometrial cancer, pancreatic cancer, prostate cancer, bladder cancer, gastrointestinal cancer, digestive tract cancer, cervical cancer, squamous cell carcinoma, peritoneal cancer, liver cancer, kidney cancer, thyroid cancer, leukemia, malignant lymphoma, plasmacytoma, myeloma, glioma, osteosarcoma, sarcoma, oral squamous cell carcinoma, and melanoma, preferably selected from breast cancer, colorectal cancer, skin cancer, lung cancer, esophageal cancer, and oral squamous cell carcinoma, wherein the lung cancer is preferably non-small cell lung cancer, the skin cancer is preferably cutaneous squamous cell carcinoma, and the colorectal cancer is preferably rectal cancer.

30. The bispecific antibody according to any one of claims 1-7, the bispecific antibody-drug conjugate according to any one of claims 12-24, and/or the pharmaceutical composition according to claim 27 for use in the prevention and/or treatment of a cancer, wherein preferably, the cancer is a cancer with positive expression of EGFR and/or Her3, for example, the cancer is selected from breast cancer, skin cancer, gastric cancer, colorectal cancer, lung cancer, esophageal cancer, biliary tract cancer, head and neck cancer, thyroid cancer, ovarian cancer, endometrial cancer, pancreatic cancer, prostate cancer, bladder cancer, gastrointestinal cancer, digestive tract cancer, cervical cancer, squamous cell carcinoma, peritoneal cancer, liver cancer, kidney cancer, thyroid cancer, leukemia, malignant lymphoma, plasmacytoma, myeloma, glioma, osteosarcoma, sarcoma, oral squamous cell carcinoma, and melanoma, preferably selected from breast cancer, colorectal cancer, skin cancer, lung cancer, esophageal cancer, and oral squamous cell carcinoma, wherein the lung cancer is preferably non-small cell lung cancer, the skin cancer is preferably cutaneous squamous cell carcinoma, and the colorectal cancer is preferably rectal cancer.

31. A combination therapy, comprising separately administering to a subject in need the bispecific antibody according to any one of claims 1-7, the bispecific antibody-drug conjugate according to any one of claims 12-24, and/or the pharmaceutical composition according to claim 27, and a second therapeutic agent, wherein preferably, the cancer is a cancer with positive expression of EGFR and/or Her3, for example, the cancer is selected from breast cancer, skin cancer, gastric cancer, colorectal cancer, lung cancer, esophageal cancer, biliary tract cancer, head and neck cancer, thyroid cancer, ovarian cancer, endometrial cancer, pancreatic cancer, prostate cancer, bladder cancer, gastrointestinal cancer, digestive tract cancer, cervical cancer, squamous cell carcinoma, peritoneal cancer, liver cancer, kidney cancer, thyroid cancer, leukemia, malignant lymphoma, plasmacytoma, myeloma, glioma, osteosarcoma, sarcoma, oral squamous cell carcinoma, and melanoma, preferably selected from breast cancer, colorectal cancer, skin cancer, lung cancer, esophageal cancer, and oral squamous cell carcinoma, wherein the lung cancer is preferably non-small cell lung cancer, the skin cancer is preferably cutaneous squamous cell carcinoma, and the colorectal cancer is preferably rectal cancer.

32. Use of the bispecific antibody according to any one of claims 1-7, the isolated nucleic acid according to claim 8, the recombinant expression vector according to claim 9, the transformant according to claim 10, the bispecific antibody-drug conjugate according to any one of claims 12-24, and/or the pharmaceutical composition according to claim 27 in the preparation of EGFR and/or HER3 inhibitors.
